# FASCICULE DE BREVET EUROPEEN

(11) **EP 0 822 988 B1**
(45) Date de publication et mention de la délivrance du brevet: **21.12.2005**
(21) Numéro de dépôt: 96915066.3
(22) Date de dépôt: 19.04.1996
(51) Int. Cl.: C12N 15/55, C12N 15/82, A01H 5/00, A23K 1/14, C12P 7/64, A61K 38/46, C10L 1/02, C10L 1/14, C10L 1/16

(54) **LIPASES PREDUODENALES RECOMBINANTES ET POLYPEPTIDES DERIVES PRODUITS PAR LES PLANTES, LEURS PROCEDES D'OBTENTION ET LEURS UTILISATIONS**
DURCH PFLANZEN HERGESTELLTE REKOMBINANTE PREDUODENALE LIPASE UND ABGELEITETE POLYPEPTIDE, VERFAHREN ZUR HERSTELLUNG UND IHRE VERWENDUNG
RECOMBINANT PREDUODENAL LIPASES AND POLYPEPTIDE DERIVATIVES PRODUCED BY PLANTS, PROCESSES FOR OBTAINING THEM AND THEIR USES

(30) Priorité: 20.04.1995 FR 9504754
(43) Date de publication de la demande: 11.02.1998
(73) Titulaire: MERISTEM THERAPEUTICS, 63100 Clermont-Ferrand (FR)
(72) Inventeur: LENEE, Philippe, Boîte postale 14555 98800 Nouméa (NC); GRUBER, Véronique Résidence Sainte-Madeleine, F-63400 Chamalières (FR); BAUDINO, Sylvie, F-63870 Orcines (FR); MEROT, Bertrand, F-63530 Volvic (FR); BENICOURT, Claude, F-78800 Houilles (FR); CUDREY, Claire, F-92160 Antony (FR)
(74) Mandataire: Thurgood, Alexander John
(86) Numéro de dépôt international: PCT/FR1996/000606
(87) Numéro de publication internationale: WO 1996/033277

(56) Documents cités:
- EP-A- 0 261 016
- EP-A- 0 449 376
- EP-A- 0 542 629
- WO-A-86/01532
- WO-A-91/06661
- WO-A-92/01042
- WO-A-93/03161
- WO-A-94/13816
- WO-A-94/20610
- WO-A-96/03511
- GB-A- 2 090 613
- GB-A- 2 188 057
- BIOTECHNOLOGY, vol. 10, pages 292-296, XP002013284 PEN, J., ET AL.: "Production of active Bacillus licheniformis alpha-amylase in tobacco and its application in starch liquefaction"
- ESCOBAR, H., F. BAQUERO AND L. SUAREZ (ED.). INTERNATIONAL CONGRESS SERIES, NO. 1034. CLINICAL ECOLOGY OF CYSTIC FIBROSIS, 1993, 18TH EUROPEAN CYSTIC FIBROSIS CONFERENCE, MADRID, SPAIN, MAY 21-26, 1993.ELSEVIER SCIENCE PUBLISHERS B.V.: AMSTERDAM, NETHERLANDS; NEW YORK,NEW YORK, USA.ISBN: 0-444-81670-4, pages 291-295, XP000603098 BENICOURT C ET AL: "Potential use of a recombinant dog gastric lipase as an enzymatic supplement to pancreatic extracts in cystic fibrosis."
- MOKRZYCKI-ISSARTEL ET AL.: "A transient tobacco expression system coupled to MALDI-TOF-MS allows validation of the impact of differential targeting on structure and activity of a recombinant therapeutic glycoprotein produced in plants." FEBS LETTERS, vol. 552, 2003, pages 170-176,

### LISTE DE SEQUENCES

(1) INFORMATION GENERALE:
   (i) DEPOSANT:
      (A) NOM: BIOCEM S.A.
      (B) RUE: 24, avenue des Landais
      (C) VILLE: AUBIERE
      (E) PAYS: FRANCE
      (F) CODE POSTAL: 63170

      (A) NOM: INSTITUT DE RECHERCHE JOUVEINAL
      (B) RUE: 3-9, rue de la Loge
      (C) VILLE: FRESNES
      (E) PAYS: FRANCE
      (F) CODE POSTAL: 94265 CEDEX
   (ii) TITRE DE L' INVENTION: LIPASES PREDUODENALES RECOMBINANTES ET POLYPEPTIDES DERIVES PRODUITS PAR LES PLANTES, LEURS PROCEDES D'OBTENBTION ET LEURS UTILISATIONS
   (iii) NOMBRE DE SEQUENCES: 6
   (iv) FORME LISIBLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
   (vi) DONNEES DE LA DEMANDE ANTERIEURE:
      (A) NUMERO DE DEPOT: FR 9504754
      (B) DATE DE DEPOT: 20-APR-1995
(2) INFORMATION POUR LA SEQ ID NO: 1:
   (i) CARACTERISTIQUES DE LA SEQUENCE:
      (A) LONGUEUR: 1528 paires de bases
      (B) TYPE: acide nucléique
      (C) NOMBRE DE BRINS: simple
      (D) CONFIGURATION: linéaire
   (ii) TYPE DE MOLECULE: ADNc pour ARNm
   (ix) CARACTERISTIQUE ADDITIONELLE:
      (A) NOM/CLE: CDS
      (B) EMPLACEMENT: 1..1137
   (xi) DESCRIPTION DE LA SEQUENCE: SEQ ID NO: 1:
   TTG TTT GGA AAG CTT CAT CCC ACA AAC CCT GAA GTG ACC ATG AAT ATA         48
   Leu Phe Gly Lys Leu His Pro Thr Asn Pro Glu Val Thr Met Asn Ile
     1               5                  10                  15
   AGT CAG ATG ATC ACC TAC TGG GGA TAC CCA GCT GAG GAA TAT GAA GTT      96
   Ser Gln Met Ile Thr Tyr Trp Gly Tyr Pro Ala Glu Glu Tyr Glu Val
               20                  25                  30
   GTG ACC GAA GAC GGT TAT ATC CTT GGG ATC GAC AGA ATT CCT TAT GGG     144
   Val Thr Glu Asp Gly Tyr Ile Leu Gly Ile Asp Arg Ile Pro Tyr Gly
           35                  40                  45
   AGG AAA AAT TCA GAG AAT ATA GGC CGG AGA CCT GTT GCA TTT TTG CAA     192
   Arg Lys Asn Ser Glu Asn Ile Gly Arg Arg Pro Val Ala Phe Leu Gln
       50                  55                  60
   CAC GGT TTG CTC GCA TCA GCC ACA AAC TGG ATC TCC AAC CTG CCC AAC     240
   His Gly Leu Leu Ala Ser Ala Thr Asn Trp Ile Ser Asn Leu Pro Asn
   65                  70                  75                      80
   AAC AGC CTG GCC TTC ATC CTG GCC GAC GCC GGG TAC GAC GTG TGG CTG     288
   Asn Ser Leu Ala Phe Ile Leu Ala Asp Ala Gly Tyr Asp Val Trp Leu
                   85                  90                  95
   GGG AAC AGC AGG GGC AAC ACC TGG GCC AGG AGG AAT CTG TAC TAC TCG     336
   Gly Asn Ser Arg Gly Asn Thr Trp Ala Arg Arg Asn Leu Tyr Tyr Ser
               100                 105                 110
   CCC GAC TCC GTC GAA TTC TGG GCT TTC AGC TTT GAC GAG ATG GCT AAA     384
   Pro Asp Ser Val Glu Phe Trp Ala Phe Ser Phe Asp Glu Met Ala Lys
           115                 120                 125
   TAT GAC CTT CCC GCC ACC ATT GAC TTC ATC TTG AAG AAA ACG GGA CAG     432
   Tyr Asp Leu Pro Ala Thr Ile Asp Phe Ile Leu Lys Lys Thr Gly Gln
       130                 135                 140
   GAC AAG CTA CAC TAC GTT GGC CAT TCC CAG GGC ACC ACC ATT GGT TTC     480
   Asp Lys Leu His Tyr Val Gly His Ser Gln Gly Thr Thr Ile Gly Phe
   145                 150                 155                 160
   ATC GCC TTT TCC ACC AAT CCC AAG CTG GCG AAA CGG ATC AAA ACC TTC     528
   Ile Ala Phe Ser Thr Asn Pro Lys Leu Ala Lys Arg Ile Lys Thr Phe
                   165                 170                 175
   TAT GCA TTA GCT CCC GTT GCC ACC GTG AAG TAC ACC GAA ACC CTG TTA     576
   Tyr Ala Leu Ala Pro Val Ala Thr Val Lys Tyr Thr Glu Thr Leu Leu
               180                 185                 190
   AAC AAA CTC ATG CTC GTC CCT TCG TTC CTC TTC AAG CTT ATA TTT GGA     624
   Asn Lys Leu Met Leu Val Pro Ser Phe Leu Phe Lys Leu Ile Phe Gly
           195                 200                 205
   AAC AAA ATA TTC TAC CCA CAC CAC TTC TTT GAT CAA TTT CTC GCC ACC     672
   Asn Lys Ile Phe Tyr Pro His His Phe Phe Asp Gln Phe Leu Ala Thr
       210                 215                 220
   GAG GTA TGC TCC CGC GAG ACG GTG GAT CTC CTC TGC AGC AAC GCC CTG     720
   Glu Val Cys Ser Arg Glu Thr Val Asp Leu Leu Cys Ser Asn Ala Leu
   225                 230                 235                 240
   TTT ATC ATT TGT GGA TTT GAC ACT ATG AAC TTG AAC ATG AGT CGC TTG      768
   Phe Ile Ile Cys Gly Phe Asp Thr Met Asn Leu Asn Met Ser Arg Leu
                   245                     250                     255
   GAT GTG TAT CTG TCA CAT AAT CCA GCA GGA ACA TCG GTT CAG AAC GTG      816
   Asp Val Tyr Leu Ser His Asn Pro Ala Gly Thr Ser Val Gln Asn Val
               260                     265                     270
   CTC CAC TGG TCC CAG GCT GTT AAG TCT GGG AAG TTC CAA GCT TTT GAC      864
   Leu His Trp Ser Gln Ala Val Lys Ser Gly Lys Phe Gln Ala Phe Asp
           275                     280                     285
   TGG GGA AGC CCA GTT CAG AAC ATG ATG CAC TAT CAT CAG AGC ATG CCT      912
   Trp Gly Ser Pro Val Gln Asn Met Met His Tyr His Gln Ser Met Pro
       290                     295                     300
   CCC TAC TAC AAC CTG ACA GAC ATG CAT GTG CCA ATC GCA GTG TGG AAC      960
   Pro Tyr Tyr Asn Leu Thr Asp Met His Val Pro Ile Ala Val Trp Asn
   305                 310                     315                 320
   GGT GGC AAC GAC TTG CTG GCC GAC CCT CAC GAT GTT GAC CTT TTG CTT     1008
   Gly Gly Asn Asp Leu Leu Ala Asp Pro His Asp Val Asp Leu Leu Leu
                   325                     330                     335
   TCC AAG CTC CCC AAT CTC ATT TAC CAC AGG AAG ATT CCT CCT TAC AAT     1056
   Ser Lys Leu Pro Asn Leu Ile Tyr His Arg Lys Ile Pro Pro Tyr Asn
               340                     345                     350
   CAC TTG GAC TTT ATC TGG GCC ATG GAT GCC CCT CAA GCG GTT TAC AAT     1104
   His Leu Asp Phe Ile Trp Ala Met Asp Ala Pro Gln Ala Val Tyr Asn
           355                     360                     365
   GAA ATT GTT TCC ATG ATG GGA ACA GAT AAT AAG TAGTTCTAGA TTTAAGGAAT   1157
   Glu Ile Val Ser Met Met Gly Thr Asp Asn Lys
       370                     375
   TATTCTTTTA TTGTTCCAAA ATACGTTCTT CTCTCACACG TGGTTTTCTA TCATGTTTGA   1217
   GACACGGTGA TTGTTCCCAT GGTTTTGATT TCAGAAATGT GTTAGCATCA ACAATCTTTC   1277
   CATTGGTAAT TTTTGAATTT AAAATGATTT TTAAATTTGG GGCATCTGGG TGGCTCAGTT   1337
   GGCTAAGTCG TCTGCCTTGG CTTAAGTCAT GATCTCGGGG TCCTAGGATG GAGCCTTGTG   1397
   TCTGGGCTCC TGCCGGGGCG GGGGTCTGCT TCTCCTCCTG CTGCTCCCCC CTGCTGCTGT   1457
   GTGCACACAC GCTCTCTCTC TCTCAAATAA ATAAATAAAT AAATACTTAA TAAAATAAAA   1517
   AAAAAAAAAA A                                                        1528
(2) INFORMATION POUR LA SEQ ID NO: 2:
   (i) CARACTERISTIQUES DE LA SEQUENCE:
      (A) LONGUEUR: 379 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 2:
   Leu Phe Gly Lys Leu His Pro Thr Asn Pro Glu Val Thr Met Asn Ile
    1               5                  10                  15
   Ser Gln Met Ile Thr Tyr Trp Gly Tyr Pro Ala Glu Glu Tyr Glu Val
               20                  25                  30
   Val Thr Glu Asp Gly Tyr Ile Leu Gly Ile Asp Arg Ile Pro Tyr Gly
           35                  40                  45
   Arg Lys Asn Ser Glu Asn Ile Gly Arg Arg Pro Val Ala Phe Leu Gln
       50                  55                  60
   His Gly Leu Leu Ala Ser Ala Thr Asn Trp Ile Ser Asn Leu Pro Asn
   65                  70                  75                  80
   Asn Ser Leu Ala Phe Ile Leu Ala Asp Ala Gly Tyr Asp Val Trp Leu
                   85                  90                  95
   Gly Asn Ser Arg Gly Asn Thr Trp Ala Arg Arg Asn Leu Tyr Tyr Ser
               100                 105                 110
   Pro Asp Ser Val Glu Phe Trp Ala Phe Ser Phe Asp Glu Met Ala Lys
           115                 120                 125
   Tyr Asp Leu Pro Ala Thr Ile Asp Phe Ile Leu Lys Lys Thr Gly Gln
       130                 135                 140
   Asp Lys Leu His Tyr Val Gly His Ser Gln Gly Thr Thr Ile Gly Phe
   145                 150                 155                 160
   Ile Ala Phe Ser Thr Asn Pro Lys Leu Ala Lys Arg Ile Lys Thr Phe
                   165                 170                 175
   Tyr Ala Leu Ala Pro Val Ala Thr Val Lys Tyr Thr Glu Thr Leu Leu
               180                 185                 190
   Asn Lys Leu Met Leu Val Pro Ser Phe Leu Phe Lys Leu Ile Phe Gly
           195                 200                 205
   Asn Lys Ile Phe Tyr Pro His His Phe Phe Asp Gln Phe Leu Ala Thr
       210                 215                 220
   Glu Val Cys Ser Arg Glu Thr Val Asp Leu Leu Cys Ser Asn Ala Leu
   225                 230                 235                 240
   Phe Ile Ile Cys Gly Phe Asp Thr Met Asn Leu Asn Met Ser Arg Leu
                   245                 250                 255
   Asp Val Tyr Leu Ser His Asn Pro Ala Gly Thr Ser Val Gln Asn Val
               260                 265                 270
   Leu His Trp Ser Gln Ala Val Lys Ser Gly Lys Phe Gln Ala Phe Asp
           275                 280                 285
   Trp Gly Ser Pro Val Gln Asn Met Met His Tyr His Gln Ser Met Pro
       290                 295                 300
   Pro Tyr Tyr Asn Leu Thr Asp Met His Val Pro Ile Ala Val Trp Asn
   305                 310                 315                 320
   Gly Gly Asn Asp Leu Leu Ala Asp Pro His Asp Val Asp Leu Leu Leu
                   325                 330                 335
   Ser Lys Leu Pro Asn Leu Ile Tyr His Arg Lys Ile Pro Pro Tyr Asn
               340                 345                 350
   His Leu Asp Phe Ile Trp Ala Met Asp Ala Pro Gln Ala Val Tyr Asn
           355                 360                 365
   Glu Ile Val Ser Met Met Gly Thr Asp Asn Lys
       370                 375
(2) INFORMATION POUR LA SEQ ID NO: 3:
   (i) CARACTERISTIQUES DE LA SEQUENCE:
      (A) LONGUEUR: 1048 paires de bases
      (B) TYPE: acide nucléique
      (C) NOMBRE DE BRINS: double
      (D) CONFIGURATION: linéaire
   (ii) TYPE DE MOLECULE: ADN (génomique)
   (ix) CARACTERISTIQUE ADDITIONELLE:
      (A) NOM/CLE: CDS
      (B) EMPLACEMENT: 1..975
   (xi) DESCRIPTION DE LA SEQUENCE: SEQ ID NO: 3:
   ATA GGC CGG AGA CCT GTT GCA TTT TTG CAA CAC GGT TTG CTC GCA TCA       48
   Ile Gly Arg Arg Pro Val Ala Phe Leu Gln His Gly Leu Leu Ala Ser
     1               5                  10                  15
   GCC ACA AAC TGG ATC TCC AAC CTG CCC AAC AAC AGC CTG GCC TTC ATC       96
   Ala Thr Asn Trp Ile Ser Asn Leu Pro Asn Asn Ser Leu Ala Phe Ile
                20                  25                  30
   CTG GCC GAC GCC GGG TAC GAC GTG TGG CTG GGG AAC AGC AGG GGC AAC      144
   Leu Ala Asp Ala Gly Tyr Asp Val Trp Leu Gly Asn Ser Arg Gly Asn
            35                  40                  45
   ACC TGG GCC AGG AGG AAT CTG TAC TAC TCG CCC GAC TCC GTC GAA TTC      192
   Thr Trp Ala Arg Arg Asn Leu Tyr Tyr Ser Pro Asp Ser Val Glu Phe
        50                  55                  60
   TGG GCT TTC AGC TTT GAC GAG ATG GCT AAA TAT GAC CTT CCC GCC ACC         240
   Trp Ala Phe Ser Phe Asp Glu Met Ala Lys Tyr Asp Leu Pro Ala Thr
    65                 70                  75                  80
   ATT GAC TTC ATC TTG AAG AAA ACG GGA CAG GAC AAG CTA CAC TAC GTT         288
   Ile Asp Phe Ile Leu Lys Lys Thr Gly Gln Asp Lys Leu His Tyr Val
                   85                  90                  95
   GGC CAT TCC CAG GGC ACC ACC ATT GGT TTC ATC GCC TTT TCC ACC AAT         336
   Gly His Ser Gln Gly Thr Thr Ile Gly Phe Ile Ala Phe Ser Thr Asn
               100                 105                 110
   CCC AAG CTG GCG AAA CGG ATC AAA ACC TTC TAT GCA TTA GCT CCC GTT         384
   Pro Lys Leu Ala Lys Arg Ile Lys Thr Phe Tyr Ala Leu Ala Pro Val
           115                 120                 125
   GCC ACC GTG AAG TAC ACC GAA ACC CTG TTA AAC AAA CTC ATG CTC GTC         432
   Ala Thr Val Lys Tyr Thr Glu Thr Leu Leu Asn Lys Leu Met Leu Val
       130                 135                 140
   CCT TCG TTC CTC TTC AAG CTT ATA TTT GGA AAC AAA ATA TTC TAC CCA         480
   Pro Ser Phe Leu Phe Lys Leu Ile Phe Gly Asn Lys Ile Phe Tyr Pro
   145                 150                 155                 160
   CAC CAC TTC TTT GAT CAA TTT CTC GCC ACC GAG GTA TGC TCC CGC GAG         528
   His His Phe Phe Asp Gln Phe Leu Ala Thr Glu Val Cys Ser Arg Glu
                   165                 170                 175
   ACG GTG GAT CTC CTC TGC AGC AAC GCC CTG TTT ATC ATT TGT GGA TTT         576
   Thr Val Asp Leu Leu Cys Ser Asn Ala Leu Phe Ile Ile Cys Gly Phe
               180                 185                 190
   GAC ACT ATG AAC TTG AAC ATG AGT CGC TTG GAT GTG TAT CTG TCA CAT         624
   Asp Thr Met Asn Leu Asn Met Ser Arg Leu Asp Val Tyr Leu Ser His
           195                 200                 205
   AAT CCA GCA GGA ACA TCG GTT CAG AAC GTG CTC CAC TGG TCC CAG GCT         672
   Asn Pro Ala Gly Thr Ser Val Gln Asn Val Leu His Trp Ser Gln Ala
       210                 215                 220
   GTT AAG TCT GGG AAG TTC CAA GCT TTT GAC TGG GGA AGC CCA GTT CAG         720
   Val Lys Ser Gly Lys Phe Gln Ala Phe Asp Trp Gly Ser Pro Val Gln
   225                 230                 235                 240
   AAC ATG ATG CAC TAT CAT CAG AGC ATG CCT CCC TAC TAC AAC CTG ACA         768
   Asn Met Met His Tyr His Gln Ser Met Pro Pro Tyr Tyr Asn Leu Thr
                   245                 250                 255
   GAC ATG CAT GTG CCA ATC GCA GTG TGG AAC GGT GGC AAC GAC TTG CTG         816
   Asp Met His Val Pro Ile Ala Val Trp Asn Gly Gly Asn Asp Leu Leu
               260                 265                 270
   GCC GAC CCT CAC GAT GTT GAC CTT TTG CTT TCC AAG CTC CCC AAT CTC         864
   Ala Asp Pro His Asp Val Asp Leu Leu Leu Ser Lys Leu Pro Asn Leu
           275                 280                 285
   ATT TAC CAC AGG AAG ATT CCT CCT TAC AAT CAC TTG GAC TTT ATC TGG      912
   Ile Tyr His Arg Lys Ile Pro Pro Tyr Asn His Leu Asp Phe Ile Trp
       290                 295                 300
   GCC ATG GAT GCC CCT CAA GCG GTT TAC AAT GAA ATT GTT TCC ATG ATG      960
   Ala Met Asp Ala Pro Gln Ala Val Tyr Asn Glu Ile Val Ser Met Met
   305                 310                 315                 320
   GGA ACA GAT AAT AAG TAGTTCTAGA TTTAAGGAAT TATTCTTTTA TTGTTCCAAA      1015
   Gly Thr Asp Asn Lys
                   325
   ATACGTTCTT CTCTCACACG TGGTTTTCTA TCA                                 1048
(2) INFORMATION POUR LA.SEQ ID NO: 4:
   (i) CARACTERISTIQUES DE LA SEQUENCE:
      (A) LONGUEUR: 325 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 4:
(2) INFORMATION POUR LA SEQ ID NO: 5:
   (i) CARACTERISTIQUES DE LA SEQUENCE:
      (A) LONGUEUR: 1198 paires de bases
      (B) TYPE: acide nucléique
      (C) NOMBRE DE BRINS: double
      (D) CONFIGURATION: linéaire
   (ii) TYPE DE MOLECULE: ADN (génomique)
   (ix) CARACTERISTIQUE ADDITIONELLE:
      (A) NOM/CLE: CDS
      (B) EMPLACEMENT: 1..1125
   (xi) DESCRIPTION DE LA SEQUENCE: SEQ ID NO: 5:
   CTT CAT CCC ACA AAC CCT GAA GTG ACC ATG AAT ATA AGT CAG ATG ATC        48
   Leu His Pro Thr Asn Pro Glu Val Thr Met Asn Ile Ser Gln Met Ile
     1               5                  10                  15
   ACC TAC TGG GGA TAC CCA GCT GAG GAA TAT GAA GTT GTG ACC GAA GAC      96
   Thr Tyr Trp Gly Tyr Pro Ala Glu Glu Tyr Glu Val Val Thr Glu Asp
               20                  25                  30
   GGT TAT ATC CTT GGG ATC GAC AGA ATT CCT TAT GGG AGG AAA AAT TCA     144
   Gly Tyr Ile Leu Gly Ile Asp Arg Ile Pro Tyr Gly Arg Lys Asn Ser
           35                  40                  45
   GAG AAT ATA GGC CGG AGA CCT GTT GCA TTT TTG CAA CAC GGT TTG CTC     192
   Glu Asn Ile Gly Arg Arg Pro Val Ala Phe Leu Gln His Gly Leu Leu
       50                  55                  60
   GCA TCA GCC ACA AAC TGG ATC TCC AAC CTG CCC AAC AAC AGC CTG GCC     240
   Ala Ser Ala Thr Asn Trp Ile Ser Asn Leu Pro Asn Asn Ser Leu Ala
   65                  70                  75                  80
   TTC ATC CTG GCC GAC GCC GGG TAC GAC GTG TGG CTG GGG AAC AGC AGG     288
   Phe Ile Leu Ala Asp Ala Gly Tyr Asp Val Trp Leu Gly Asn Ser Arg
                   85                  90                  95
   GGC AAC ACC TGG GCC AGG AGG AAT CTG TAC TAC TCG CCC GAC TCC GTC     336
   Gly Asn Thr Trp Ala Arg Arg Asn Leu Tyr Tyr Ser Pro Asp Ser Val
               100                 105                 110
   GAA TTC TGG GCT TTC AGC TTT GAC GAG ATG GCT AAA TAT GAC CTT CCC     384
   Glu Phe Trp Ala Phe Ser Phe Asp Glu Met Ala Lys Tyr Asp Leu Pro
           115                 120                 125
   GCC ACC ATT GAC TTC ATC TTG AAG AAA ACG GGA CAG GAC AAG CTA CAC     432
   Ala Thr Ile Asp Phe Ile Leu Lys Lys Thr Gly Gln Asp Lys Leu His
       130                 135                 140
   TAC GTT GGC CAT TCC CAG GGC ACC ACC ATT GGT TTC ATC GCC TTT TCC     480
   Tyr Val Gly His Ser Gln Gly Thr Thr Ile Gly Phe Ile Ala Phe Ser
   145                 150                 155                 160
   ACC AAT CCC AAG CTG GCG AAA CGG ATC AAA ACC TTC TAT GCA TTA GCT     528
   Thr Asn Pro Lys Leu Ala Lys Arg Ile Lys Thr Phe Tyr Ala Leu Ala
                   165                 170                 175
   CCC GTT GCC ACC GTG AAG TAC ACC GAA ACC CTG TTA AAC AAA CTC ATG     576
   Pro Val Ala Thr Val Lys Tyr Thr Glu Thr Leu Leu Asn Lys Leu Met
               180                 185                 190
   CTC GTC CCT TCG TTC CTC TTC AAG CTT ATA TTT GGA AAC AAA ATA TTC     624
   Leu Val Pro Ser Phe Leu Phe Lys Leu Ile Phe Gly Asn Lys Ile Phe
           195                 200                 205
   TAC CCA CAC CAC TTC TTT GAT CAA TTT CTC GCC ACC GAG GTA TGC TCC     672
   Tyr Pro His His Phe Phe Asp Gln Phe Leu Ala Thr Glu Val Cys Ser
       210                 215                 220
   CGC GAG ACG GTG GAT CTC CTC TGC AGC AAC GCC CTG TTT ATC ATT TGT     720
   Arg Glu Thr Val Asp Leu Leu Cys Ser Asn Ala Leu Phe Ile Ile Cys
   225                 230                 235                 240
   GGA TTT GAC ACT ATG AAC TTG AAC ATG AGT CGC TTG GAT GTG TAT CTG          768
   Gly Phe Asp Thr Met Asn Leu Asn Met Ser Arg Leu Asp Val Tyr Leu
                   245                 250                 255
   TCA CAT AAT CCA GCA GGA ACA TCG GTT CAG AAC GTG CTC CAC TGG TCC          816
   Ser His Asn Pro Ala Gly Thr Ser Val Gln Asn Val Leu His Trp Ser
               260                 265                 270
   CAG GCT GTT AAG TCT GGG AAG TTC CAA GCT TTT GAC TGG GGA AGC CCA          864
   Gln Ala Val Lys Ser Gly Lys Phe Gln Ala Phe Asp Trp Gly Ser Pro
           275                 280                 285
   GTT CAG AAC ATG ATG CAC TAT CAT CAG AGC ATG CCT CCC TAC TAC AAC          912
   Val Gln Asn Met Met His Tyr His Gln Ser Met Pro Pro Tyr Tyr Asn
       290                 295                 300
   CTG ACA GAC ATG CAT GTG CCA ATC GCA GTG TGG AAC GGT GGC AAC GAC          960
   Leu Thr Asp Met His Val Pro Ile Ala Val Trp Asn Gly Gly Asn Asp
   305                 310                 315                 320
   TTG CTG GCC GAC CCT CAC GAT GTT GAC CTT TTG CTT TCC AAG CTC CCC         1008
   Leu Leu Ala Asp Pro His Asp Val Asp Leu Leu Leu Ser Lys Leu Pro
                   325                 330                 335
   AAT CTC ATT TAC CAC AGG AAG ATT CCT CCT TAC AAT CAC TTG GAC TTT         1056
   Asn Leu Ile Tyr His Arg Lys Ile Pro Pro Tyr Asn His Leu Asp Phe
               340                 345                 350
   ATC TGG GCC ATG GAT GCC CCT CAA GCG GTT TAC AAT GAA ATT GTT TCC         1104
   Ile Trp Ala Met Asp Ala Pro Gln Ala Val Tyr Asn Glu Ile Val Ser
           355                 360                 365
   ATG ATG GGA ACA GAT AAT AAG TAGTTCTAGA TTTAAGGAAT TATTCTTTTA            1155
   Met Met Gly Thr Asp Asn Lys
       370                 375
   TTGTTCCAAA ATACGTTCTT CTCTCACACG TGGTTTTCTA TCA                         1198
(2) INFORMATION POUR LA SEQ ID NO: 6:
   (i) CARACTERISTIQUES DE LA SEQUENCE:
      (A) LONGUEUR: 375 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 6:
   Leu His Pro Thr Asn Pro Glu Val Thr Met Asn Ile Ser Gln Met Ile
    1               5                  10                  15
   Thr Tyr Trp Gly Tyr Pro Ala Glu Glu Tyr Glu Val Val Thr Glu Asp
               20                  25                  30
   Gly Tyr Ile Leu Gly Ile Asp Arg Ile Pro Tyr Gly Arg Lys Asn Ser
           35                  40                  45
   Glu Asn Ile Gly Arg Arg Pro Val Ala Phe Leu Gln His Gly Leu Leu
       50                  55                  60
   Ala Ser Ala Thr Asn Trp Ile Ser Asn Leu Pro Asn Asn Ser Leu Ala
   65                  70                  75                  80
   Phe Ile Leu Ala Asp Ala Gly Tyr Asp Val Trp Leu Gly Asn Ser Arg
                   85                  90                  95
   Gly Asn Thr Trp Ala Arg Arg Asn Leu Tyr Tyr Ser Pro Asp Ser Val
               100                 105                 110
   Glu Phe Trp Ala Phe Ser Phe Asp Glu Met Ala Lys Tyr Asp Leu Pro
           115                 120                 125
   Ala Thr Ile Asp Phe Ile Leu Lys Lys Thr Gly Gln Asp Lys Leu His
       130                 135                 140
   Tyr Val Gly His Ser Gln Gly Thr Thr Ile Gly Phe Ile Ala Phe Ser
   145                 150                 155                 160
   Thr Asn Pro Lys Leu Ala Lys Arg Ile Lys Thr Phe Tyr Ala Leu Ala
                   165                 170                 175
   Pro Val Ala Thr Val Lys Tyr Thr Glu Thr Leu Leu Asn Lys Leu Met
               180                 185                 190
   Leu Val Pro Ser Phe Leu Phe Lys Leu Ile Phe Gly Asn Lys Ile Phe
           195                 200                 205
   Tyr Pro His His Phe Phe Asp Gln Phe Leu Ala Thr Glu Val Cys Ser
       210                 215                 220
   Arg Glu Thr Val Asp Leu Leu Cys Ser Asn Ala Leu Phe Ile Ile Cys
   225                 230                 235                 240
   Gly Phe Asp Thr Met Asn Leu Asn Met Ser Arg Leu Asp Val Tyr Leu
                   245                 250                 255
   Ser His Asn Pro Ala Gly Thr Ser Val Gln Asn Val Leu His Trp Ser
               260                 265                 270
   Gln Ala Val Lys Ser Gly Lys Phe Gln Ala Phe Asp Trp Gly Ser Pro
           275                 280                 285
   Val Gln Asn Met Met His Tyr His Gln Ser Met Pro Pro Tyr Tyr Asn
       290                 295                 300
   Leu Thr Asp Met His Val Pro Ile Ala Val Trp Asn Gly Gly Asn Asp
   305                 310                 315                 320
   Leu Leu Ala Asp Pro His Asp Val Asp Leu Leu Leu Ser Lys Leu Pro
                   325                 330                 335
   Asn Leu Ile Tyr His Arg Lys Ile Pro Pro Tyr Asn His Leu Asp Phe
               340                 345                 350
   Ile Trp Ala Met Asp Ala Pro Gln Ala Val Tyr Asn Glu Ile Val Ser
           355                 360                 365
   Met Met Gly Thr Asp Asn Lys
       370                 375

## Description

La présente invention a pour objet la production par les plantes de lipases préduodénales recombinantes, notamment de lipases gastriques recombinantes, et autres dérivés polypeptidiques de ces dernières possédant une activité lipasique, ainsi que leurs utilisations, notamment en tant qu'aliments fonctionnels, ou dans des compositions pharmaceutiques, ou dans des formulations enzymatiques à applications agro-alimentaires ou industrielles.

La lipase gastrique de chien (LGC) est une glycoprotéine de 379 acides aminés (AA) d'un poids moléculaire d'environ 50 kilodaltons (kDa) synthétisée sous forme d'un précurseur contenant un peptide signal à l'extrémité aminoterminale (NH₂-terminale) et sécrétée par les cellules médianes de la muqueuse fundique de l'estomac de chien (Carrière F. *et al*., 1991).

La lipase gastrique humaine (LGH) est naturellement synthétisée sous forme d'un précurseur et est décrite dans la publication de Bodmer M.W. *et al*., 1987. La protéine mature LGH est constituée de 379 acides aminés. Son peptide signal (PSLGH) est composé de 19 acides aminés.

Ces enzymes appartiennent à une famille de lipases dites "préduodénales" dont certains membres ont déjà été purifiés et parfois même clonés (Docherty A.J.P. *et al*., 1985; Bodmer M.W. *et al*., 1987; Moreau H. *et al*., 1988; Brevets européens n° 0 191 061 et n° 0 261 016).

Pendant longtemps, on a tenu pour acquis que l'hydrolyse des lipides alimentaires s'effectuait au niveau de l'intestin grêle grâce à l'action d'enzymes produites par le pancréas (Bernard C., 1849).

Des observations laissaient cependant penser que l'hydrolyse des triglycérides pouvait avoir lieu dans l'estomac par le biais d'enzymes préduodénales (Volhard, F., 1901; Shonheyder, F., and Volquartz, K., 1945). Ces enzymes, et en particulier la lipase gastrique de chien, ont des propriétés enzymatiques et physico-chimiques qui les différencient des lipases pancréatiques de mammifères. Ces différences entre lipases gastriques et pancréatiques concernent essentiellement les points suivants: poids moléculaire, composition en acides aminés, résistance à la pepsine, spécificité de substrat, pH optimum d'action, et stabilité en milieu acide.

De plus, *in vitro,* dans certaines conditions, on peut mettre en évidence une synergie d'action entre les lipases gastrique et pancréatique sur l'hydrolyse de triglycérides à chaînes longues (Gargouri, Y. *et al.,* 1989).

On connaît plusieurs situations pathologiques (mucoviscidose, insuffisance pancréatique exocrine) où les patients sont totalement ou partiellement dépourvus de sécrétion pancréatique exocrine et donc des enzymes nécessaires à l'hydrolyse des aliments (amylases, lipases, protéases). La non-absorption des graisses au niveau intestinal, et notamment des triglycérides à longues chaînes, se traduit par une augmentation très importante de la stéatorrhée chez ces patients et par un ralentissement très sensible de la prise de poids chez les jeunes malades. Pour corriger cela on administre à ces sujets des extraits pancréatiques de porc au moment des repas. L'efficacité thérapeutique de ces extraits pourrait être nettement améliorée par la co-prescription de LGC grâce à sa spécificité d'action sur les triglycérides à longues chaînes.

Dans l'article de Carrière *et al*. (1991) sont décrites la purification et la détermination de la séquence NH₂-terminale de la LGC. Un procédé permettant d'extraire cette enzyme à partir d'estomacs de chien est également décrit dans cette publication. Ce procédé consiste essentiellement à faire subir à des estomacs de chiens une macération dans un milieu acide (pH 2,5) en présence de sels hydrosolubles favorisant le relargage de la lipase dans ledit milieu. Des étapes de filtrations sur tamis moléculaire et de chromatographies par échange d'ions, permettent de purifier la LGC à homogénéité. La LGC purifiée obtenue par ces procédés est une glycoprotéine ayant un poids moléculaire de 49 000 daltons, dont 6 000 correspondent à des sucres et 43 000 à la partie protéique.

Des raisons évidentes de difficultés d'approvisionnement en estomacs de chiens empêchent tout développement de ce procédé tant au niveau du laboratoire qu'au niveau industriel. D'où la nécessité de trouver un procédé qui permette de produire la LGC en grande quantité, en faisant abstraction de l'utilisation d'estomacs de chiens.

Les séquences nucléotidique et peptidique de la LGC ont été déterminées dans le but de produire industriellement la LGC par un procédé faisant appel au génie génétique. Ces travaux ont fait l'objet de la demande internationale n° WO 94/13816 déposée le 16 décembre 1993.

Le procédé de production de LGC recombinante décrit dans cette demande internationale revendique *Escherichia coli* (*E. coli*) en tant que cellule hôte transformée pouvant produire la LGC.

Certaines difficultés rencontrées lors de la production de LGC recombinante par *E*. *coli,* notamment la nécessité de cultiver des quantités importantes d'*E*. *coli* en fermenteur, avec des coûts élevés, a conduit les inventeurs à rechercher d'autres procédés de production de cette LGC.

Les cellules de mammifères sont, *a priori,* plus adaptées à l'expression de gènes de mammifères. Leur utilisation pose cependant des problèmes de maturation des protéines. L'équipement enzymatique qui réalise la maturation post-traductionnelle est différent d'un tissu, d'un organe ou d'une espèce à l'autre. Par exemple, il a été rapporté que la maturation post-traductionnelle d'une protéine plasmatique peut être différente lorsqu'elle est obtenue à partir du sang humain ou bien lorsqu'elle est produite par une cellule recombinante comme les cellules d'ovaires de hamster chinois ou dans le lait d'un animal transgénique. Par ailleurs, les faibles niveaux d'expression obtenus avec les cellules des mammifères impliquent des cultures *in vitro* en très grands volumes à des coûts élevés. La production de protéines recombinantes dans le lait des animaux transgéniques (souris, brebis et vaches) permet de diminuer les coûts de production et de surmonter les problèmes de niveau d' expression. Cependant, il reste des problèmes d'éthique et de contaminations virales et subvirales (prions).

Pour ces raisons, la transgénèse de gènes de mammifères dans une cellule végétale pourrait offrir une voie de production en grandes quantités de protéines recombinantes nouvelles, à un coût de production réduit et sans risque de contaminations virales ou subvirales .

En 1983, plusieurs laboratoires ont découvert qu'il était possible de transférer un gène hétérologue dans le génome d'une cellule végétale (Bevan *et al.,* 1983; Herrera-Estrella *et al.,* 1983 a et b) et de régénérer des plantes transgéniques à partir de ces cellules génétiquement modifiées. Toutes les cellules de la plante possèdent alors le caractère génétiquement modifié qui est transmis à la descendance par fécondation sexuée.

Grâce à ces travaux, diverses équipes se sont intéressées à la production de protéines recombinantes de mammifères dans les cellules végétales ou dans des plantes transgéniques (Barta *et al.,* 1986; Marx, 1982). L'un des premiers résultats vraiment significatif dans ce domaine a été la production d'anticorps dans les plantes de tabac transgéniques (Hiatt *et al.,* 1989).

Pour exprimer une protéine hétérologue dans la graine, lieu de stockage des protéines chez les végétaux, l'équipe de Vandekerckhove (1989) a fusionné la séquence codant pour la leu-enképhaline au gène codant pour l'albumine 2S d'*Arabidopsis thaliana.* Avec cette construction, des colza transgéniques ont été produits qui expriment la leu-enképhaline spécifiquement dans les graines à des niveaux d'expression de l'ordre de 0,1% des protéines totales. En 1990, Sijmons et collaborateurs ont transféré le gène de la sérum albumine humaine dans des cellules de tabac et de pomme de terre. Quelle que soit l'origine des peptides signaux (humaine ou végétale), des taux de sérum albumine humaine de l'ordre de 0,02% des protéines totales ont été obtenus dans les feuilles, les tiges et les tubercules de pomme de terre.

D'autres protéines recombinantes de mammifères ont été aussi produites dans les plantes: l'antigène de surface de l'hépatite B (Mason *et al*., 1992); les interférons (De Zoeten *et al*., 1989; Edelbaum *et al*., 1992; Truve *et al*., 1993); un anticorps de souris anti-*Streptococcus mutans,* agent de la carie dentaire (Hiatt et Ma, 1992; Ma *et al*., 1994), des fragments d'anticorps scFV anti-cellules cancéreuses (Russel D., 1994), un anticorps anti-Herpès (Russel D., 1994), l'hirudine (Moloney *et al*., 1994), la toxine du choléra (Hein R., 1994) et le facteur de croissance de l'épiderme humain (E.G.F.) (Higo *et al*., 1993).

L'ensemble de ces recherches a permis de montrer que la production de protéines recombinantes de mammifères dans les cellules végétales est possible et que les mécanismes de la synthèse des protéines à partir des séquences d'ADN sont similaires chez les cellules animales et les cellules végétales. Quelques différences existent néanmoins entre les cellules végétales et animales, notamment au niveau de la maturation des glycannes polymannosidiques en glycannes complexes, ou encore au niveau des sites de clivage des peptides signaux, ne permettant pas ainsi de garantir l'obtention de protéines de mammifères actives ou suffisamment actives par transformation de cellules végétales.

Les inventeurs ont mis en évidence que l'utilisation de cellules végétales transformées par une séquence nucléotidique recombinante appropriée, permet d'obtenir de la LGC recombinante, ou de la LGH recombinante, ou des polypeptides dérivés de ces dernières, présentant une activité enzymatique suffisante pour être susceptibles d'être développés dans une application industrielle.

Le but de la présente invention est de fournir un nouveau procédé d'obtention de lipases préduodénales recombinantes de mammifères, et plus particulièrement de LGC ou LGH recombinante, par les plantes, ou de polypeptides dérivés de ces dernières, présentant une activité enzymatique, et plus particulièrement une activité lipasique, telle que lesdites lipases recombinantes, ou leurs polypeptides dérivés puissent être utilisés industriellement.

Un autre but de la présente invention est de fournir les outils pour la mise en oeuvre d'un tel procédé, notamment de nouvelles séquences nucléotidiques recombinantes, des cellules de plantes transformées génétiquement, des plantes ou parties de plantes (notamment feuilles, tiges, fruits, semences ou graines, racines) transformées génétiquement, et des fragments de ces plantes ou parties de plantes transformées génétiquement.

L'invention a également pour but de fournir de nouvelle(s) lipases préduodénales recombinante(s) de mammifères, ou tout polypeptide dérivé, enzymatiquement actifs et tels qu'obtenus à partir de cellules de plantes ou de plantes, transformées génétiquement.

L'invention a également pour but de fournir de nouvelles compositions enzymatiques susceptibles d'être utilisées dans le cadre de la mise en oeuvre de réactions enzymatiques, notamment à l'échelle industrielle.

L'invention a également pour but de fournir de nouvelles compositions pharmaceutiques, notamment dans le cadre du traitement de pathologies associées à un déficit de production de lipase dans l'organisme, telles que la mucoviscidose.

Un autre but de la présente invention est de fournir de nouveaux carburants, encore désignés biocarburants, présentant l'avantage d'être moins polluants que les carburants dérivés du pétrole, et d'être d'un coût de revient moins élevé.

L'invention est illustrée dans ce qui suit à l'aide des figures suivantes:
- la figure 1 représente la séquence nucléotidique de l'ADNc dont les nucléotides situés aux positions 1 à 1137 codent pour la LGC représentée sur la figure 2 et correspondant à SEQ ID NO 2,
- la figure 2 représente la séquence en acides aminés de la LGC et correspondant à SEQ ID NO 2,
- la figure 3 représente une séquence nucléotidique dérivée de l'ADNc représenté sur la figure 1 et correspondant à SEQ ID NO 1, les nucléotides situés aux positions 1 à 1137 de ladite séquence dérivée codant pour la LGC telle que représentée sur la figure 2 et correspondant à SEQ ID NO 2,
- la figure 4 représente la séquence nucléotidique de l'ADNc dont les nucléotides situés aux positions 47 à 1240 codent pour le précurseur de la LGH de 398 acides aminés, et les nucléotides situés aux positions 104 à 1240 codent pour la LGH mature de 379 acides aminés,
- la figure 5 représente la séquence en acides aminés de la LGH, le précurseur de la LGH étant délimité par les acides aminés situés aux positions 1 et 398, la LGH mature étant délimitée par les acides aminés situés aux positions 20 et 398,
- les figures 6, 7 et 8 représentent les résultats d'expériences d'immunodétection du type "Western" des polypeptides recombinants produits par les feuilles et graines de tabac Xanthi, les graines de colza, et par les feuilles et fruits de tomate, transformées à l'aide des séquences recombinantes selon l'invention,
- les figures 9 et 10 représentent respectivement l'analyse par électrophorèse sur gel de polyacrylamide et le résultat du transfert de ce gel sur membrane de nitrocellulose et révélation à partir d'anticorps anti-lipase gastrique de chien, de la LGC purifiée à partir de feuilles de tabac,
- la figure 11 représente un exemple de détection sur membrane des résidus glycaniques de la LGC recombinante,
- la figure 12 représente l'analyse par électrophorèse sur gel de polyacrylamide de la LGC purifiée à partir de graines de colza,
- les figures 13, 14 et 15 illustrent les différents essais effectués dans le cadre de l'obtention de méthylester oléique à partir de graines transformées selon l'invention.

La présente invention a pour objet l'utilisation d'une séquence nucléotidique recombinante contenant, d'une part, un ADNc codant pour toute lipase préduodénale de mammifères, à savoir les lipases dont les séquences nucléotidiques codant pour ces dernières présentent entre elles un pourcentage d'homologie d'au moins environ 75%, notamment d'au moins environ 77% à environ 85%, et dont les séquences en acides aminés présentent entre elles un pourcentage d'homologie d'au moins environ 70%, notamment d'au moins environ 80% à environ 90%, et présentant la propriété d'être acidorésistantes et d'être actives à un pH d'environ 1 à environ 5, et plus particulièrement à un pH d'environ 1,5 à environ 2, notamment un ADNc codant pour toute lipase gastrique de mammifères, ou un ADNc codant pour tout polypeptide dérivé des lipases préduodénales susmentionnées par addition et/ou suppression et/ou substitution d'un (ou plusieurs) acide(s) aminé(s), ce polypeptide dérivé possédant les propriétés décrites ci-dessus des lipases préduodénales, et, d'autre part, les éléments permettant à une cellule végétale de produire la lipase préduodénale codée par ledit ADNc, ou de produire un polypeptide dérivé tel que défini ci-dessus, notamment un promoteur et un terminateur de transcription reconnus par la machinerie transcriptionnelle des cellules végétales, pour la transformation de cellules de plantes en vue de l'obtention, à partir de ces cellules, ou de plantes obtenues à partir de ces dernières, d'une lipase préduodénale recombinante de mammifères sous forme d'enzyme active, ou d'un (ou plusieurs) polypeptide(s) dérivé(s) de cette dernière tel(s) que défini(s) ci-dessus.

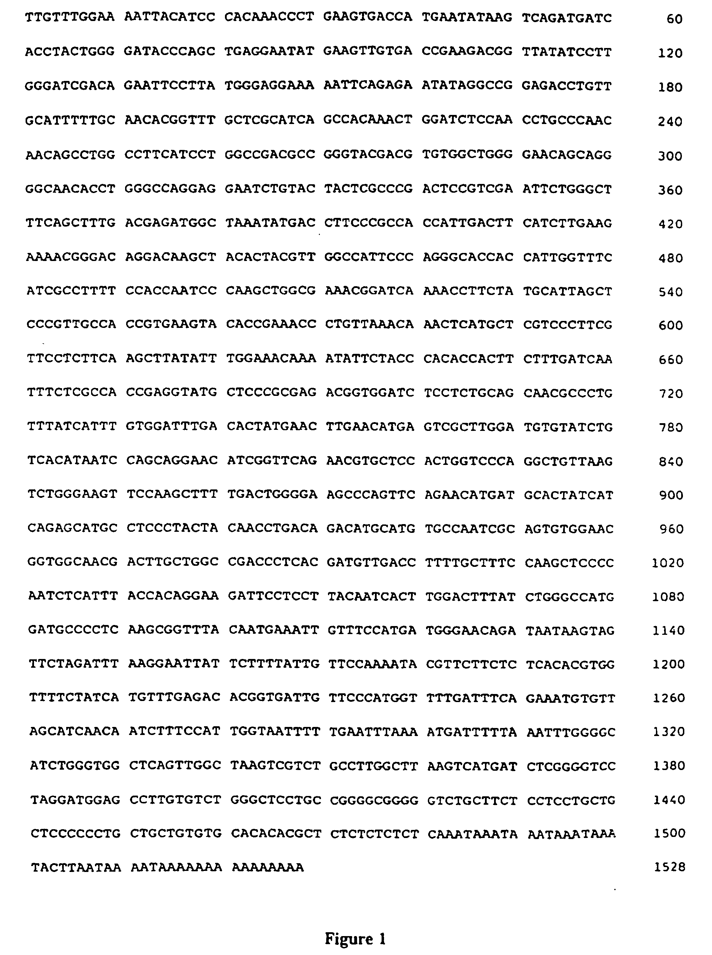

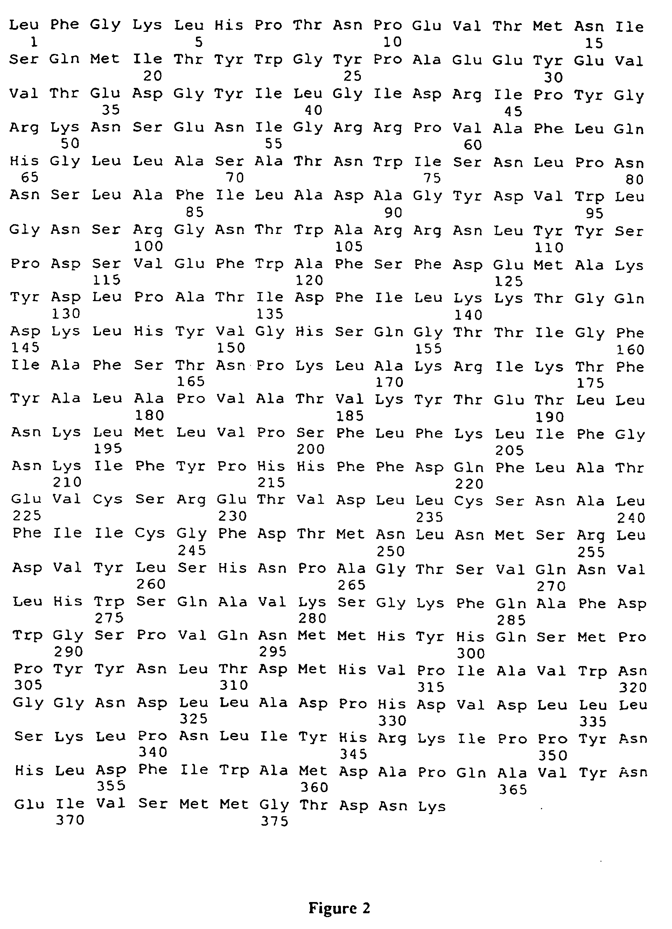

La présente invention a plus particulièrement pour objet l'utilisation d'une séquence nucléotidique recombinante contenant d'une part, l'ADNc représenté sur la figure 1 et codant pour la lipase gastrique de chien (LGC) représentée sur la figure 2, ou une séquence nucléotidique dérivée de cet ADNc, notamment par addition et/ou suppression et/ou substitution d'un (ou plusieurs) nucléotide(s), ladite séquence dérivée étant susceptible de coder pour un polypeptide dont la séquence en acides aminés est identique à celle de la LGC représentée sur la figure 2, ou pour un polypeptide dérivé de la LGC par addition et/ou suppression et/ou substitution d'un (ou plusieurs) acide(s) aminé(s), ce polypeptide dérivé présentant une activité lipasique, et d'autre part, les éléments permettant à une cellule végétale de produire le polypeptide codé par ledit ADNc ou par une séquence dérivée susmentionnée, notamment un promoteur et un terminateur de transcription reconnus par la machinerie transcriptionnelle des cellules végétales (et plus particulièrement par les ARN polymérases de ces dernières), pour la transformation de cellules de plantes en vue de l'obtention, à partir de ces cellules, ou des plantes obtenues à partir de ces dernières, de LGC recombinante sous forme d'enzyme active, ou d'un (ou plusieurs) polypeptide(s) dérivé(s) de cette dernière tel(s) que défini(s) ci-dessus.

L'invention concerne également toute séquence nucléotidique recombinante telle que décrite ci-dessus, contenant à titre d'ADNc, celui représenté sur la figure 1, ou une séquence nucléotidique dérivée de ce dernier telle que définie ci-dessus.

A ce titre, l'invention a plus particulièrement pour objet toute séquence nucléotidique recombinante telle que décrite ci-dessus, contenant l'ADNc représenté sur la figure 1 et codant pour la lipase gastrique de chien (LGC) représentée sur la figure 2.

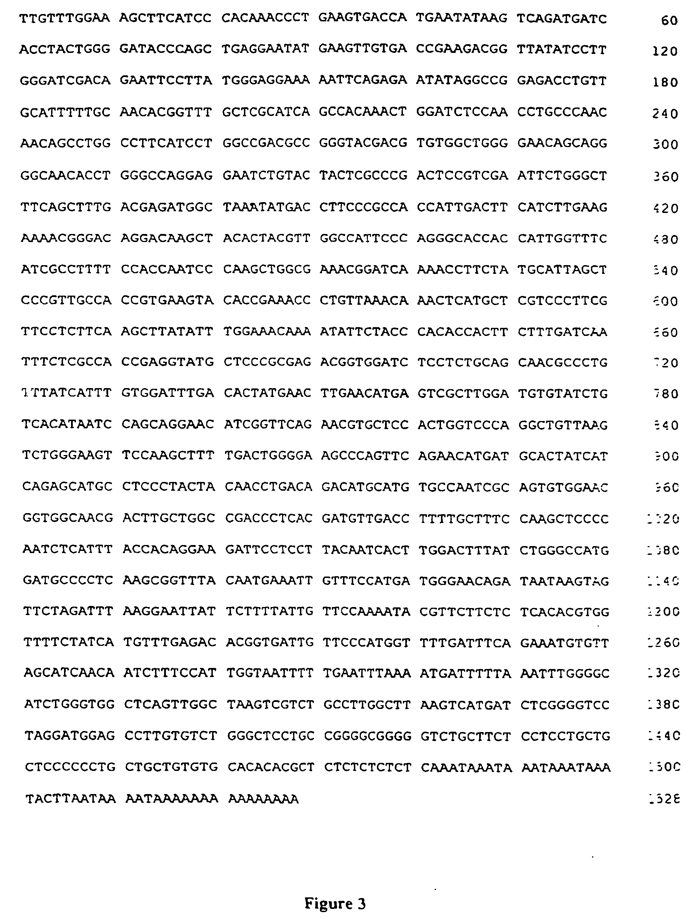

L'invention a plus particulièrement encore pour objet toute séquence nucléotidique recombinante telle que décrite ci-dessus, contenant une séquence nucléotidique dérivée de l'ADNc représenté sur la figure 1, ladite séquence nucléotidique dérivée étant telle que définie ci-dessus et codant pour la lipase gastrique de chien (LGC) représentée sur la figure 2. Une telle séquence dérivée est avantageusement celle représentée sur la figure 3, et correspond à celle représentée sur la figure 1 dans laquelle le nucléotide A en position 12 est remplacé par le nucléotide G, le nucléotide T en position 13 est remplacé par le nucléotide C, et le nucléotide A en position 15 est remplacé par le nucléotide T.

Avantageusement, les séquences nucléotidiques recombinantes selon l'invention contiennent une (ou plusieurs) séquence(s) codant pour un peptide responsable de l'adressage des polypeptides recombinants de l'invention (à savoir de la LGC recombinante ou des polypeptides dérivés susmentionnés) dans un compartiment déterminé de la cellule végétale, notamment dans le réticulum endoplasmique ou dans les vacuoles, ou bien même à l'extérieur de la cellule, dans la paroi pectocellulosique ou dans l'espace extracellulaire aussi appelé apoplasme.

Parmi les terminateurs de transcription susceptibles d'être utilisés pour la transformation de cellules de plantes dans le cadre de la présente invention, on peut citer le terminateur polyA 35S du virus de la mosaïque du chou-fleur (CaMV), décrit dans l'article de Franck. *et al.,* 1980, ou le terminateur polyA NOS, qui correspond à la région en 3' non codante du gène de la nopaline synthase du plasmide Ti d'*Agrobacterium tumefaciens* souche à nopaline (Depicker *et al.,* 1982).

A ce titre, l'invention a pour objet toute séquence nucléotidique recombinante telle que décrite ci-dessus contenant en aval dudit ADNc ou de sa séquence dérivée, le terminateur polyA 35S du CaMV, ou le terminateur polyA NOS d'*Agrobacterium tumefaciens.*

Parmi les promoteurs de transcription susceptibles d'être utilisés pour la transformation de cellules de plantes dans le cadre de la présente invention, on peut citer:
- le promoteur 35S, ou avantageusement le promoteur constitutif double 35S (pd35S) du CaMV, ces promoteurs permettant l'expression des polypeptides recombinants de l'invention dans l'ensemble de la plante obtenue à partir de cellules transformées selon l'invention, et sont décrits dans l'article de Kay *et al.,* 1987,
- le promoteur pCRU du gène de la cruciférine de radis permettant l'expression des polypeptides recombinants de l'invention uniquement dans les semences (ou graines) de la plante obtenue à partir de cellules transformées selon l'invention, et décrit dans l'article de Depigny-This *et al.,* 1992,
- les promoteurs pGEA1 et pGEA6 correspondant à la région 5' non codante ***des gènes*** de la protéine de réserve de graines, GEA1 et GEA6, respectivement, d'*Arabidopsis thaliana* (Gaubier *et al.,* 1993), et permettant une expression spécifique dans les graines,
- le promoteur chimérique super-promoteur pSP (PCT/US94/12946), constitué de la fusion d'une triple répétition d'un élément activateur transcriptionnel du promoteur du gène de l'octopine synthase d'*Agrobacterium tumefaciens,* d'un élément activateur transcriptionnel du promoteur du gène de mannopine synthase et du promoteur mannopine synthase d'*Agrobacterium tumefaciens,*
- le promoteur actine du riz suivi de l' intron actine de riz (pAR-IAR) contenu dans le plasmide pAct1-F4 décrit par McElroy ***et al. (1991)**,*
- le promoteur du gène de γzéine de maïs (pγzéine) contenu dans le plasmide pγ63 décrit dans Reina *et al.* (1990), et permettant l'expression dans l'albumen des semences de maïs.

A ce titre, l'invention a pour objet toute séquence nucléotidique recombinante telle que décrite ci-dessus, contenant en amont dudit ADNc ou de sa séquence dérivée, le promoteur constitutif double 35S (pd35S) du CaMV, ou le promoteur pCRU du gène de la cruciférine de radis, ou les promoteurs pGEA1 ou pGEA6 d'*Arabidopsis thaliana,* ou le super-promoteur pSP d'*Agrobacterium tumefaciens,* ou le promoteur pAR-IAR du riz, ou le promoteur pγzéine du maïs.

Les séquences codant pour un peptide d'adressage utilisées dans le cadre de la présente invention, peuvent être d'origine végétale, humaine ou animale.

Parmi les séquences codant pour un peptide d'adressage d'origine végétale, on peut citer:
- la séquence nucléotidique de 69 nucléotides (indiquée dans les exemples qui suivent) codant pour le prépeptide (peptide signal) de 23 acides aminés de la sporamine A chez la patate douce, ce peptide signal permettant l'entrée des polypeptides recombinants de l'invention dans le système de sécrétion des cellules végétales transformées selon l'invention (à savoir principalement dans le réticulum endoplasmique),
- la séquence nucléotidique de 42 nucléotides (indiquée dans les exemples qui suivent) codant pour le propeptide N-terminal d'adressage vacuolaire de 14 acides aminés de la sporamine A chez la patate douce, permettant l'accumulation des polypeptides recombinants de l'invention dans les vacuoles des cellules végétales transformées selon l'invention,
- la séquence nucléotidique de 111 nucléotides (indiquée dans les exemples qui suivent) codant pour le prépropeptide de 37 acides aminés de la sporamine A constitué de la partie N-terminale vers la partie C-terminale des 23 acides aminés du peptide signal susmentionné suivis par les 14 acides aminés du propeptide susmentionné, ce prépropeptide permettant l'entrée de polypeptides recombinants de l'invention dans le système de sécrétion et leur accumulation dans les vacuoles des cellules végétales transformées selon l'invention,
   les trois séquences susmentionnées étant décrites dans les articles de Murakami *et al*., 1986, et Matsuoka et Nakamura, 1991,
- le propeptide carboxyterminal de la lectine d'orge décrit notamment dans les articles de Schroeder *et al*., 1993, et de Bednarek et Ralkhel, 1991.

Parmi les séquences codant pour un peptide d'adressage d'origine humaine ou animale, on peut citer celles codant pour le peptide signal de la lipase gastrique humaine (LGH) tel que décrit dans le brevet européen n° 0 191 061, ou pour celui de la lipase gastrique de lapin (LGL) tel que décrit dans la demande de brevet européen n° 0 542 629, dont la séquence est indiquée dans les exemples qui suivent, ou pour celui de la lipase pancréatique humaine (LPH) ou encore pour celui de la lipase gastrique de chien (LGC).

On peut également citer, parmi les séquences codant pour un peptide d'adressage, celle codant pour le tétrapeptide KDEL, et permettant un adressage dans le réticulum endoplasmique.

A ce titre, l'invention a pour objet toute séquence nucléotidique recombinante telle que décrite ci-dessus, contenant une séquence codant pour tout ou partie d'un peptide signal tel que celui de la sporamine A de la patate douce, ou celui de la LGH, ou de la LGL, ou de la LGC, cette séquence codant pour un peptide signal étant située, dans ladite séquence nucléotidique recombinante, en amont dudit ADNc ou de sa séquence dérivée et en aval du promoteur utilisé, de telle sorte que le dernier acide aminé C-terminal du peptide signal soit lié au premier acide aminé N-terminal du polypeptide codé par ledit ADNc ou sa séquence dérivée, dans la protéine codée par ladite séquence nucléotidique recombinante.

L'invention a également pour objet toute séquence nucléotidique recombinante telle que décrite ci-dessus, contenant une séquence codant pour tout ou partie d'un peptide d'adressage vacuolaire, notamment celui de la sporamine A de la patate douce, cette séquence codant pour un peptide d'adressage vacuolaire étant située, dans ladite séquence nucléotidique recombinante, entre la séquence codant pour un peptide signal et celle codant pour ledit ADNc ou sa séquence dérivée, de telle sorte que le premier acide aminé N-terminal du peptide d'adressage vacuolaire soit lié au dernier acide aminé C-terminal du peptide signal, et que le dernier acide aminé C-terminal dudit peptide d'adressage soit lié au premier acide aminé N-terminal du polypeptide codé par ledit ADNc ou sa séquence dérivée, dans la protéine codée par ladite séquence nucléotidique recombinante.

L'invention a également pour objet toute séquence nucléotidique recombinante telle que décrite ci-dessus, contenant une séquence codant pour tout ou partie d'un peptide d'adressage vacuolaire, notamment celui de la lectine d'orge, cette séquence codant pour un peptide d'adressage vacuolaire étant située, dans ladite séquence nucléotidique recombinante, en aval de la séquence codant pour ledit ADNc ou sa séquence dérivée, de telle sorte que le premier acide aminé N-terminal du peptide d'adressage vacuolaire soit lié au dernier acide aminé C-terminal du polypeptide codé par ledit ADNc ou sa séquence dérivée, dans la protéine codée par ladite séquence nucléotidique recombinante.

L'invention a plus particulièrement pour objet les séquences nucléotidiques recombinantes suivantes:
- celle (désignée pd35S-PS-LGC) contenant dans le sens . 5' → 3' le promoteur pd35S du CaMV, la séquence codant pour le peptide signal de la sporamine A, cette dernière étant immédiatement suivie par la séquence nucléotidique représentée sur la figure 3, puis le terminateur polyA 35S du CaMV,
- celle (désignée pd35S-PPS-LGC) contenant dans le sens 5' → 3' le promoteur pd35S du CaMV, la séquence codant pour le prépropeptide de la sporamine A, cette dernière étant immédiatement suivie par la séquence nucléotidique représentée sur la figure 3, puis le terminateur polyA 35S du CaMV,
- celle (désignée pd35S-PSLGL-LGC) contenant dans le sens 5' → 3' le promoteur pd35S du CaMV, la séquence codant pour une partie du peptide signal de la LGL (à savoir pour une séquence constituée des 19 premiers acides aminés, indiquée dans les exemples qui suivent ci-après, les 9 nucléotides codant pour les 3 derniers acides aminés C-terminaux étant supprimés), cette dernière étant immédiatement suivie par l'ADNc représenté sur la figure 1, puis le terminateur polyA 35S du CaMV,
- celle (désignée pCRU-PS-LGC) contenant dans le sens 5' → 3' le promoteur pCRU de la cruciférine, la séquence codant pour le peptide signal de la sporamine A, cette dernière étant immédiatement suivie par la séquence nucléotidique représentée sur la figure 3, puis le terminateur polyA 35S du CaMV.
- celle (désignée pCRU-PPS-LGC) contenant dans le sens 5' → 3' le promoteur pCRU de la cruciférine, la séquence codant pour le prépropeptide de la sporamine A, cette dernière étant immédiatement suivie par la séquence nucléotidique représentée sur la figure 3, puis le terminateur polyA 35S du CaMV,
- celle (désignée pCRU-PSLGL-LGC) contenant dans le sens 5' → 3' le promoteur pCRU de la cruciférine, la séquence codant pour une partie du peptide signal de la LGL (telle que décrite ci-dessus), cette dernière étant immédiatement suivie par l'ADNc représenté sur la figure 1, ou sur la figure 3, puis le terminateur polyA 35S du CaMV,
- celle (désignée pGEA1-PSLGL-LGC) contenant dans le sens 5' → 3' le promoteur pGEA1 d'*Arabidopsis thaliana,* la séquence codant pour une partie du peptide signal de la LGL (telle que décrite ci-dessus), cette dernière étant immédiatement suivie par l'ADNc représenté sur la figure 1, ou sur la figure 3, puis le terminateur polyA 35S du CaMV,
- celle (désignée pGEA6-PSLGL-LGC) contenant dans le sens 5' → 3' le promoteur pGEA6 d'*Arabidopsis thaliana,* la séquence codant pour une partie du peptide signal de la LGL (telle que décrite ci-dessus), cette dernière étant immédiatement suivie par l'ADNc représenté sur la figure 1, ou sur la figure 3, puis le terminateur polyA 35S du CaMV,
- celle (désignée pAR-IAR-PSLGL-LGC) contenant dans le sens 5' → 3' le promoteur pAR-IAR du riz, la séquence codant pour une partie du peptide signal de la LGL (telle que décrite ci-dessus), cette dernière étant immédiatement suivie par l' ADNc représenté sur la figure 1, ou sur la figure 3, puis le terminateur polyA 35S du CaMV, ou le terminateur polyA NOS d'*Agrobacterium tumefaciens,*
- celle (désignée pγzéine-PSLGL-LGC) contenant dans le sens 5' → 3' le promoteur pγzéine du maïs, la séquence codant pour une partie du peptide signal de la LGL (telle que décrite ci-dessus), cette dernière étant immédiatement suivie par l'ADNc représenté sur la figure 1, ou sur la figure 3, puis le terminateur polyA 35S du CaMV,
- celle (désignée pγzéine-PSLGL-LGC-KDEL) contenant dans le sens 5' → 3' le promoteur pγzéine du maïs, la séquence codant pour une partie du peptide signal de la LGL (telle que décrite ci-dessus), cette dernière étant immédiatement suivie par l'ADNc représenté sur la figure 1, ou sur la figure 3, puis la séquence codant pour le tétrapeptide KDEL, puis le terminateur polyA 35S du CaMV.

Avantageusement, les séquences nucléotidiques recombinantes de l'invention contiennent également une séquence nucléotidique utilisable en tant que marqueur desdites séquences recombinantes, notamment pour différencier (et ainsi sélectionner) celles des cellules de plantes qui sont transformées par lesdites séquences recombinantes de celles qui ne le sont pas.

De préférence, une telle séquence nucléotidique utilisable en tant que marqueur desdites séquences recombinantes, est choisie parmi les gènes de résistance aux antibiotiques, notamment le gène de résistance à la kanamycine.

L'invention a également pour objet tout vecteur, notamment plasmidique, contenant une séquence nucléotidique recombinante selon l'invention, insérée en un site non essentiel pour sa réplication.

L'invention concerne également tout hôte cellulaire, notamment toute bactérie telle que *Agrobacterium tumefaciens,* transformé par un vecteur tel que défini ci-dessus.

La présente invention a également pour objet tout procédé d'obtention de LGC recombinante sous forme d'enzyme active, et/ou d'un (ou plusieurs) polypeptide(s) dérivé(s) de cette dernière, notamment par addition et/ou suppression et/ou substitution d'un (ou plusieurs) acide(s) aminé(s), ce (ou ces) polypeptide(s) dérivé(s) présentant une activité lipasique, caractérisé en ce qu'il comprend:
- la transformation de cellules végétales, de manière à intégrer, dans le génome de ces cellules, une (ou plusieurs) séquence(s) nucléotidique(s) recombinante(s) selon l'invention,
- le cas échéant, l'obtention de plantes transformées à partir des cellules transformées susmentionnées,
- la récupération de la LGC recombinante et/ou du (ou des) polypeptide(s) dérivé(s) susmentionné(s) produit(s) dans lesdites cellules ou plantes transformées susmentionnées, notamment par extraction, suivie, le cas échéant, par une purification.

Selon un mode de réalisation du procédé susmentionné de l'invention, la transformation de cellules végétales peut être réalisée par transfert de la séquence nucléotidique recombinante de l'invention dans les protoplastes, notamment après incubation de ces derniers dans une solution de polyéthylène glycol (PEG) en présence de cations divalents (Ca²⁺) selon la méthode décrite dans l'articie de Krens *et al*., 1982.

La transformation des cellules végétales peut également être réalisée par électroporation notamment selon la méthode décrite dans l'article de Fromm *et al*., 1986.

La transformation des cellules végétales peut également être réalisée par utilisation d'un canon à gène permettant la projection, à très grande vitesse, de particules métalliques recouvertes de séquences nucléotidiques recombinantes selon l'invention, délivrant ainsi des gènes à l'intérieur du noyau cellulaire, notamment selon la technique décrite dans l'article de Sanford, 1988.

Une autre méthode de transformation des cellules végétales, est celle de la micro-injection cytoplasmique ou nucléaire telle que décrite dans l'article de De La Penna *et al*., 1987.

Selon un mode de réalisation particulièrement préféré du procédé susmentionné de l'invention, les cellules végétales sont transformées par mise en présence de ces dernières avec un hôte cellulaire transformé par un vecteur selon l'invention, tel que décrit ci-dessus, ledit hôte cellulaire étant susceptible d'infecter lesdites cellules végétales en permettant l'intégration dans le génome de ces dernières, des séquences nucléotidiques recombinantes de l'invention initialement contenues dans le génome du vecteur susmentionné.

Avantageusement, l'hôte cellulaire susmentionné utilisé est *Agrobacterium tumefaciens,* notamment selon les méthodes décrites dans les articles de Bevan, 1984 et d'An *et al*., 1986, ou encore *Agrobacterium rhizogenes,* notamment selon la méthode décrite dans l'article de Jouanin *et al*., 1987.

Parmi les cellules végétales susceptibles d'être transformées dans le cadre de la présente invention, on peut citer celles du colza, tabac, maïs, pois, tomate, carotte, blé, orge, pomme de terre, soja, tournesol, laitue, riz et luzerne.

Selon un mode de réalisation du procédé susmentionné de l' invention, les cellules végétales transformées selon l'invention, sont cultivées *in vitro,* notamment en bioréacteurs selon la méthode décrite dans l'article de Brodelius, 1988, en milieu liquide, ou selon la méthode décrite dans l'article de Brodelius *et al.,* 1979, sous forme immobilisées, ou encore selon la méthode décrite dans l'article de Deno *et al.,* 1987, procédant par culture de racines transformées *in vitro.*

Les milieux des cultures *in vitro* susmentionnés sont ensuite récupérés pour en extraire, et le cas échéant purifier, notamment par chromatographie, la LGC recombinante et/ou les polypeptide(s) dérivé(s) définis ci-dessus, produits par lesdites cellules transformées cultivées *in vitro.*

Selon un mode de réalisation préféré du procédé susmentionné d'obtention de LGC recombinante et/ou de polypeptide(s) dérivé(s) selon l'invention, la transformation de cellules végétales est suivie par une étape d'obtention de plantes transformées par mise en culture desdites cellules transformées dans un milieu approprié. La LGC recombinante et/ou le (ou les) polypeptide(s) dérivé(s), produit(s) dans les cellules des plantes entières ainsi obtenues, sont récupérés par extraction réalisée à partir des plantes entières, ou de ***parties*** de ces plantes (notamment à partir des feuilles ou des tiges ***ou des fruits***)*,* ou encore à partir de semences issues de ces plantes, cette extraction étant le cas échéant suivie par une étape de purification de la LGC recombinante et/ou du (ou des) polypeptide(s) dérivé(s).

Les plantes transformées utilisées pour la récupération de la LGC recombinante et/ou du (ou des) polypeptide(s) dérivé(s) dans le cadre du procédé susmentionné, sont celles de la génération T0, à savoir celles obtenues à partir de culture de cellules transformées de l'invention sur un milieu approprié, ou avantageusement celles des générations suivantes (T1, T2 etc.) obtenues par autofécondation des plantes de la génération précédente et dans lesquelles les séquences nucléotidiques recombinantes de l'invention se reproduisent selon les lois de Mendel.

Parmi les polypeptides dérivés de la LGC recombinante susceptibles d'être obtenus dans le cadre de la mise en oeuvre d'un procédé selon l'invention, on peut citer :
- le polypeptide délimité par les acides aminés situés aux positions 55 et 379 de la figure 2, encore désigné polypeptide (Δ54) et représenté par SEQ ID NO 4, ledit polypeptide étant codé par la séquence nucléotidique représentée par SEQ ID NO 3,
- le polypeptide délimité par les acides aminés situés aux positions 5 et 379 de la figure 2, encore désigné polypeptide (Δ4) et représenté par SEQ ID NO 6, ledit polypeptide étant codé par la séquence nucléotidique représentée par SEQ ID NO 5.

L'invention a plus particulièrement pour objet tout procédé tel que décrit ci-dessus, d'obtention de la LGC recombinante représentée sur la figure 2, et le cas échéant, d'obtention d'un (ou de plusieurs) polypeptide(s) dérivé(s), notamment du polypeptide (Δ54) et/ou du polypeptide (Δ4) susmentionnés, ledit procédé étant caractérisé en ce que l'étape de transformation des cellules végétales est réalisée par intégration dans le génome de ces dernières, d'une séquence recombinante telle que décrite ci-dessus, contenant d'une part l'ADNc représenté sur la figure 1, et, d'autre part, la séquence codant pour le peptide signal de 22 acides aminés de la LGL, avantageusement celle codant pour les 19 premiers acides aminés du peptide signal de la LGL.

L'invention vise plus particulièrement un procédé d'obtention tel que décrit ci-dessus de la LGC recombinante représentée sur la figure 2, le cas échéant en association avec le polypeptide (Δ54) et/ou le polypeptide (Δ4), caractérisé en ce qu'il comprend:
- la transformation de cellules d'explants de feuilles d'une plante, par mise en présence de ces dernières avec une souche d'*Agrobacterium tumefaciens* transformée par un plasmide tel que décrit ci-dessus contenant la séquence nucléotidique recombinante pd35S-PSLGL-LGC susmentionnée, sur un milieu de culture approprié,
- sélection des explants transformés sur un milieu contenant de la kanamycine,
- obtention de plantes transformées à partir des explants transformés susmentionnés par culture de ces derniers sur des milieux appropriés,
- extraction de la LGC recombinante, et le cas échéant du polypeptide (Δ54) et/ou du polypeptide (Δ4), notamment par broyage des feuilles et/ou des graines et/ou des fruits des plantes transformées susmentionnées dans un tampon approprié, centrifugation et récupération du surnageant constituant l'extrait végétal à activité enzymatique,
- le cas échéant purification de la LGC recombinante à partir de l'extrait obtenu lors de l'étape précédente, notamment par chromatographie réalisée à partir du surnageant, ce qui conduit à l'obtention de LGC recombinante sous forme essentiellement pure.

L'invention a également pour objet l'application du procédé susmentionné à l'obtention du polypeptide (Δ54) ou du polypeptide (Δ4) sous forme essentiellement pure, par purification de ces derniers à partir de l'extrait obtenu dans le procédé susmentionné, notamment par chromatographie réalisée à partir du surnageant d'extraction.

L'invention a plus particulièrement pour objet un procédé d'obtention du polypeptide (Δ4) susmentionné, le cas échéant sous forme essentiellement pure, par mise en oeuvre du procédé décrit ci-dessus dans lequel les cellules transformées avec la séquence pd35S-PSLGL-LGC, sont des cellules d'explants de feuilles de solanacées, notamment de tabac ou de tomate.

Selon un mode de réalisation du procédé susmentionné, le polypeptide (Δ4) peut être spécifiquement obtenu par extraction à partir des feuilles de plantes de tabac transformées susmentionnées, notamment par broyage de ces feuilles dans un tampon approprié, centrifugation et récupération du surnageant. Le polypeptide (Δ4) peut ensuite être purifié à partir de l'extrait de feuilles susmentionné, contenant ledit polypeptide (Δ4), notamment par chromatographie réalisée à partir du surnageant susmentionné.

L'invention a plus particulièrement pour objet tout procédé, tel que décrit ci-dessus, d'obtention de LGC recombinante et/ou d'un (ou plusieurs) polypeptide(s) dérivé(s) de cette dernière, tels que définis ci-dessus, caractérisé en ce que l'étape de transformation des cellules végétales est réalisée par intégration, dans le génome de ces dernières, d'une séquence contenant, d'une part, la séquence nucléotidique représentée sur la figure 3, et d'autre part, la séquence codant pour le peptide signal de la sporamine A décrit ci-dessus.

Parmi les polypeptides dérivés de la LGC recombinante, susceptibles d'être obtenus dans le cadre de la mise en oeuvre du procédé décrit ci-dessus, on peut citer le polypeptide (Δ54) et/ou le polypeptide (Δ4) susmentionnés.

L'invention vise plus particulièrement un procédé d'obtention de LGC recombinante et/ou du polypeptide (Δ54) et/ou du polypeptide (Δ4) susmentionnés, caractérisé en ce qu'il comprend:
- la transformation de cellules d'explants d'une plante (notamment d'explants de feuilles), par mise en présence de ces dernières avec une souche d'*Agrobacterium tumefaciens* transformée par un plasmide tel que décrit ci-dessus contenant la séquence nucléotidique recombinante pd35S-PS-LGC et/ou la séquence pd35S-PPS-LGC,
- sélection des explants transformés sur un milieu contenant de la kanamycine,
- obtention de plantes transformées à partir des explants transformés susmentionnés par culture de ces derniers sur des milieux appropriés,
- extraction de la LGC recombinante et/ou du polypeptide (Δ54) et/ou du polypeptide (Δ4), notamment par broyage, dans un tampon approprié, des feuilles et/ou des graines et/ou des fruits des plantes transformées susmentionnées, centrifugation et récupération du surnageant constituant l'extrait végétal à activité enzymatique,
- le cas échéant, purification de la LGC recombinante et/ou du polypeptide (Δ54) et/ou du polypeptide (Δ4) à partir de l'extrait obtenu lors de l'étape précédente, notamment par chromatographie réalisée à partir du surnageant, ce qui conduit à l'obtention de la LGC recombinante et/ou du polypeptide (Δ54) et/ou du polypeptide (Δ4) sous forme essentiellement pure.

L'invention vise plus particulièrement un procédé d'obtention du polypeptide (Δ54) et/ou du polypeptide (Δ4) susmentionnés, par mise en oeuvre du procédé décrit ci-dessus dans lequel les cellules transformées sont des cellules d'explants de feuilles de solanacées, notamment de tabac ou de tomate.

Selon un mode particulier de réalisation du procédé susmentionné de l'invention, le polypeptide (Δ54) peut être spécifiquement obtenu par extraction à partir des graines de tabac transformées susmentionnées, notamment par broyage de ces graines dans un tampon approprié, centrifugation et récupération du surnageant. Le polypeptide (Δ54) peut ensuite être purifié à partir de l'extrait de graines susmentionné, contenant ledit polypeptide (Δ54), notamment par chromatographie réalisée à partir du surnageant susmentionné.

L'invention vise plus particulièrement un procédé d'obtention de LGC recombinante et/ou du polypeptide (Δ54) et/ou du polypeptide (Δ4), caractérisé en ce qu'il comprend:
- la transformation de cellules d'explants d'une plante, par mise en présence de ces dernières avec une souche d'*Agrobacterium tumefaciens* transformée par un plasmide tel que décrit ci-dessus contenant la séquence nucléotidique recombinante pCRU-PPS-LGC et/ou la séquence pCRU-PS-LGC et/ou la séquence pGEA1-PSLGL-LGC et/ou la séquence pGEA6-PSLGL-LGC et/ou la séquence pAR-IAR-PSLGL-LGC et/ou la séquence pγzéine-PSLGL-LGC et/ou la séquence pγzéine-PSLGL-LGC-KDEL,
- sélection des explants transformés sur un milieu contenant de la kanamycine,
- obtention de plantes transformées à partir des explants transformés susmentionnés par culture de ces derniers sur des milieux appropriés,
- extraction de la LGC recombinante et/ou du polypeptide (Δ54) et/ou du polypeptide (Δ4), notamment par broyage, dans un tampon approprié, des graines issues des plantes transformées susmentionnées, centrifugation et récupération du surnageant constituant l'extrait végétal à activité enzymatique,
- le cas échéant, purification de la LGC recombinante et/ou du polypeptide (Δ54) et/ou du polypeptide (Δ4), à partir de l'extrait obtenu lors de l'étape précédente, notamment par chromatographie réalisée à partir du surnageant, ce qui conduit à l'obtention de la LGC recombinante et/ou du polypeptide (Δ54) et/ou du polypeptide (Δ4), sous forme essentiellement pure.

L'invention a plus particulièrement pour objet un procédé d'obtention du polypeptide (Δ54) susmentionné, par mise en oeuvre du procédé décrit ci-dessus dans lequel les cellules transformées sont des cellules d'explants de colza, ou de tabac, et l'extraction du polypeptide (Δ54) est effectuée notamment par broyage des graines transformées.

Les cellules végétales transformées dans les procédés décrits ci-dessus sont avantageusement choisies parmi celles de tabac, colza, maïs, pois, tomate, carotte, blé, orge, pomme de terre, soja, tournesol, laitue, riz et luzerne.

L'invention vise plus particulièrement un procédé d'obtention de LGC recombinante et/ou du polypeptide (Δ54) et/ou du polypeptide (Δ4), caractérisé en ce qu'il comprend:
- la transformation de cellules de cals de maïs, par bombardement de ces dernières à l'aide d'un canon à particules, avec des plasmides contenant la séquence nucléotidique recombinante pAR-IAR-PSLGL-LGC et/ou la séquence pγzéine-PSLGL-LGC et/ou la séquence pyzéine-PSLGL-LGC-KDEL,
- sélection des cals transformés sur un milieu contenant un agent sélectif tel que la kanamycine,
- obtention de plantes de maïs transformées à partir des cals transformés susmentionnés par culture de ces derniers sur des milieux appropriés,
- extraction de la LGC recombinante et/ou du polypeptide (Δ54) et/ou du polypeptide (Δ4), notamment par broyage, dans un tampon approprié, des semences issues des plantes transformées susmentionnées, centrifugation et récupération du surnageant constituant l'extrait végétal à activité enzymatique,
- le cas échéant, purification de la LGC recombinante et/ou du polypeptide (Δ54) et/ou du polypeptide (Δ4), à partir de l'extrait obtenu lors de l'étape précédente, notamment par chromatographie réalisée à partir du surnageant, ce qui conduit à l'obtention de la LGC recombinante et/ou du polypeptide (Δ54) et/ou du polypeptide (Δ4), sous forme essentiellement pure.

L'invention a également pour objet toute cellule végétale transformée génétiquement, contenant une (ou plusieurs) séquence(s) nucléotidique(s) recombinante(s) telle(s) que décrite(s) ci-dessus, selon l'invention, intégrée(s) de façon stable dans leur génome.

L'invention concerne également toute cellule végétale transgénique telle que décrite ci-dessus, contenant un (ou plusieurs) polypeptide(s) recombinant(s) selon l'invention, tels que la LGC recombinante et/ou le polypeptide (Δ54) et/ou le polypeptide (Δ4), ladite cellule végétale étant encore désignée cellule végétale à activité enzymatique, et plus particulièrement à activité lipasique telle que définie ci-après.

L'invention a également pour objet des semences transformées génétiquement contenant une (ou plusieurs) séquence(s) nucléotidique(s) recombinante(s) telle(s) que décrite(s) ci-dessus, selon l'invention, intégrée(s) de façon stable dans leur génome.

L'invention concerne également les semences transgéniques décrites ci-dessus, et contenant un (ou plusieurs) polypeptide(s) recombinant(s) selon l'invention, tels que la LGC recombinante et/ou le polypeptide (Δ54) et/ou le polypeptide (Δ4), lesdites semences étant encore désignées semences à activité enzymatique, et plus particulièrement à activité lipasique telle que définie ci-après.

Les semences transformées selon l'invention sont celles récoltées à partir de plantes transformées génétiquement selon l'invention, ces plantes transformées étant soit celles de la génération T0 susmentionnée et obtenues par mise en culture de cellules transformées selon l'invention, soit celles des générations suivantes (T1, T2 etc...) obtenues par autofécondation ou par croisement des plantes des générations précédentes (comme indiqué ci-dessus).

L'invention a également pour objet les plantes ou ***parties*** de plantes (notamment explants, tiges, feuilles, fruits, racines, pollen, etc...) transformé(e)s génétiquement, caractérisé(e)s en ce qu'elles (ils) contiennent une (ou plusieurs) séquence(s) nucléotidique(s) recombinante(s) telle(s) que décrite(s) ci-dessus, selon l'invention, intégrée(s) de façon stable dans leur génome.

L'invention concerne également les plantes ou parties de plantes transgéniques décrits ci-dessus, contenant un (ou plusieurs) polypeptide(s) recombinant(s) selon l'invention, tels que la LGC recombinante et/ou le polypeptide (Δ54) et/ou le polypeptide (Δ4), lesdit(e)s plantes ou parties de plantes étant encore désigné(e)s plantes ou parties de plantes à activité enzymatique, et plus particulièrement à activité lipasique telle que définie ci-après.

L'invention a plus particulièrement pour objet, les plantes transformées susmentionnées, telles qu'obtenues par mise en culture de cellules ou de semences telles que décrites ci-dessus, selon l'invention.

Les plantes, ou parties de plantes, transformées selon l'invention sont avantageusement choisis parmi le colza, le tabac, le maïs, le pois, la tomate, la carotte, le blé, l'orge, la pomme de terre, le soja, le tournesol, le riz, la laitue, la luzerne et la betterave, ou les parties de ces plantes.

La présente invention a pour objet tout extrait végétal à activité enzymatique, et plus particulièrement à activité lipasique définie ci-après, tel qu'obtenu par mise en oeuvre de l'un des procédés décrits ci-dessus de l'invention, contenant, à titre d'enzymes actives, un (ou plusieurs) polypeptide(s) recombinant(s) selon l'invention, tels que la LGC recombinante et/ou le polypeptide (Δ54) et/ou le polypeptide (Δ4).

L'activité lipasique (ou lipolytique) des plantes ou ***parties*** de plantes, et des extraits végétaux à activité enzymatique de l'invention, peut être mesurée notamment selon la méthode de Gargouri (Gargouri *et al*., 1986) utilisant un triglycéride à chaîne courte (tel que la tributyrine) comme substrat. L'activité enzymatique est rapportée en unités U, une unité U correspondant à la quantité d'enzyme nécessaire pour libérer une *µ*mole d'acides gras libres par minute à 37°C dans les conditions optimales de pH.

Les extraits végétaux à activité enzymatique de l'invention sont avantageusement tels que le pourcentage en poids de polypeptides recombinants enzymatiquement actifs, représente environ 0,1% à 20%, notamment environ 1% à environ 15%, par rapport au poids total de protéines présentes dans ces extraits, ce qui correspond à des mesures d'activité enzymatique d'environ 0,5 U par g de poids frais (PF) de feuilles à environ 1000 U/g PF de feuilles, notamment d'environ 10 U/g PF à environ 300 U/g PF de feuilles, ou encore d'environ 1 U/g PF de graines à environ 5000 U/g PF, notamment à environ 10 U/g PF de graines à environ 1000 U/g PF de graines.

L'invention a plus particulièrement pour objet les extraits végétaux à activité enzymatique suivants:
* les extraits de feuilles et/ou de fruits et/ou de graines de plantes, tels qu'obtenus par transformation des cellules d'explants de ces plantes avec la séquence pd35S-PSLGL-LGC, ou la séquence pd35S-PS-LGC, ou la séquence pd35S-PPS-LGC, selon l'un des procédés décrits ci-dessus, et contenant la LGC recombinante et/ou le polypeptide (Δ54), et/ou le polypeptide (Δ4), notamment:
   - l'extrait de feuilles de tabac, tel qu'obtenu par transformation de cellules d'explants de feuilles de tabac avec la séquence pd35S-PS-LGC, ou la séquence pd35S-PPS-LGC, selon le procédé décrit ci-dessus, et contenant le polypeptide (Δ54), en association avec le polypeptide (Δ4), le pourcentage en poids du mélange de ces deux polypeptides par rapport au poids total de protéines présentes dans ledit extrait étant d'environ 0,1% à environ 20%, l'activité enzymatique dudit extrait étant d'environ 100 U/g PF à environ 300 U/g PF,
   - l'extrait de feuilles ou de fruits de tomate, tels qu'obtenus par transformation de cellules d'explants de feuilles de tomate avec la séquence pd35S-PS-LGC, ou la séquence pd35S-PPS-LGC, selon le procédé décrit ci-dessus, et contenant le polypeptide (Δ54), en association avec le polypeptide (Δ4), le pourcentage en poids du mélange de ces deux polypeptides par rapport au poids total de protéines présentes dans ledit extrait étant d'environ 0,1% à environ 20%, l'activité enzymatique dudit extrait étant d'environ 100 U/g PF à environ 300 U/g PF,
   - l'extrait de feuilles de tabac, tel qu'obtenu par transformation de cellules d'explants de feuilles de tabac avec la séquence pd35S-PSLGL-LGC, selon le procédé décrit ci-dessus, et contenant le polypeptide (Δ4), le pourcentage en poids de ce polypeptide par rapport au poids total de protéines présentes dans ledit extrait étant d'environ 0,1% à environ 20%, l'activité enzymatique dudit extrait étant d'environ 100 U/g PF à environ 300 U/g PF,
   - l'extrait de graines de tabac, tel qu'obtenu par transformation de cellules d'explants de feuilles de tabac avec la séquence pd35S-PS-LGC, ou la séquence pd35S-PPS-LGC, selon le procédé décrit ci-dessus, et contenant le polypeptide (Δ54), le pourcentage en poids du polypeptide (Δ54) par rapport au poids total de protéines présentes dans ledit extrait étant d'environ 0,1% à environ 1%, l'activité enzymatique dudit extrait étant d'environ 10 U/g PF à environ 300 U/g PF,
* les extraits de graines de plantes tels qu'obtenus par transformation de cellules d'explants de ces plantes avec la séquence pCRU-PS-LGC, ou la séquence pCRU-PPS-LGC, ou la séquence pGEA1-PSLGL-LGC, ou la séquence pGEA6-PSLGL-LGC, selon l'un des procédés décrits ci-dessus, et contenant la LGC recombinante et/ou le polypeptide (Δ54), et/ou le polypeptide (Δ4), notamment:
   - l'extrait de graines de colza, tel qu'obtenu par transformation de cellules d'explants de feuilles de colza avec la séquence pCRU-PS-LGC, ou la séquence pCRU-PPS-LGC, ou la séquence pGEA1-PSLGL-LGC, ou la séquence pGEA6-PSLGL-LGC, selon le procédé décrit ci-dessus, et contenant le polypeptide (Δ54), le pourcentage en poids du polypeptide (Δ54) par rapport au poids total de protéines présentes dans ledit extrait étant d'environ 0,1% à environ 1%, l'activité enzymatique dudit extrait étant d'environ 10 U/g PF à environ 1000 U/g PF,
* les extraits de semences de plantes tels qu'obtenus par transformation de cellules d'explants de ces plantes avec la séquence pAR-IAR-PSLGL-LGC et/ou la séquence pγzéine-PSLGL-LGC et/ou la séquence pγzéine-PSLGL-LGC-KDEL, selon l'un des procédés décrits ci-dessus, et contenant la LGC recombinante et/ou le polypeptide (Δ54), et/ou le polypeptide (Δ4), notamment:
   - l'extrait de semences de maïs, tel qu'obtenu par transformation de cellules de maïs (notamment de cals de maïs) avec la séquence pAR-IAR-PSLGL-LGC et/ou la séquence pγzéine-PSLGL-LGC et/ou la séquence pγzéine-PSLGL-LGC-KDEL, selon le procédé décrit ci-dessus, et contenant le polypeptide (Δ54), le pourcentage en poids du polypeptide (Δ54) par rapport au poids total de protéines présentes dans ledit extrait étant d'environ 0,1% à environ 1%, l'activité enzymatique dudit extrait étant d'environ 10 U/g PF à environ 1000 U/g PF.

La présente invention a également pour objet toute LGC recombinante enzymatiquement active, dont la séquence en acides aminés est celle représentée sur la figure 2, ou polypeptides dérivés de cette dernière, notamment par addition et/ou suppression et/ou substitution d'un (ou plusieurs) acide(s) aminé(s), ces polypeptides dérivés présentant une activité lipasique, tels qu'obtenus sous forme essentiellement pure par mise en oeuvre d'un des procédés décrits ci-dessus de l'invention, ces procédés comprenant une étape de purification des polypeptides recombinants de l'invention, notamment par chromatographie réalisée à partir des extraits enzymatiques décrits ci-dessus.

A titre de polypeptides dérivés de la LGC recombinante susmentionnée, l'invention a plus particulièrement pour objet les polypeptides (Δ54) et (Δ4) cités ci-dessus, dont les poids moléculaires sont respectivement d'environ 37 kDa et environ 49 kDa.

Par LGC recombinante enzymatiquement active, ou polypeptides dérivés présentant une activité lipasique, tels que mentionnés ci-dessus, on entend tout polypeptide recombinant susceptible de présenter une activité lipasique telle que mesurée selon la méthode de Gargouri citée ci-dessus.

A titre d'illustration, les polypeptides recombinants selon l'invention présentent une activité lipasique d'environ 10 U/mg de polypeptides recombinants à environ 1000 U/mg, avantageusement d'environ 100 U/mg à environ 600 U/mg.

L'invention concerne plus particulièrement la LGC recombinante telle qu'obtenue par purification de l'extrait enzymatique de feuilles ou de graines de tabac, ces feuilles ou graines provenant de plantes de tabac transformées, elles-mêmes obtenues à partir de cellules de tabac transformées avec la séquence pd35S-PSLGL-LGC selon le procédé décrit ci-dessus, ladite LGC recombinante présentant une activité lipasique telle que décrite ci-dessus.

L'invention a également pour objet le polypeptide (Δ54) et le polypeptide (Δ4) tels qu'obtenus par purification de l'extrait enzymatique de feuilles ***et***/***ou*** graines et/ou fruits de plantes, notamment de solanacées, telles que le tabac ou la tomate transformés, ces dernières étant elles-mêmes obtenues à partir de cellules de plantes transformées avec la séquence pd35S-PS-LGC, ou la séquence pd35S-PPS-LGC, ou la séquence pd35S-PSLGL-LGC, selon le procédé décrit ci-dessus, lesdits polypeptides (Δ54) et (Δ4) recombinants présentant une activité lipasique telle que décrite ci-dessus.

L'invention concerne également le polypeptide (Δ54), tel qu'obtenu par purification de l'extrait enzymatique de graines de tabac, ou de celui de graines de colza, ces graines provenant respectivement de plantes de tabac ou de colza transformées, elles-mêmes obtenues respectivement à partir de cellules de tabac ou de colza transformées avec la séquence pCRU-PS-LGC, ou la séquence pCRU-PPS-LGC, selon les procédés décrits ci-dessus, le polypeptide (Δ54) recombinant présentant une activité lipasique telle que décrite ci-dessus.

L'invention a également pour objet le polypeptide (Δ54) et le polypeptide (Δ4) tels qu'obtenus par purification de l'extrait enzymatique de graines de colza, ces graines provenant de plantes de colza transformées, elles-mêmes obtenues à partir de cellules de colza transformées avec la séquence pGEA1-PSLGL-LGC et/ou la séquence pGEA6-PSLGL-LGC, selon les procédés décrits ci-dessus, les polypeptides (Δ4) et (Δ54) recombinants présentant une activité lipasique telle que décrite ci-dessus.

L'invention a également pour objet le polypeptide (Δ54) et le polypeptide (Δ4) tels qu'obtenus par purification de l'extrait enzymatique de semences de maïs, ces graines provenant de plantes de maïs transformées, elles-mêmes obtenues à partir de cellules de maïs transformées avec la séquence pAR-IAR-PSLGL-LGC et/ou la séquence pγzéine-PSLGL-LGC et/ou la séquence pγzéine-PSLGL-LGC-KDEL, selon les procédés décrits ci-dessus, les polypeptides (Δ4) et (Δ54) recombinants présentant une activité lipasique telle que décrite ci-dessus.

Les poids moléculaires apparents des polypeptides (Δ54) et (Δ4) selon l'invention sont respectivement de 37 kDa et 49 kDa, lorsque mesurés par analyse en gel de polyacrylamide et par immunodétection après électrotransfert sur nitrocellulose (ces méthodes étant détaillées dans les exemples qui suivent de réalisation de l'invention).

L'invention concerne les anticorps dirigés contre les polypeptides recombinants de l'invention, et plus particulièrement ceux dirigés contre la LGC recombinante selon l'invention, et/ou contre le polypeptide (Δ54) et/ou contre le polypeptide (Δ4) susmentionnés, et susceptibles de reconnaître également la LGH.

De tels anticorps peuvent être obtenus par immunisation d'un animal avec ces polypeptides suivie de la récupération des anticorps formés.

Il va de soi que cette production n'est pas limitée aux anticorps polyclonaux.

Elle s'applique encore à tout anticorps monoclonal produit par tout hybridome susceptible d'être formé, par des méthodes classiques, à partir des cellules spléniques d'un animal, notamment de souris ou de rat, immunisés contre l'un des polypeptides purifiés de l'invention d'une part, et des cellules d'un myélome approprié d'autre part, et d'être sélectionné, par sa capacité à produire des anticorps monoclonaux reconnaissant le polypeptide susmentionné initialement mis en oeuvre pour l'immunisation des animaux, ainsi que la LGH.

L'invention concerne également l'utilisation de plantes, ***parties*** de plantes, cellules de plantes, ou semences transformés selon l'invention, pour l'obtention d'un (ou plusieurs) polypeptide(s) recombinant(s) selon l'invention, tels que la LGC recombinante, ou ses polypeptides dérivés tels que définis ci-dessus, notamment par mise en oeuvre d'un des procédés susmentionnés de l'invention, lesdits polypeptides recombinants étant sous forme essentiellement pure ou contenus dans des extraits végétaux à activité enzymatique tels que définis ci-dessus.

L'invention a également pour objet l'utilisation, dans le domaine de l'alimentation humaine ou animale, de plantes, ou parties de plantes, à activité enzymatique selon l'invention, ou d'extraits végétaux à activité enzymatique tels que définis ci-dessus, ou encore de polypeptides recombinants selon l'invention, tels que la LGC recombinante, ou ses polypeptides dérivés tels que définis ci-dessus.

L'invention concerne plus particulièrement l'utilisation en tant qu'aliments des plantes, ou parties de plantes, notamment des feuilles, des fruits, des semences à activité enzymatique selon l'invention.

A ce titre l'invention a plus particulièrement pour objet tout aliment constitué d'une plante à activité enzymatique telle que décrite ci-dessus, ou de parties de cette plante, notamment de feuilles ou fruits ou encore de semences issues de cette dernière, et susceptible(s) de présenter un caractère comestible chez l'Homme, ou l'animal.

L'invention vise également toute composition alimentaire contenant une (ou plusieurs) plante(s) à activité enzymatique telle(s) que décrite(s) ci-dessus, et/ou des parties de cette (ces) plante(s), notamment des feuilles et/ou des semences et/ou des fruits de cette (ces) plante(s), et/ou un (ou plusieurs) extrait(s) végétal (végétaux) à activité enzymatique tel(s) que décrit(s) ci-dessus, et/ou un (ou plusieurs) polypeptide(s) recombinant(s) de l'invention, le cas échéant en association avec un (ou plusieurs) autre(s) composé(s) comestible(s).

Avantageusement, les plantes ou parties de plantes, contenus dans la composition alimentaire susmentionnée, se présentent sous forme de broyats.

Les aliments selon l'invention, encore désignés aliments fonctionnels, ou les compositions alimentaires selon l'invention sont plus particulièrement destinés à faciliter l'absorption des graisses animales ou végétales ingérées par un individu sain ou atteint d'une ou plusieurs pathologies affectant ou non le taux de production de lipase gastrique et/ou pancréatique. A ce titre, les aliments ou compositions alimentaires de l'invention, sont avantageusement utilisés en tant que compléments nutritionnels.

L'invention a également pour objet l'utilisation de plantes, ou de parties de plantes, notamment de feuilles et/ou fruits et/ou semences, ou de cellules végétales à activité enzymatique selon l'invention, ou d'extraits végétaux à activité enzymatique tels que définis ci-dessus, ou encore de polypeptides recombinants selon l'invention, tels que la LGC recombinante, ou ses polypeptides dérivés tels que définis ci-dessus, pour l'obtention de médicaments (ou compositions pharmaceutiques) destinés à faciliter l'absorption des graisses animales ou végétales ingérées par un individu sain ou atteint d'une ou plusieurs pathologies affectant ou non le taux de production de lipase gastrique et/ou pancréatique.

Notamment, de telles compositions pharmaceutiques sont avantageusement utilisées chez les individus subissant un traitement médical altérant le mécanisme d'absorption des graisses, ou encore chez les personnes âgées.

Les compositions pharmaceutiques selon l'invention sont aussi plus particulièrement destinées au traitement des pathologies liées à l'insuffisance de lipases (notamment de lipase(s) gastrique et/ou pancréatique) dans l'organisme, et plus particulièrement de pathologies telles que la mucoviscidose, et l'insuffisance pancréatique exocrine.

L'invention a plus particulièrement pour objet toute composition pharmaceutique comprenant un (ou plusieurs) extrait(s) végétal (végétaux) à activité enzymatique(s) décrit(s) ci-dessus, et/ou un (ou plusieurs) polypeptide(s) recombinant(s) selon l'invention, le cas échéant en association avec un véhicule pharmaceutiquement acceptable.

L'invention a plus particulièrement pour objet toute composition pharmaceutique susmentionnée contenant de la LGC recombinante, et/ou le polypeptide (Δ54) et/ou le polypeptide (Δ4), sous forme essentiellement pure ou sous forme d'extraits enzymatiques tels que décrits ci-dessus.

Les compositions pharmaceutiques selon l'invention sont de préférence administrables par voie orale, et se présentent notamment sous forme de gélules, de comprimés ou de poudres à diluer.

La posologie journalière chez l'homme est avantageusement d'environ 200 mg à environ 1000 mg, répartis de préférence au moment des repas principaux, lorsque lesdites compositions pharmaceutiques contiennent des extraits enzymatiques tels que décrits ci-dessus, et d'environ 100 mg à environ 500 mg, lorsque lesdites compositions pharmaceutiques contiennent des polypeptides recombinants selon l'invention sous forme essentiellement pure.

L'invention a également pour objet l'utilisation de plantes, ou de parties de plantes, notamment de feuilles et/ou fruits et/ou semences, ou de cellules végétales à activité enzymatique selon l'invention, ou d'extraits végétaux à activité enzymatique tels que définis ci-dessus, ou encore de polypeptides recombinants selon l'invention, tels que la LGC recombinante, ou ses polypeptides dérivés tels que définis ci-dessus, pour la mise en oeuvre de réactions enzymatiques dans le domaine industriel, agro-alimentaire ou agro-industrielle, notamment dans l'industrie des corps gras, de la lipochimie et l'industrie laitière.

A ce titre, l'invention concerne tout procédé, notamment de bioconversion enzymatique, ou de biocatalyse, par mise en oeuvre d'une ou plusieurs réactions enzymatiques dans le domaine industriel, agro-alimentaire ou agro-industrielle, notamment dans l'industrie des corps gras, de la lipochimie et l'industrie laitière, ces réactions enzymatiques étant effectuées au moyen de plantes, ou de parties de plantes, notamment de feuilles et/ou fruits et/ou semences, ou de cellules végétales à activité enzymatique selon l'invention, ou d'extraits végétaux à activité enzymatique tels que définis ci-dessus, ou encore de polypeptides recombinants selon l'invention, tels que la LGC recombinante, ou ses polypeptides dérivés tels que définis ci-dessus.

L'invention a plus particulièrement pour objet des préparations enzymatiques à destination industrielle, agro-alimentaire ou agro-industrielle, susceptibles d'être utilisées dans le cadre de la mise en oeuvre d'un procédé tel que décrit ci-dessus, et comprenant un (ou plusieurs) extrait(s) végétal (végétaux) à activité enzymatique tels que définis ci-dessus, et/ou (un ou plusieurs) polypeptide(s) recombinant(s) selon l'invention, notamment la LGC recombinante et/ou le polypeptide (Δ54) et/ou le polypeptide (Δ4), le cas échéant en association avec un (ou plusieurs) additif(s) ou autre(s) enzyme(s) susceptible(s) d'être utilisé(s) dans le cadre de l'application industrielle susmentionnée.

L'invention concerne plus particulièrement l'utilisation de plantes, ou de parties de plantes, notamment de feuilles et/ou fruits et/ou semences, ou de cellules végétales à activité enzymatique selon l'invention, pour la mise en oeuvre, à l'échelle industrielle, de réactions de bioconversions enzymatiques, ou de biocatalyses, telles que hydrolyses, ou trans-estérifications enzymatiques.

Avantageusement, les plantes à activité enzymatique, ou parties de ces plantes, notamment les feuilles et/ou fruits et/ou semences, ou de cellules végétales, selon l'invention, sont utilisées à la fois en tant que source enzymatique et substrat réactionnel.

L'invention a également pour objet tout procédé de biocatalyse utilisant des plantes, ou parties de plantes, notamment des feuilles et/ou fruits et/ou semences, ou de cellules végétales à activité enzymatique selon l'invention, et plus particulièrement des plantes contenant la LGC recombinante et/ou le polypeptide (Δ54) et/ou le polypeptide (Δ4), lesdites plantes, ou parties de plantes, étant utilisées à la fois en tant que source enzymatique et substrat réactionnel.

L'invention concerne plus particulièrement l'utilisation des plantes à activité enzymatique, ou de parties de ces plantes, selon l'invention, pour l'obtention de biocarburants.

A ce titre, la présente invention a pour objet tout procédé d'obtention de biocarburant par addition d'alcool, notamment de méthanol ou d'éthanol, à un broyat de tout ou partie de plantes transformées selon l'invention, avantageusement à un broyat de graines de colza, de tournesol ou de soja, transformées selon l'invention, et récupération de biocarburant, notamment par filtration.

L'invention concerne également les esters d'acides gras végétaux tels qu'obtenus par mise en oeuvre du procédé susmentionné, notamment le méthyl ester d'acide oléique.

L'invention a également pour objet tout biocarburant tel qu'obtenu par mise en oeuvre d'un procédé tel que décrit ci-dessus, et plus particulièrement tout biocarburant susmentionné comprenant des esters d'acides gras végétaux.

L'invention vise plus particulièrement tout biocarburant tel qu'obtenu par mise en oeuvre du procédé susmentionné à partir de graines de colza, et comprenant un méthylester d'acide oléique.

L'invention vise également l'utilisation des anticorps susmentionnés dirigés contre les polypeptides recombinants de l'invention, pour la mise en oeuvre d'une méthode de détection ou de dosage de la LGC ou de la LGH dans un échantillon biologique susceptible de la contenir.

L'invention concerne plus particulièrement l'utilisation de ces anticorps pour la mise en oeuvre d'une méthode de diagnostic *in vitro* de pathologies liées à la surproduction, ou à l'inverse, à l'insuffisance, voire l'absence de production de lipase dans l'organisme.

Cette méthode de diagnostic *in vitro,* réalisée à partir d'un échantillon biologique prélevé chez un patient, comprend une étape de mise en présence de cet échantillon avec un ou plusieurs anticorps selon l'invention, suivie d'une étape de détection des éventuels complexes anticorps-LGH formés lors de l'étape précédente.

A ce titre l'invention concerne également un kit pour la mise en oeuvre d'une méthode de détection ou de diagnostic *in vitro* susmentionnée, comprenant:
- des anticorps tels que décrits ci-dessus, avantageusement marqués de manière radioactive ou enzymatique, ainsi que les réactifs pour la constitution d'un milieu propice à la réalisation de la réaction immunologique entre ces anticorps et la LGH,
- les réactifs permettant la détection des complexes immunologiques formés entre ces anticorps et la LGH.

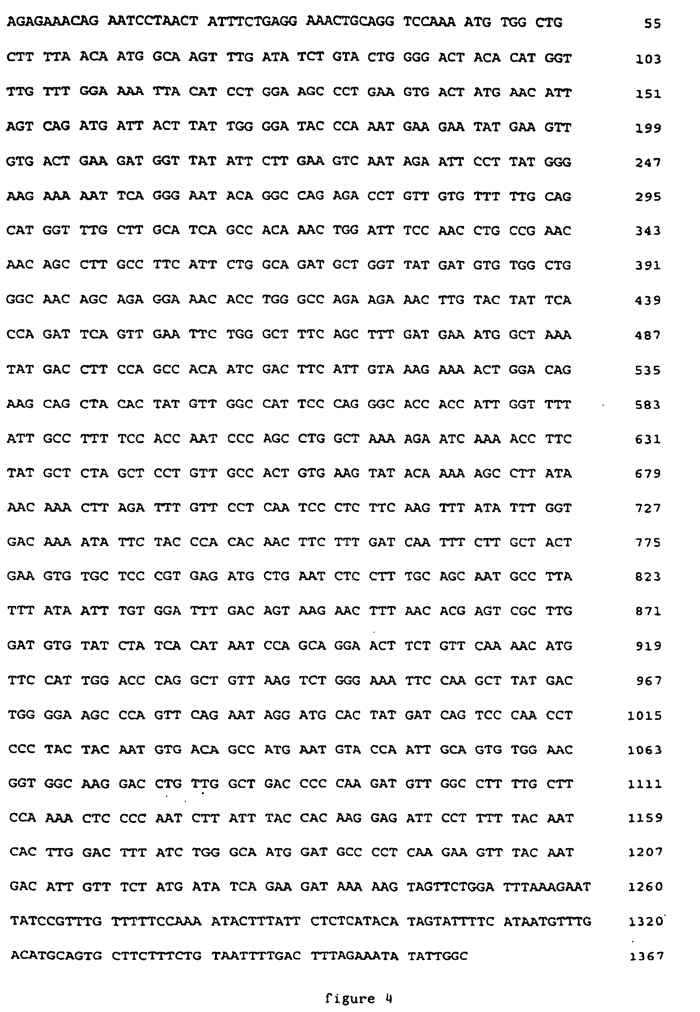

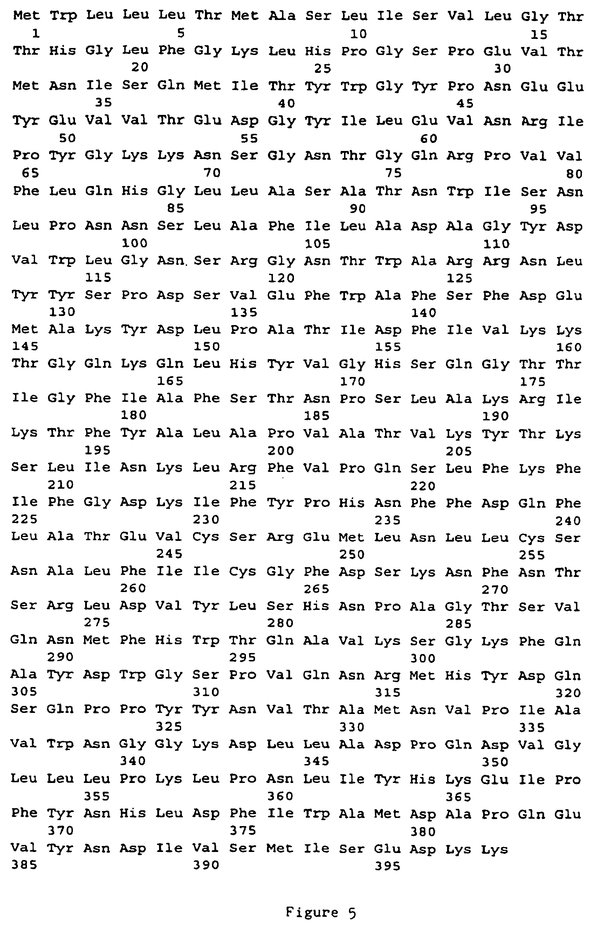

La présente invention a plus particulièrement pour objet l'utilisation d'une séquence nucléotidique recombinante contenant d'une part, l'ADNc représenté sur la figure 4 et codant pour la lipase gastrique humaine (LGH) représentée sur la figure 5, ou une séquence nucléotidique dérivée de cet ADNc, notamment par addition et/ou suppression et/ou substitution d'un (ou plusieurs) nucléotide(s), ladite séquence dérivée étant susceptible de coder pour un polypeptide dont la séquence en acides aminés est identique à celle de la LGH représentée sur la figure 5, ou pour un polypeptide dérivé de la LGH par addition et/ou suppression et/ou substitution d'un (ou plusieurs) acide(s) aminé(s), ce polypeptide dérivé présentant une activité lipasique, et d'autre part, les éléments permettant à une cellule végétale de produire le polypeptide codé par ledit ADNc ou par une séquence dérivée susmentionnée, notamment un promoteur et un terminateur de transcription reconnus par la machinerie transcriptionnelle des cellules végétales (et plus particulièrement par les ARN polymérases de ces dernières), pour la transformation de cellules de plantes en vue de l'obtention, à partir de ces cellules, ou des plantes obtenues à partir de ces dernières, de LGC recombinante sous forme d'enzyme active, ou d'un (ou plusieurs) polypeptide(s) dérivé(s) de cette dernière tel(s) que défini(s) ci-dessus.

A ce titre, l'invention concerne toute séquence nucléotidique recombinante telle que décrite ci-dessus dans le cadre de la transformation de plantes en vue de la préparation de LGC recombinante, dans laquelle la séquence nucléotidique codant pour la LGC et représentée sur la figure 1 ou la figure 3, est remplacée par la séquence nucléotidique codant pour la LGH et représentée sur la figure 4.

L'invention a plus particulièrement pour objet les séquences nucléotidiques recombinantes suivantes:
- celle (désignée pSP-PSLGH-LGH) contenant dans le sens 5' → 3' le promoteur pSP d'*Agrobacterium tumefaciens,* la séquence codant pour le peptide signal de la LGH, cette dernière étant immédiatement suivie par la séquence nucléotidique représentée sur la figure 4, puis le terminateur polyA 35S du CaMV,
- celle (désignée pSP-PSLPH-LGH) contenant dans le sens 5' → 3' le promoteur pSP d'*Agrobacterium tumefaciens,* la séquence codant pour le peptide signal de la LPH, cette dernière étant immédiatement suivie par la séquence nucléotidique représentée sur la figure 4, puis le terminateur polyA 35S du CaMV,
- celle (désignée pSP-PSLGL-LGH) contenant dans le sens 5' → 3' le promoteur pSP d'*Agrobacterium tumefaciens,* la séquence codant pour une partie du peptide signal de la LGL (telle que décrite ci-dessus), cette dernière étant immédiatement suivie par la séquence nucléotidique représentée sur la figure 4, puis le terminateur polyA 35S du CaMV.

L'invention concerne également les vecteurs et hôtes cellulaires transformés par ces vecteurs, tels que décrits ci-dessus, et contenant les séquences nucléotidiques recombinantes susmentionnées codant pour la LGH et/ou ses polypeptides dérivés.

La présente invention a également pour objet tout procédé d'obtention de LGH recombinante sous forme d'enzyme active, et/ou d'un (ou plusieurs) polypeptide(s) dérivé(s) de cette dernière, notamment par addition et/ou suppression et/ou substitution d'un (ou plusieurs) acide(s) aminé(s), ce (ou ces) polypeptide(s) dérivé(s) présentant une activité lipasique, caractérisé en ce qu'il comprend:
- la transformation de cellules végétales, de manière à intégrer, dans le génome de ces cellules, une (ou plusieurs) séquence(s) nucléotidique(s) recombinante(s) selon l'invention,
- le cas échéant, l'obtention de plantes transformées à partir des cellules transformées susmentionnées,
- la récupération de la LGH recombinante et/ou du (ou des) polypeptide(s) dérivé(s) susmentionné(s) produit(s) dans lesdites cellules ou plantes transformées susmentionnées, notamment par extraction, suivie, le cas échéant, par une purification.

L'invention a plus particulièrement pour objet tout procédé de production de LGH recombinante par mise en oeuvre d'un procédé tel que décrit ci-dessus dans le cadre de la production de LGC recombinante et/ou ses polypeptides dérivés à l'aide d'une séquence recombinante susmentionnée comprenant la séquence représentée sur la figure 4.

Parmi les polypeptides dérivés de la LGH recombinante susceptibles d'être obtenus dans le cadre de la mise en oeuvre d'un procédé selon l'invention, on peut citer :
- le polypeptide délimité par les acides aminés situés aux positions 74 et 398 de la figure 5, encore désigné polypeptide (Δ54LGH),
- le polypeptide délimité par les acides aminés situés aux positions 24 et 398 de la figure 5, encore désigné polypeptide (Δ4LGH).

L'invention vise plus particulièrement un procédé d'obtention tel que décrit ci-dessus de la LGH recombinante et/ou du polypeptide (Δ54LGH) et/ou du polypeptide (Δ4LGH), caractérisé en ce qu'il comprend:
- la transformation de cellules d'explants de feuilles d'une plante, par mise en présence de ces dernières avec une souche d'*Agrobacterium tumefaciens* transformée par un plasmide tel que décrit ci-dessus contenant la séquence nucléotidique recombinante pSP-PSLGH-LGH et/ou pSP-PSLPH-LGH et/ou pSP-PSLGL-LGH, susmentionnées, sur un milieu de culture approprié,
- sélection des explants transformés sur un milieu contenant de la kanamycine,
- obtention de plantes transformées à partir des explants transformés susmentionnés par culture de ces derniers sur des milieux appropriés,
- extraction de la LGH recombinante et/ou du polypeptide (Δ54LGH) et/ou du polypeptide (Δ4LGH), notamment par broyage des feuilles et/ou des graines et/ou des fruits des plantes transformées susmentionnées dans un tampon approprié, centrifugation et récupération du surnageant constituant l'extrait végétal à activité enzymatique,
- le cas échéant purification de la LGH recombinante et/ou du polypeptide (Δ54LGH) et/ou du polypeptide (Δ4LGH), à partir de l'extrait obtenu lors de l'étape précédente, notamment par chromatographie réalisée à partir du surnageant, ce qui conduit à l'obtention de LGH recombinante et/ou du polypeptide (Δ54LGH) et/ou du polypeptide (Δ4LGH), sous forme essentiellement pure.

L'invention concerne également toute plante ou partie de cette plante, notamment feuilles et/ou fruits et/ou semences, contenant une (ou plusieurs) séquence(s) nucléotidique(s) recombinante(s) telle(s) que décrite(s) ci-dessus, selon l'invention, intégrée(s) de façon stable dans leur génome.

La présente invention a pour objet tout extrait végétal à activité enzymatique, et plus particulièrement à activité lipasique définie ci-après, tel qu'obtenu par mise en oeuvre de l'un des procédés décrits ci-dessus de l'invention, contenant, à titre d'enzymes actives, un (ou plusieurs) polypeptide(s) recombinant(s) selon l'invention, tels que la LGH recombinante et/ou le polypeptide (Δ54LGH) et/ou le polypeptide (Δ4LGH).

L'invention a plus particulièrement pour objet les extraits de feuilles et/ou de graines de plantes, tels qu'obtenus par transformation des cellules d'explants de ces plantes avec la séquence pSP-PSLGH-LGH, ou la séquence pSP-PSLPH-LGH, ou la séquence pSP-PSLGL-LGH, selon l'un des procédés décrits ci-dessus, et contenant la LGH recombinante et/ou le polypeptide (Δ54LGH), et/ou le polypeptide (Δ4LGH), notamment:
- l'extrait de feuilles ou de graines de tabac, tel qu'obtenu par transformation de cellules d'explants de feuilles de tabac avec la séquence pSP-PSLGH-LGH, ou la séquence pSP-PSLPH-LGH, ou la séquence pSP-PSLGL-LGH, selon le procédé décrit ci-dessus, et contenant le polypeptide (Δ54LGH), en association avec le polypeptide (Δ4LGH), le pourcentage en poids du mélange de ces deux polypeptides par rapport au poids total de protéines présentes dans ledit extrait étant d'environ 0,1% à environ 20%, l'activité enzymatique dudit extrait étant d'environ 100 U/g PF à environ 300 U/g PF,
- l'extrait de feuilles de tabac, tel qu'obtenu par transformation de cellules d'explants de feuilles de tabac avec la séquence pSP-PSLGL-LGH, et contenant le polypeptide (Δ4LGH), le pourcentage en poids de ce polypeptide par rapport au poids total de protéines présentes dans ledit extrait étant d'environ 0,1% à environ 20%, l'activité enzymatique dudit extrait étant d'environ 100 U/g PF à environ 300 U/g PF.

La présente invention a également pour objet toute LGH recombinante enzymatiquement active, dont la séquence en acides aminés est celle représentée sur la figure 5, ou polypeptides dérivés de cette dernière, notamment par addition et/ou suppression et/ou substitution d'un (ou plusieurs) acide(s) aminé(s), et plus particulièrement les polypeptides (Δ54LGH) et (Δ4LGH), ces polypeptides dérivés présentant une activité lipasique, tels qu'obtenus sous forme essentiellement pure par mise en oeuvre d'un des procédés décrits ci-dessus de l'invention, ces procédés comprenant une étape de purification des polypeptides recombinants de l'invention, notamment par chromatographie réalisée à partir des extraits enzymatiques décrits ci-dessus.

Comme cela a été décrit précédemment dans le cadre de la LGC recombinante, l'invention a également pour objet :
- les anticorps polyclonaux ou monoclonaux dirigés contre la LGH recombinante ou ses polypeptides dérivés selon l'invention, et leurs applications telles que décrites ci-dessus,
- les aliments, ou compositions alimentaires, ou compositions pharmaceutiques, ou préparations enzymatiques à destination industrielle à base de plantes, ou de parties de plantes, notamment de feuilles et/ou fruits et/ou semences, ou de cellules végétales, ou d'extraits végétaux à activité enzymatique tels que définis ci-dessus, ou encore de LGH recombinante ou de ses polypeptides dérivés selon l'invention,
- tout procédé de bioconversion enzymatique, ou de biocatalyse, ou de préparation de biocarburants, tels que décrits ci-dessus, effectués à partir de LGH recombinante ou ses polypeptides dérivés selon l'invention, ou de plantes, ou de parties de plantes, notamment de feuilles et/ou fruits et/ou semences et/ou d'extraits végétaux à activité enzymatique tels que définis ci-dessus.

L'invention sera davantage illustrée dans la description détaillée qui suit d'obtention de séquences nucléotidiques recombinantes telles que décrites ci-dessus, ainsi que de plantes transformées produisant des polypeptides recombinants selon l'invention, et d'un procédé d'obtention de biocarburant.

### I. CONSTRUCTION DE GENES CHIMERIQUES CODANT POUR LA PROTEINE RECOMBINANTE DE LIPASE GASTRIQUE DE CHIEN ET PERMETTANT UNE EXPRESSION DANS LES FEUILLES ET GRAINES DE SOLANACEES.

### I-A) Construction de gènes chimériques codant pour la LGC recombinante et permettant une expression dans le tabac

L'expression dans les feuilles et les graines de tabac du gène codant pour la lipase gastrique de chien (LGC) a nécessité les séquences régulatrices suivantes:
1. le promoteur constitutif double 35S (pd35S) du CaMV (virus de la mosaïque du chou-fleur).
   Il correspond à une duplication des séquences activant la transcription situées en amont de l'élément TATA du promoteur 35S naturel (Kay *et al*., 1987);
2. la séquence terminatrice de transcription, terminateur polyA 35S, qui correspond à la région 3' non codante de la séquence du virus à ADN bicaténaire circulaire de la mosaïque du chou-fleur produisant le transcrit 35S (Franck *et al*., 1980).

Les constructions des différents plasmides via l'utilisation de techniques d'ADN recombinant (Sambrook *et al.,* 1989) dérivent de pBIOC4. Ce plasmide binaire dérive de pGA492 (An, 1986) qui contient entre les bordures droite et gauche; issues du plasmide pTiT37 d'*Agrobacterium tumefaciens,* sur son ADN de transfert, les séquences suivantes:
le promoteur constitutif du gène nos codant pour la nopaline synthase (Depicker *et al*., 1982), la séquence codante du gène nptII codant pour la néomycine phosphotransférase II (Berg et Berg, 1983) délétée de la région des 8 premiers codons dont le codon initiateur méthionine ATG et fusionnée à la séquence des 14 premiers codons de la séquence codante du gène nos (Depicker *et al.,* 1982), la séquence codante du gène nos dépourvue de la région des 14 premiers codons, le terminateur nos (Depicker *et al.,* 1982), une région contenant des sites multiples de clonage (encore désignée polylinker) (HindIII-XbaI-SacI-HpaI-KpnI-ClaI-BglII) précédant le gène cat codant pour la chloramphénicol acétyltransférase (Close et Rodriguez, 1982) et les séquences terminatrices du gène 6 du plasmide pTiA6 d'*Agrobacterium tumefaciens* (Liu *et al.,* 1993). Pour éliminer la quasi-totalité de la séquence codante du gène cat, le plasmide pGA492 a été doublement digéré par SacI (site de restriction du polylinker) et par ScaI (site de restriction présent dans la séquence du gène cat) puis soumis à l'action de l'enzyme T4 DNA polymérase (New England Biolabs) selon les recommandations du fabricant. La ligation du plasmide modifié (20 ng) a été réalisée dans un milieu réactionnel de 10 µl contenant 1 µl du tampon T4 DNA ligase x 10 (Amersham); 2,5 U d'enzyme T4 DNA ligase (Amersham) à 14°C pendant 16 heures. Les bactéries *Escherichia coli* DH5α rendues préalablement compétentes, ont été transformées (Hanahan, 1983). L'ADN plasmidique des clones obtenus, sélectionnés sur 12 µg/ml tétracycline, a été extrait selon la méthode de la lyse alcaline (Birnboim et Doly, 1979) et analysé par digestion enzymatique par des enzymes de restriction. Puis, le site de restriction HindIII de l'ADN plasmidique du clone retenu a été modifié en un site de restriction EcoRI à l'aide d'un adaptateur HindIII-EcoRI phosphorylé (Stratagene Cloning Systems). Pour réaliser cette modification, 500 ng d'ADN plasmidique du clone retenu ont été digérés par HindIII, déphosphorylés par l'enzyme phosphatase alcaline d'intestin de veau (Boehringer Mannheim) selon les recommandations du fabricant et coprécipités en présence de 1500 ng d'ADN adaptateur HindIII-EcoRI, 1/10 volume d'acétate de sodium 3M pH 4,8 et 2,5 volumes d'éthanol absolu à -80°C pendant 30 min. Après centrifugation à 12000 g pendant 30 min., l'ADN précipité a été lavé à l'éthanol 70%, séché, repris dans 8 µl d'eau, porté à 65°C pendant 10 min., puis ligué en présence de 1 µl de tampon T4 DNA ligase x 10 (Amersham) et 2,5 U d'enzyme T4 DNA ligase (Amersham) à 14°C pendant 16 heures. Après inactivation de la T4 DNA ligase à 65°C pendant 10 min., le mélange réactionnel de ligation a été digéré par EcoRI, purifié par électrophorèse sur gel d'agarose 0,8%, électroélué (Sambrook *et al*., 1989), précipité en présence de 1/10 de volume de 3M acétate de sodium pH 4,8 et de 2,5 volumes d'éthanol absolu à -80°C pendant 30 min., centrifugé à 12000 g pendant 30 min., lavé à l'éthanol 70%, séché, puis ligué comme décrit ci-dessus. Les bactéries *Escherichia coli* DH5α rendues préalablement compétentes, ont été transformées (Hanahan, 1983). L'ADN plasmidique des clones obtenus, sélectionnés sur 12 µg/ml tétracycline, a été extrait selon la méthode de la lyse alcaline (Birnboim et Doly, 1979) et analysé par digestion enzymatique par HindIII et EcoRI notamment. Le plasmide binaire résultant, qui ne possède plus que les 9 derniers codons de la séquence codante du gène cat et dont le site EcoRI est unique, a été appelé **pBIOC4.**

La cassette d'expression, constituée du promoteur pd35S et du terminateur polyA 35S, a été isolée à partir du plasmide pJIT163Δ. Le plasmide pJIT163Δ dérive du plasmide pJIT163 qui dérive lui-même du plasmide pJIT60 (Guerineau et Mullineaux, 1993). Le plasmide pJIT163 possède un codon ATG entre les sites HindIII et SalI du polylinker. Pour supprimer cet ATG et obtenir le plasmide pJIT163Δ, l'ADN plasmidique pJIT163 a été digéré doublement par HindIII et SalI, purifié par électrophorèse sur gel d'agarose 0,8%, électroélué (Sambrook *et al*., 1989), précipité en présence de 1/10 de volume de 3M acétate de sodium pH 4,8 et de 2,5 volumes d'éthanol absolu à -80°C pendant 30 min., centrifugé à 12000 g pendant 30 min., lavé à l'éthanol 70%, séché, soumis à l'action de l'enzyme Klenow (New England Biolabs) selon les recommandations du fabricant, déprotéinisé par extraction avec 1 volume de phénol: chloroforme: alcool isoamylique (25:24:1) puis 1 volume de chloroforme: alcool isoamylique (24: 1), précipité en présence de 1/10 volume de 3M acétate de sodium pH 4,8 et de 2,5 volumes d'éthanol absolu à -80°C pendant 30 min., centrifugé à 12000 g pendant 30 min., lavé à l'éthanol 70%, séché, et enfin, ligué en présence de 1 *µ*l du tampon T4 DNA ligase x 10 (Amersham) et 2,5 U d'enzyme T4 DNA ligase (Amersham) à 14°C pendant 16 heures. Les bactéries *Escherichia coli* DH5α rendues préalablement compétentes, ont été transformées (Hanahan, 1983). L'ADN plasmidique des clones obtenus, sélectionnés sur 50 *µ*g/ml ampicilline a été extrait selon la méthode de la lyse alcaline (Birnboim et Doly, 1979) et analysé par digestion enzymatique par des enzymes de restriction. Pour isoler la cassette d'expression constituée du promoteur pd35S et du terminateur polyA 35S (fragment SacI-XhoI), l'ADN plasmidique du clone pJIT163Δ retenu a été digéré par SacI et XhoI. Le fragment SacI-XhoI, portant la cassette d'expression a été purifié par électrophorèse sur gel d'agarose 0,8%, électroélué (Sambrook *et al.,* 1989), précipité en présence de 1/10 de volume de 3M acétate de sodium pH 4,8 et de 2,5 volumes d'éthanol absolu à -80°C pendant 30 min., centrifugé à 12000 g pendant 30 min., lavé à l'éthanol 70%, séché, puis soumis à l'action de l'enzyme Mung Bean Nuclease (New England Biolabs) selon les recommandations du fabricant. Cet insert purifié (200 ng) a été cloné dans l'ADN plasmidique de **pBIOC4** (20 ng) digéré par EcoRI, traité par l'enzyme Mung Bean Nuclease et déphosphorylé par l'enzyme phosphatase alcaline d'intestin de veau (Boehringer Mannheim) selon les recommandations du fabricant. La réaction de ligation a été effectuée dans 20 *µ*l en présence de 2 *µ*l de tampon T4 DNA ligase x 10 (Amersham), de 2 *µ*l de 50% polyéthylène glycol 8000 et de 5 U d'enzyme T4 DNA ligase (Amersham) à 14°C pendant 16 heures. Les bactéries *Escherichia coli* DH5α rendues préalablement compétentes, ont été transformées (Hanahan, 1983). L'ADN plasmidique des clones obtenus, sélectionnés sur 12 µl/ml tétracycline, a été extrait selon la méthode de la lyse alcaline (Birnboim et Doly, 1979) et analysé par digestion enzymatique par des enzymes de restriction. Le plasmide résultant a été appelé **pBIOC21.**

La lipase gastrique de chien (LGC) est naturellement synthétisée sous forme d'un précurseur. La protéine mature LGC est constituée de 379 acides aminés. L'ADN complémentaire de la LGC a été cloné aux sites BglII et SalI du vecteur d'expression p**R**U303 conduisant au vecteur pDGL5.303 décrit dans la demande internationale n° WO 94/13816. Il a été utilisé pour la construction des plasmides binaires **pBIOC25** contenant PS-LGC et **pBIOC26** contenant PPS-LGC où la séquence codant pour la LGC mature est précédée de celle codant pour un peptide signal (PS) ou un prépropeptide (PPS c'est-à-dire un peptide signal suivi des séquences N-terminales d'adressage vacuolaire) d'origine végétale respectivement. Les séquences PS et PPS, constituées respectivement de 23 et 37 acides aminés, sont celles d'une protéine de réserve des racines tubérisées de patate douce: la sporamine A (Murakami *et al.,* 1986; Matsukoa et Nakamura, 1991).

Pour simplifier les fusions entre la séquence LGC mature et celle des signaux d'adressage, PS ou PPS, le plasmide pDGL5.303 a été modifié par l'introduction d'un site de restriction HindIII supplémentaire dans les quatrième et cinquième codons de la séquence LGC mature par mutagénèse dirigée par PCR en utilisant 2 oligodésoxynucléotides, 5' caggagatc TTG TTT GGA AAG CTT CAT CCC 3' (contenant le site BglII unique dans le plasmide et apportant le site HindIII supplémentaire) et 5' CAT ATT CCT CAG CTG GGT ATC 3' (contenant le site PvuII unique dans le plasmide). L'amplification par PCR du fragment BgIII-PvuII a été réalisée dans 100 µl de milieu réactionnel comprenant 10 µl de tampon Taq DNA polymérase x 10 (500 mM KCl, 100 mM Tris-HCl, pH 9,0 et 1% Triton x 100), 6 µl de 25 mM MgCl₂, 3 µl de 10 mM dNTP (dATP, dCTP, dGTP et dTTP), 100 pM de chacun des 2 oligodésoxynucléotides décrits ci-dessus, 5 ng d'ADN matrice (vecteur d'expression pRU303 renfermant l'ADN complémentaire de la LGC), 2,5 U de Taq DNA polymérase (Promega) et 2 gouttes d'huile de vaseline. L'ADN a été dénaturé à 94°C pendant 5 min., soumis à 30 cycles constitués chacun de 1 min. de dénaturation à 94°C. 1 min. d'hybridation à 50°C et de 1 min. d'élongation à 72°C, puis l'élongation à 72°C a été poursuivie pendant 5 min. Cette réaction PCR a été réalisée dans la machine "DNA Thermal Cycler" de PERKIN ELMER CETUS. L'huile a été éliminée par extraction au chloroforme. Puis, les fragments d'ADN contenus dans le milieu réactionnel ont été précipités en présence de 1/10 volume de 3M acétate de sodium pH 4,8 et de 2,5 volumes d'éthanol absolu à -80°C pendant 30 min., centrifugés à 12000 g pendant 30 min., lavés à l'éthanol 70%, séchés et digérés par les 2 enzymes de restriction BglII et PvuII. Les fragments d'ADN digérés issus de PCR ont été purifiés par électrophorèse sur gel d'agarose 2%, électroélués (Sambrook *et al*., 1989), précipités en présence de 1/10 de volume de 3M acétate de sodium pH 4,8 et de 2,5 volumes d'éthanol absolu à -80°C pendant 30 min., centrifugés à 12000 g pendant 30 min., lavés à l'éthanol 70%, séchés, puis ligués à l'ADN plasmidique du vecteur pDGL5.303 doublement digéré par BglII et PvuII, purifié par électrophorèse sur gel d'agarose 0,8%, électroélué, soumis à la précipitation alcoolique, séché et déphosphorylé par l'enzyme phosphatase alcaline d'intestin de veau (Boehringer Mannheim) selon les recommandations du fabricant. La ligation a été réalisée avec 100 ng du vecteur déphosphorylé décrit ci-dessus et 50 ng de fragments d'ADN digérés issus de l'amplification par PCR décrits ci-dessus dans un milieu réactionnel de 10 µl en présence de 1 µl de tampon T4 DNA ligase x 10 (Amersham) et de 2,5 U d'enzyme T4 DNA ligase (Amersham) à 14°C pendant 16 heures. Les bactéries *Escherichia coli* DH5α rendues préalablement compétentes, ont été transformées (Hanahan, 1983). L'ADN plasmidique des clones obtenus, sélectionnés sur 50 µg/ml ampicilline, a été extrait selon la méthode de la lyse alcaline (Birnboim et Doly, 1979) et analysé par digestion enzymatique par des enzymes de restriction. L'ADN plasmidique de certains clones retenus a été vérifié par séquençage à l'aide du kit de séquençage T7™ commercialisé par Pharmacia selon la méthode des didésoxynucléotides (Sanger *et al*., 1977). L'introduction de ce site de restriction HindIII ne modifie pas le code génétique de la LGC. En effet, la séquence LGC naturelle AAA TTA (Lys-Leu) devient AAG CTT (Lys-Leu). Le plasmide résultant a été appelé pBIOC22 et renferme la séquence de la protéine LGC mature correspondant à: **LEU**: premier codon de la LGC mature, **AMB**: codon stop.

### a. CONSTRUCTION DU PLASMIDE BINAIRE pBIOC25 CONTENANT PS-LGC.

Le plasmide pBIOC22 a été digéré totalement par BglII et partiellement par HindIII afin de supprimer la séquence codant le polypeptide Leu-Phe-Gly-Lys (4 premiers acides aminés) de la protéine LGC mature. Cette séquence a été remplacée par celle codant pour le peptide signal PS de 23 acides aminés (*ATG AAA GCC TTC ACA CTC GCT CTC TTC TTA GCT CTT TCC CTC TAT CTC CTG CCC AAT CCA GCC CAT TCC*) fusionnée à celle des 4 premiers codons de la séquence codant pour la protéine LGC mature ("PS-4 premiers codons de LGC mature"). La séquence "PS-4 premiers codons de LGC mature" a été amplifiée par PCR à partir du plasmide pMAT103 (Matsuoka et Nakamura, 1991) à l'aide des 2 oligodésoxynucléotides suivants 5' cagg**agatctg***ATG AAA GCC TTC ACA CTC GC* 3' et 5' G ATG AAG CTT TCC AAA CAA *GGA ATG GGC TGG ATT GGG CAG* G 3', en suivant le protocole d'amplification PCR décrit ci-dessus dans le paragraphe I. Après double digestion enzymatique par BgIII et HindIII, les fragments d'ADN issus de l'amplification PCR ont été purifiés par électrophorèse sur gel d'agarose 2%, électroélués (Sambrook *et al.,* 1989), précipités en présence de 1/10 de volume de 3M acétate de sodium pH 4,8 et de 2,5 volumes d'éthanol absolu à -80°C pendant 30 min., centrifugés à 12000 g pendant 30 min., lavés à l'éthanol 70%, séchés, puis ligués à l'ADN plasmidique de pBIOC22 doublement digéré par BgIII et HindIII, purifié par électrophorèse sur gel d'agarose 0,8%, électroélué (Sambrook *et al*., 1989), soumis à la précipitation alcoolique, séché et déphosphorylé par l'enzyme phosphatase alcaline d'intestin de veau (Boehringer Mannheim) selon les recommandations du fabricant. La ligation a été réalisée avec 100 ng du vecteur déphosphorylé décrit ci-dessus et 50 ng de fragments d'ADN digérés issus de l'amplification PCR décrits ci-dessus dans un milieu réactionnel de 10 µl en présence de 1 µl de tampon T4 DNA ligase x 10 (Amersham) et de 2,5 U d'enzyme T4 DNA ligase (Amersham) à 14°C pendant 16 heures. Les bactéries *Escherichia coli* DH5α rendues préalablement compétentes, ont été transformées (Hanahan, 1983). L'ADN plasmidique des clones obtenus, sélectionnés sur 50 µg/ml ampicilline, a été extrait selon la méthode de la lyse alcaline (Birnboim et Doly, 1979) et analysé par digestion enzymatique par des enzymes de restriction. L'ADN plasmidique de certains clones retenus a été vérifiée par séquençage à l'aide du kit de séquençage T7™ commercialisé par Pharmacia selon la méthode des didésoxynucléotides (Sanger *et al.,* 1977). Les séquences du PS et de LGC mature ont été clonées en maintenant leurs phases de lecture ouverte. La séquence de clivage entre les séquences du PS et de LGC mature est Ser-Leu. Le plasmide résultant a été appelé **pBIOC23.** A partir de pBIOC23, le fragment BglII-XbaI portant la séquence de PS-LGC a été isolé par double digestion enzymatique par BglII et XbaI, purification par électrophorèse sur gel d'agarose 0,8%, électroélution (Sambrook *et al*., 1989), précipitation alcoolique, séchage. Puis, ce fragment d'ADN a été traité par l'enzyme Klenow selon les recommandations du fabricant et ligué à l'ADN plasmidique de pBIOC21 digéré au site HindIII, traité à la Klenow et déphosphorylé par l'enzyme phosphatase alcaline d'intestin de veau (Boehringer Mannheim) selon les recommandations du fabricant. La ligation a été réalisée avec 20 ng du vecteur déphosphorylé décrit ci-dessus et 200 ng de fragments d'ADN contenant PS-LGC décrits ci-dessus dans un milieu réactionnel de 20 *µ*l en présence de 2 *µ*l de tampon T4 DNA ligase x 10 (Amersham), de 2 *µ*l de 50% polyéthylène glycol 8000 et de 5 U d'enzyme T4 DNA ligase (Amersham) à 14°C pendant 16 heures. Les bactéries *Escherichia coli* DH5α rendues préalablement compétentes, ont été transformées (Hanahan, 1983). L'ADN plasmidique des clones obtenus, sélectionnés sur 12 *µ*g/ml tétracycline, a été extrait selon la méthode de la lyse alcaline (Birnboim et Doly, 1979) et analysé par digestion enzymatique par des enzymes de restriction. Le clone résultant a été appelé **pBIOC25.** La séquence nucléotidique du fragment codant pour la protéine recombinante PS-LGC a été vérifiée par séquençage à l'aide du kit de séquençage T7™ commercialisé par Pharmacia selon la méthode des didésoxynucléotides (Sanger *et al.,* 1977). L'ADN plasmidique du vecteur binaire pBIOC25 a été introduit par transformation directe dans la souche LBA4404 d'*Agrobacterium tumefaciens* selon le procédé de Holsters *et al.* (1978). La validité du clone retenu a été vérifiée par digestion enzymatique de l'ADN plasmidique introduit.

### b. CONSTRUCTION DU PLASMIDE BINAIRE pBIOC26 CONTENANT PPS-LGC.

Le plasmide **pBIOC22** a été digéré totalement par BgIII et partiellement par HindIII afin de supprimer la séquence codant le polypeptide Leu-Phe-Gly-Lys de la protéine LGC mature. Cette séquence a été remplacée par celle codant pour le peptide signal PPS de 37 acides aminés (*ATG AAA GCC TTC* *ACA CTC GCT CTC TTC TTA GCT CTT TCC CTC TAT CTC CTG CCC AAT CCA GCC CAT TCC AGG TTC AAT CCC ATC CGC CTC CCC ACC ACA CAC GAA CCC GCC*) fusionnée à celle des 4 premiers codons de la protéine LGC mature ("PPS-4 premiers codons de LGC mature"). La séquence "PPS-4 premiers codons de LGC mature" a été amplifiée par PCR à partir du plasmide pMAT103 (Matsuoka et Nakamura, 1991) à l'aide des 2 oligodésoxynucléotides suivants, 5' cagg**agatctg***ATG AAA GCC TTC ACA CTC GC* 3' et 5' G ATG AAG CTT TCC AAA CAA *GGC GGG TTC GTG TGT GGT TG* 3', en suivant le protocole d'amplification PCR décrit ci-dessus dans le paragraphe I. Après double digestion enzymatique par BglII et HindIII, les fragments d'ADN issus de l'amplification PCR ont été purifiés par électrophorèse sur gel d'agarose 2%, électroélués (Sambrook *et al.,* 1989), précipités en présence de 1/10 de volume de 3M acétate de sodium pH 4,8 et de 2,5 volumes d'éthanol absolu à -80°C pendant 30 min., centrifugés à 12000 g pendant 30 min., lavés à l'éthanol 70%, séchés, puis ligués à l'ADN plasmidique de pBIOC22 doublement digéré par BglII et HindIII, purifié par électrophorèse sur gel d'agarose 0,8%, électroélué, soumis à la précipitation alcoolique, séché et déphosphorylé par l'enzyme phosphatase alcaline d'intestin de veau (Boehringer Mannheim) selon les recommandations du fabricant. La ligation a été réalisée avec 100 ng du vecteur déphosphorylé décrit ci-dessus et 50 ng de fragments d'ADN digérés issus de l'amplification PCR décrits ci-dessus dans un milieu réactionnel de 10 *µ*l en présence de 1 *µ*l de tampon T4 DNA ligase x 10 (Amersham) et de 2,5 U d'enzyme T4 DNA ligase (Amersham) à 14°C pendant 16 heures. Les bactéries *Escherichia coli* DH5α rendues préalablement compétentes, ont été transformées (Hanahan, 1983). L'ADN plasmidique des clones obtenus, sélectionnés sur 50 *µ*g/ml d'ampicilline, a été extrait selon la méthode de la lyse alcaline (Birnboim et Doly, 1979), et analysé par digestion enzymatique par des enzymes de restriction.

L'ADN plasmidique de certains des clones retenus a été vérifié par séquençage à l'aide du kit de séquençage T7™ commercialisé par Pharmacia selon la méthode des didésoxynucléotides (Sanger *et al.,* 1977). Les séquences du PPS et de LGC mature ont été clonées en maintenant leurs phases de lecture ouvertes. La séquence de clivage entre les deux séquences est Ala-Leu. Le plasmide résultant a été appelé **pBIOC24.** A partir de pBIOC24, le fragment BglII-XbaI portant la séquence de PPS-LGC a été isolé par double digestion enzymatique par BglII et XbaI, purifié par électrophorèse sur gel d'agarose 0,8%, électroélué (Sambrook *et al.,* 1989), précipité à l'alcool, séché. Puis, ce fragment d'ADN a été traité par l'enzyme Klenow selon les recommandations du fabricant et ligué à l'ADN plasmidique de pBIOC21 digéré au site HindIII, traité à la Klenow et déphosphorylé par l'enzyme phosphatase alcaline d'intestin de veau (Boehringer Mannheim) selon les recommandations du fabricant. La ligation a été réalisée avec 20 ng du vecteur déphosphorylé décrit ci-dessus et 200 ng de fragments d'ADN contenant PPS-LGC décrits ci-dessus dans un milieu réactionnel de 20 µl en présence de 2 µl de tampon T4 DNA ligase x 10 (Amersham), de 2 µl de 50% polyéthylène glycol 8000 et de 5 U d'enzyme T4 DNA ligase (Amersham) à 14°C pendant 16 heures. Les bactéries *Escherichia coli* DH5α rendues préalablement compétentes, ont été transformées (Hanahan, 1983). L'ADN plasmidique des clones obtenus, sélectionnés sur 12 µg/ml tétracycline, a été extrait selon la méthode de la lyse alcaline (Birnboim et Doly, 1979) et analysé par digestion enzymatique par des enzymes de restriction. Le clone résultant a été appelé **pBIOC26.** La séquence nucléotidique du fragment codant pour la protéine recombinante PPS-LGC a été vérifiée par séquençage à l'aide du kit de séquençage T7™ commercialisé par Pharmacia selon la méthode des didésoxynucléotides (Sanger *et al.,* 1977). L'ADN plasmidique du vecteur binaire pBIOC26 a été introduit par transformation directe dans la souche LBA4404 d'*Agrobacterium tumefaciens* selon le procédé de Holsters *et al.* (1978). La validité du clone obtenu a été vérifiée par digestion enzymatique de l'ADN plasmidique introduit.

### c. CONSTRUCTION DU PLASMIDE BINAIRE pBIOC41 CONTENANT PSLGL-LGC.

La lipase gastrique de lapin est synthétisée sous forme d'un précurseur composé d'un peptide signal de 22 acides aminés, situé à l'extrémité NH₂-terminale et précédant la séquence polypeptidique de la lipase mature, le clone pJO101 contenant l'ADNc complet codant pour la lipase gastrique de lapin est décrit dans la demande de brevet européen n° 92 403 055.4 déposée le 12.11.92 par l'Institut de Recherche Jouveinal" et s'intitulant "Acides nucléiques codant pour la lipase gastrique de lapin et dérivés polypeptidiques, leur utilisation pour la production de ces polypeptides, et compositions pharmaceutiques à base de ces derniers".

L'alignement des séquences polypeptidiques de la lipase gastrique de chien et du précurseur de la lipase gastrique de lapin a montré que la séquence LFGK est présente dans les deux protéines. Dans la séquence polypeptidique de la lipase de lapin déterminée à partir de la protéine naturelle purifiée (Moreau *et al.,* 1988), les trois premiers résidus L, F et G sont absents et font partie du peptide signal de 22 acides aminés de la LGL. De ce fait, le peptide signal de la lipase gastrique de lapin dépourvu de ces trois acides aminés communs a été fusionné à la séquence protéique mature de la lipase gastrique de chien. Sa séquence polypeptidique est constituée des 19 acides aminés suivants: MWVLFMVAALLSALGTTHG.

Le plasmide pBIOC22 a donc été digéré totalement par BgIII et partiellement par HindIII afin de supprimer la séquence codant le polypeptide Leu-Phe-Gly-Lys (4 premiers acides aminés) de la protéine LGC mature. Cette séquence a été remplacée par celle codant pour le peptide signal ***PSLGL*** de la lipase gastrique de lapin de 19 acides aminés (*ATG TGG GTG CTT TTC ATG GTG GCA GCT TTG CTA TCT GCA CTT GGA ACT ACA CAT GGT*) fusionnée à celle des 4 premiers codons de la protéine LGC mature ("***PSLGL***-4 premiers codons de LGC mature"). La séquence "PSLGL-4 premiers codons de LGC mature" a été amplifiée par PCR à partir du plasmide pJO101 à l'aide des 2 oligodésoxynucléotides suivants: 5' agg**agatct**caaca*ATG TGG GTG CTT TTC ATG GTG* 3' et 5' G ATG AAG CTT TCC AAA CAA *ACC ATG TGT AGT TCC AAG TG* 3', en suivant le protocole d'amplification PCR décrit ci-dessus dans le paragraphe I. Après double digestion enzymatique par BgIII et HindIII, les fragments d'ADN issus de l'amplification PCR ont été purifiés par électrophorèse sur gel d'agarose 2% , électroélués (Sambrook *et al.,* 1989), précipités en présence de 1/10 de volume de 3M acétate de sodium pH 4,8 et de 2,5 volumes d'éthanol absolu à -80°C pendant 30 min., centrifugés à 12000 g pendant 30 min., lavés à l'éthanol 70%, séchés, puis ligués à l'ADN plasmidique de pBIOC22 doublement digéré par BgIII et HindIII, purifié par électrophorèse sur gel d'agarose 0,8%, électroélué (Sambrook *et al*., 1989), soumis à la précipitation alcoolique, séché et déphosphorylé par l'enzyme phosphatase alcaline d'intestin de veau (Boehringer Mannheim) selon les recommandations du fabricant. La ligation a été réalisée avec 100 ng du vecteur déphosphorylé décrit ci-dessus et 50 ng de fragments d'ADN digérés issus de l'amplification PCR décrits ci-dessus dans un milieu réactionnel de 10 µl en présence de 1 µl de tampon T4 DNA ligase x 10 (Amersham) et de 2,5 U d'enzyme T4 DNA ligase (Amersham) à 14°C pendant 16 heures. Les bactéries *Escherichia coli* DH5α rendues préalablement compétentes, ont été transformées (Hanahan, 1983). L'ADN plasmidique des clones obtenus, sélectionnés sur un milieu contenant 50 µg/ml d'ampicilline, a été extrait selon la méthode de la lyse alcaline (Birnboim et Doly, 1979) et analysé par digestion enzymatique par des enzymes de restriction. L'ADN plasmidique de certains clones retenus a été vérifié par séquençage à l'aide du kit de séquençage T7™ commercialisé par Pharmacia selon la méthode des didésoxynucléotides (Sanger *et al.,* 1977). Les séquences du PSLGL et de la LGC mature ont été clonées en maintenant leurs phases de lecture ouvertes (c'est-à-dire, de telle sorte qu'elles constituent une phase ouverte de lecture unique). La séquence de clivage entre les séquences PSLGL et LGC mature est Gly-Leu. Le plasmide résultant a été appelé **pBIOC40.** A partir de **pBIOC40,** le fragment BglII-XbaI portant la séquence PSLGL-LGC a été isolé par double digestion enzymatique par BglII et XbaI, purification par électrophorèse sur gel d'agarose 0,8%, électroélution (Sambrook *et al.,* 1989), précipitation alcoolique, séchage. Puis, ce fragment d'ADN a été traité par l'enzyme Klenow selon les recommandations du fabricant et ligué à l'ADN plasmidique de pBIOC21 digéré au site HindIII, traité à la Klenow et déphosphorylé par l'enzyme phosphatase alcaline d'intestin de veau (Boehringer Mannheim) selon les recommandations du fabricant. La ligation a été réalisée avec 20 ng du vecteur déphosphorylé décrit ci-dessus et 200 ng de fragments d'ADN contenant PSLGL-LGC décrits ci-dessus dans un milieu réactionnel de 20 *µ*l en présence de 2 *µ*l de tampon T4 DNA ligase x 10 (Amersham), de 2 *µ*l de 50% polyéthylène glycol 8000 et de 5 U d'enzyme T4 DNA ligase (Amersham) à 14°C pendant 16 heures. Les bactéries *Escherichia coli* DH5α rendues préalablement compétentes, ont été transformées (Hanahan, 1983). L'ADN plasmidique des clones obtenus, sélectionnés sur un milieu contenant 12 *µ*g/ml de tétracycline, a été extrait selon la méthode de la lyse alcaline (Birnboim et Doly, 1979) et analysé par digestion enzymatique par des enzymes de restriction. Le clone résultant a été appelé **pBIOC41.** La séquence nucléotidique des fragments codant pour la protéine recombinante PSLGL-LGC a été vérifiée par séquençage à l'aide du kit de séquençage T7™ commercialisé par Pharmacia selon la méthode des didésoxynucléotides (Sanger *et al*., 1977). L'ADN plasmidique du vecteur binaire pBIOC41 a été introduit par transformation directe dans la souche LBA4404 d'*Agrobacterium tumefaciens* selon le procédé de Holsters *et al*. (1978). La validité du clone retenu a été vérifiée par digestion enzymatique de l'ADN plasmidique introduit.

### I-B) Construction de gènes chimériques codant pour la LGC recombinante et permettant une expression dans la tomate.

Les constructions utilisées sont les mêmes que celles citées pour la transformation génétique du tabac, à savoir les plasmides **pBIOC25, pBIOC26** et **pBIOC41.**

### II. CONSTRUCTION DE GENES CHIMERIQUES CODANT POUR LA PROTEINE RECOMBINANTE DE LIPASE GASTRIQUE DE CHIEN ET PERMETTANT UNE EXPRESSION DANS LES GRAINES DE COLZA.

### a) Construction du plasmide binaire pBIOC28 contenant le promoteur pCRU.

L'expression dans les graines de colza de la lipase gastrique de chien (LGC) a nécessité l'insertion de l'ADNc codant pour la LGC entre les séquences régulatrices suivantes:
1. le promoteur pCRU correspondant à la région 5' non codante du gène de la protéine de réserve de graines, la CRUCIFERINE A de radis (Depigny-This *et al.,* 1992) et permettant une expression spécifique dans les graines;
2. la séquence terminatrice de transcription, terminateur polyA 35S, qui correspond à la région 3' non codante de la séquence du virus à ADN bicaténaire circulaire de la mosaïque du chou-fleur produisant le transcrit 35S (Franck *et al.,* 1980).

Pour obtenir un plasmide binaire similaire à **pBIOC21** mais dont le promoteur pd35S a été remplacé par le promoteur pCRU, le fragment "EcoRI traité à la Klenow-BamHI", contenant le promoteur pCRU a été isolé à partir du plasmide pBI221-CRURSP dérivant de pBI221 (commercialisé par Clontech) par remplacement du promoteur 35S par le promoteur pCRU.

Le fragment "EcoRI traité à la Klenow-BamHI" portant le promoteur pCRU a été purifié par électrophorèse sur gel d'agarose 0,8%, électroélué (Sambrook *et al.,* 1989), soumis à la précipitation alcoolique, séché et ligué à l'ADN plasmidique de pJIT163 (décrit dans le paragraphe I.), digéré par KpnI, traité à la T4 DNA Polymérase (New England Biolabs) selon les recommandations du fabricant, puis digéré par BamHI, purifié par électrophorèse sur gel d'agarose 0,8%, électroélué (Sambrook *et al.,* 1989), soumis à la précipitation alcoolique, séché et déphosphorylé par l'enzyme phosphatase alcaline d'intestin de veau (Boehringer Mannheim) selon les recommandations du fabricant. La ligation a été réalisée avec 20 ng du vecteur déphosphorylé décrit ci-dessus et 200 ng de fragments d'ADN "EcoRI traité à la Klenow-BamHI" décrits ci-dessus dans un milieu réactionnel de 20 µl en présence de 2 µl de tampon T4 DNA ligase x 10 (Amersham), de 2 µl de 50% polyéthylène glycol 8000 et de 5 U d'enzyme T4 DNA ligase (Amersham) à 14°C pendant 16 heures. Les bactéries *Escherichia coli* DH5α rendues préalablement compétentes, ont été transformées (Hanahan, 1983). L'ADN plasmidique des clones obtenus, sélectionnés sur un milieu contenant 50 µg/ml d'ampicilline, a été extrait selon la méthode de la lyse alcaline (Birnboim et Doly, 1979) et analysé par digestion enzymatique par des enzymes de restriction. Le plasmide résultant a été appelé **pBIOC27.**

La cassette d'expression, constituée du promoteur pCRU et du terminateur polyA 35S a été isolée à partir de **pBIOC27** par digestion totale XhoI suivie d'une digestion partielle EcoRI. Elle a été purifiée par électrophorèse sur gel d'agarose 0,8%, électroéluée (Sambrook *et al.,* 1989), soumise à la précipitation alcoolique, séchée, traitée à la Klenow (New England Biolabs) selon les recommandations du fabricant et liguée à l'ADN plasmidique de pBIOC24 au site EcoRI traité à la Klenow et déphosphorylé par l'enzyme phosphatase alcaline d'intestin de veau (Boehringer Mannheim) selon les recommandations du fabricant. La ligation a été réalisée avec 20 ng du vecteur déphosphorylé décrit ci-dessus et 200 ng de fragments d'ADN XhoI-EcoRI décrits ci-dessus dans un milieu réactionnel de 20 µl en présence de 2 µl de tampon T4 DNA ligase x 10 (Amersham), de 2 µl de 50% polyéthylène glycol 8000 et de 5 U d'enzyme T4 DNA ligase (Amersham) à 14°C pendant 16 heures. Les bactéries *Escherichia coli* DH5α rendues préalablement compétentes, ont été transformées (Hanahan, 1983). L'ADN plasmidique des clones obtenus, sélectionnés sur un milieu contenant 12 µg/ml de tétracycline, a été extrait selon la méthode de la lyse alcaline (Birnboim et Doly, 1979) et analysé par digestion enzymatique par des enzymes de restriction. Le plasmide résultant a été appelé **pBIOC28.**

### b) Construction du plasmide binaire pBIOC29 contenant PPS-LGC.

L'isolement du fragment BgIII-XbaI portant la séquence PPS-LGC à partir de **pBIOC24** a déjà été décrit en 1-A-b. Ce fragment a été ligué à l'ADN plasmidique de **pBIOC28** au site EcoRI traité à la Klenow et déphosphorylé par l'enzyme phosphatase alcaline d'intestin de veau (Boehringer Mannheim) selon les recommandations du fabricant. La ligation a été réalisée avec 20 ng du vecteur déphosphorylé décrit ci-dessus et 200 ng de fragments d'ADN BgIII-XbaI contenant PPS-LGC dans un milieu réactionnel de 20 µl en présence de tampon T4 DNA ligase x 10 (Amersham), de 2 µl de 50% polyéthylène glycol 8000 et de 5 U d'enzyme T4 DNA ligase (Amersham) à 14°C pendant 16 heures. Les bactéries *Escherichia coli* DH5α rendues préalablement compétentes, ont été transformées (Hanahan, 1983). L'ADN plasmidique des clones obtenus, sélectionnés sur un milieu contenant 12 µg/ml de tétracycline, a été extrait selon la méthode de la lyse alcaline (Birnboim et Doly, 1979) et analysé par digestion enzymatique par des enzymes de restriction. Le plasmide résultant a été appelé **pBIOC29.** La séquence nucléotidique de la protéine recombinante PPS-LGC a été vérifiée par séquençage à l'aide du kit de séquençage T7™ commercialisé par Pharmacia selon la méthode des didésoxynucléotides (Sanger *et al.,* 1977). L'ADN plasmidique du vecteur binaire pBIOC29 a été introduit par transformation directe dans la souche LBA4404 d'*Agrobacterium tumefaciens* selon le procédé de Holsters *et al.* (1978). La validité du clone retenu a été vérifiée par digestion enzymatique de l'ADN plasmidique introduit.

### c) Construction des plasmides binaires pBIOC90 et pBIOC91 contenant le promoteur pGEA1D.

L'expression dans les graines de colza du gène animal codant pour la lipase gastrique de chien (LGC) a nécessité les séquences régulatrices suivantes :
1. le promoteur pGEA1 correspondant à la région 5' non codante du gène de la protéine de réserve de graines, GEA1 d'*Arabidopsis thaliana* (Gaubier *et al.,* 1993), et permettant une expression spécifique dans les graines ;
2. la séquence terminatrice de transcription, terminateur polyA 35S, qui correspond à la région en 3' non codante de la séquence du virus à ADN bicaténaire circulaire de la mosaïque du chou-fleur produisant le transcrit 35S (Franck *et al*., 1980).

Pour obtenir le plasmide binaire **pBIOC90** similaire à **pBIOC21** mais dont le promoteur pd35S a été remplacé par le promoteur pGEA1D, le fragment HindIII - BamHI traité à la Klenow, contenant le promoteur pGEA1, a été isolé à partir du plasmide pGUS2-pGEA1. Le clone pGUS-2-pGEA1 dérivant de pBI221 par remplacement du promoteur p35S par le promoteur pGEA1, comporte 2 ATG en phase : ATG du gène GEA1 (Em2) et ATG du gène gus. L'ATG du gène GEA1 a été détruit. Le fragment d'ADN compris entre le site SalI et les séquences en amont de l'ATG du gène GEA1 du clone pGUS-2-pGEA1 a donc été amplifié par PCR à l'aide des 2 oligonucléotides : 5' CAAACGTGTACAATAGCCC 3' et 5' CCCGGGGATCCTTTTTTG 3'. La température d'hybridation a été adaptée. Le fragment amplifié par PCR a été digéré par SalI et BamHI, purifié par électrophorèse sur gel d'agarose 2%, électroélué (Sambrook *et al*., 1989), précipité en présence de 1/10 de volume de 3M acétate de sodium pH4,8 et de 2,5 volumes d'éthanol absolu à -80°C pendant 30 min., centrifugé à 12000g pendant 30 min., lavé à l'éthanol 70%, séché, puis ligué à l'ADN plasmidique de pGUS-2-GEA1 doublement digéré par SalI et BamHI, purifié par électrophorèse sur gel d'agarose 0,8%, électroélué (Sambrook *et al*., 1989), soumis à la précipitation alcoolique, séché. La ligation a été réalisée avec 100 ng du vecteur et 50 ng de fragments d'ADN digérés issus de l'amplification PCR, décrits ci-dessus, dans un milieu réactionnel de 10 *µ*l en présence de 1 *µ*l de tampon T4 DNA ligase x 10 (Amersham) et de 2,5 U d'enzyme T4 DNA ligase (Amersham) à 14°C pendant 16 heures. Les bactéries, *Escherichia coli* DH5α rendues préalablement compétentes, ont été transformées (Hanahan, 1983). L'ADN plasmidique des clones obtenus, sélectionnés sur 50 *µ*g/ml ampicilline, a été extrait selon la méthode de la lyse alcaline (Birnboim et Doly, 1979) et analysé par digestion enzymatique par des enzymes de restriction. Certains des clones retenus ont été vérifiés par séquençage à l'aide du kit de séquençage T7™ commercialisé par Pharmacia selon la méthode des didésoxynucléotides (Sanger et al., 1977). Le clone résultant a été appelé pGUS-2-pGEA1D.

Le fragment HindIII - BamHI portant le promoteur pGEA1D isolé à partir de pGUS-2-pGEA1D, traité à la Klenow selon les recommandations du fabricant (Biolabs), a été purifié par électrophorèse sur gel d'agarose 0,8%, électroélué (Sambrook *et al.,* 1989), soumis à la précipitation alcoolique, séché et ligué à l'ADN plasmidique de **pBIOC21** aux sites KpnI et HindIII traités par l'enzyme T4 DNA polymérase (Biolabs) selon les recommandations du fabricant. La ligation a été réalisée avec 20 ng de **pBIOC21** déphosphorylé et 200 ng de fragments d'ADN XhoI-EcoRI décrits ci-dessus dans un milieu réactionnel de 20 *µ*l en présence de 2 *µ*l de tampon T4 DNA ligase x 10 (Amersham), de 2 *µ*l de 50% polyethylène glycol 8000 et de 5 U d'enzyme T4 DNA ligase (Amersham) à 14°C pendant 16 heures. Les bactéries, *Escherichia coli* DH5α rendues préalablement compétentes, ont été transformées (Hanahan, 1983). L'ADN plasmidique des clones obtenus, sélectionnés sur 12 µg/ml tétracycline, a été extrait selon la méthode de la lyse alcaline (Birnboim et Doly, 1979) et analysé par digestion enzymatique par des enzymes de restriction. Le plasmide résultant a été appelé **pBIOC90.**

Pour obtenir le plasmide binaire **pBIOC91,** la cassette d'expression "pGEA1D - tNOS" , isolée à partir de pBSII-pGEA1D, a été introduite dans le plasmide binaire gSCV1.2 lui-même obtenu par clonage du fragment HindIII portant la cassette d'expression "p35S - nptII - tNOS" décrite par Fromm et al. (1986) au site HindIII de pSCV1 construit par Edwards G.A. en 1990 en suivant les procédés usuels de clonage.

Le plasmide pBSII-pGEAID a été obtenu en deux étapes :
- d'une part, le fragment SacI-EcoRI portant tNOS (terminateur du gène de la nopaline synthase) d'*Agrobacterium tumefaciens,* traité à l'enzyme T4 DNA polymérase (Biolabs) selon les recommandations du fabricant et purifié, a été cloné au site EcoRV de pBSIISK+ commercialisé par Stratagene, déphosphorylé par l'enzyme phosphatase alcaline d'intestin de veau (Boehringer Mannheim) selon les recommandations du fabricant. La ligation a été réalisée avec 20 ng du vecteur déphosphorylé et 200 ng de fragments d'ADN contenant tNOS décrits ci-dessus dans un milieu réactionnel de 20 µl en présence de 2 µl de tampon T4 DNA ligase x 10 (Amersham), de 2 µl de 50% polyethylène glycol 8000 et de 5 U d'enzyme T4 DNA ligase (Amersham) à 14°C pendant 16 heures. Les bactéries, *Escherichia coli* DH5α rendues préalablement compétentes, ont été transformées (Hanahan, 1983). L'ADN plasmidique des clones obtenus, sélectionnés sur 50 µg/ml ampicilline, a été extrait selon la méthode de la lyse alcaline (Birnboim et Doly, 1979) et analysé par digestion enzymatique par des enzymes de restriction. Certains des clones retenus ont été vérifiés par séquençage à l'aide du kit de séquençage T7™ commercialisé par Pharmacia selon la méthode des didésoxynucléotides (Sanger et al., 1977). Le plasmide résultant a été appelé pBSII-tNOS.
- d'autre part, le fragment portant pGEA1D digéré doublement par HindIII traité à l'enzyme Klenow et BamHI, purifié, a été cloné aux sites "XbaI traité à la Klenow et BamHI" du plasmide pBSII-tNOS. La ligation a été réalisée avec 100 ng du vecteur décrit ci-dessus et 50 ng de fragments d'ADN décrits ci-dessus dans un milieu réactionnel de 10 µl en présence de 1 µl de tampon T4 DNA ligase x 10 (Amersham) et de 2,5 U d'enzyme T4 DNA ligase (Amersham) à 14°C pendant 16 heures. Les bactéries, *Escherichia coli* DH5α rendues préalablement compétentes, ont été transformées (Hanahan, 1983). L'ADN plasmidique des clones obtenus, sélectionnés sur 50 µg/ml ampicilline, a été extrait selon la méthode de la lyse alcaline (Birnboim et Doly, 1979) et analysé par digestion enzymatique par des enzymes de restriction. Le plasmide résultant a été appelé pBSII-pGEA1D.

Puis, la cassette d'expression "pGEA1D - tNOS" portée par le fragment XbaI-HindIII traité à la Klenow, a été clonée au site SmaI de pSCV 1.2 déphosphorylé par l'enzyme phosphatase alcaline d'intestin de veau (Boehringer Mannheim) selon les recommandations du fabricant. La ligation a été réalisée avec 20 ng de pSCV1.2 déphosphorylé et 200 ng de fragments portant la cassette d'expression "pGEA1D - tNOS", dans un milieu réactionnel de 20 µl en présence de 2 µl de tampon T4 DNA ligase x 10 (Amersham), de 2 µl de 50% polyethylène glycol 8000 et de 5 U d'enzyme T4 DNA ligase (Amersham) à 14°C pendant 16 heures. Les bactéries, *Escherichia coli* DH5α rendues préalablement compétentes, ont été transformées (Hanahan, 1983). L'ADN plasmidique des clones obtenus, sélectionnés sur 50 µg/ml ampicilline, a été extrait selon la méthode de la lyse alcaline (Birnboim et Doly, 1979) et analysé par digestion enzymatique par des enzymes de restriction. Le plasmide résultant a été appelé **pBIOC91.**

### d) Construction du plasmide binaire pBIOC92 contenant le promoteur pGEA6D.

L'expression dans les graines de colza du gène animal codant pour la lipase gastrique de chien (LGC) a nécessité les séquences régulatrices suivantes :
1. le promoteur pGEA6 correspondant à la région 5' non codante du gène de la protéine de réserve de graines, GEA6 d'*Arabidopsis thaliana* (Gaubier *et al.,* 1993), et permettant une expression spécifique dans les graines;
2. la séquence terminatrice de transcription, terminateur polyA 35S, qui correspond à la région en 3' non codante de la séquence du virus à ADN bicaténaire circulaire de la mosaïque du chou-fleur produisant le transcrit 35S (Franck *et al.,* 1980).

Pour obtenir le plasmide binaire **pBIOC92** similaire à **pBIOC21** mais dont le promoteur pd35S a été remplacé par le promoteur pGEA6D, le fragment EcoRI - BamHI traité à la Klenow, contenant le promoteur pGEA6, a été isolé à partir du plasmide pGUS2-pGEA6. Le clone pGUS-2-pGEA6 dérivant de pUC18 comporte 2 ATG en phase : ATG du gène GEA6 (Em6) et ATG du gène gus. L'ATG du gène GEA6 a été détruit. Le fragment d'ADN compris entre le site AccI et les séquences en amont de l'ATG du gène GEA6 du clone pGUS-2-pGEA6 a donc été amplifié par PCR à l'aide des 2 oligonucléotides : 5' AAGTACGGCCACTACCACG 3' et 5' CCCGGGGATCCTGGCTC 3'. La température d'hybridation a été adaptée.

Le fragment amplifié par PCR a été digéré par AccI et BamHI, purifié par électrophorèse sur gel d'agarose 2%, électroélué (Sambrook *et al.,* 1989), précipité en présence de 1/10 de volume de 3M acétate de sodium pH4,8 et de 2,5 volumes d'éthanol absolu à -80°C pendant 30 min., centrifugé à 12000g pendant 30 min., lavé à l'éthanol 70%, séché, puis ligué à l'ADN plasmidique de pGUS-2-GEA6 doublement digéré par AccI et BamHI. purifié par électrophorèse sur gel d'agarose 0,8%, électroélué (Sambrook *et al.,* 1989), soumis à la précipitation alcoolique, séché. La ligation a été réalisée avec 100 ng du vecteur décrit ci-dessus et 50 ng de fragments d'ADN digérés issus de l'amplification PCR décrits ci-dessus dans un milieu réactionnel de 10 µl en présence de 1 µl de tampon T4 DNA ligase x 10 (Amersham) et de 2,5 U d'enzyme T4 DNA ligase (Amersham) à 14°C pendant 16 heures. Les bactéries, *Escherichia coli* DH5α rendues préalablement compétentes, ont été transformées (Hanahan, 1983). L'ADN plasmidique des clones obtenus, sélectionnés sur 50 µg/ml ampicilline, a été extrait selon la méthode de la lyse alcaline (Birnboim et Doly, 1979) et analysé par digestion enzymatique par des enzymes de restriction. Certains des clones retenus ont été vérifiés par séquençage à l'aide du kit de séquençage T7™ commercialisé par Pharmacia selon la méthode des didésoxynucléotides (Sanger *et al*., 1977). Le clone résultant a été appelé pGUS-2-pGEA6D.

Le fragment EcoRI - BamHI portant le promoteur pGEA6D isolé à partir de pGUS-2-pGEA6D, traité à la Klenow selon les recommandations du fabricant (Biolabs), a été purifié par électrophorèse sur gel d'agarose 0,8%, électroélué (Sambrook *et al.,* 1989), soumis à la précipitation alcoolique, séché et ligué au site XhoI traité par l'enzyme Klenow de l'ADN plasmidique de **pBIOC21** modifié. Le plasmide **pBIOC21** modifié a été obtenu par double digestion par HindIII traité à la Klenow et KpnI de **pBIOC21** pour déléter le fragment portant le promoteur pd35S et le remplacer par le fragment KpnI-EcoRV portant le polylinker composé des sites KpnI-XhoI-SaII-ClaI-HindIII-BamHI-SmaI-EcoRI-EcoRV de pBSIISK+.

La ligation a été réalisée avec 20 ng de **pBIOC21** modifié déphosphorylé et 200 ng de fragments d'ADN EcoRI-BamHI, décrits ci-dessus, dans un milieu réactionnel de 20 *µ*l en présence de 2 *µ*l de tampon T4 DNA ligase x 10 (Amersham), de 2 *µ*l de 50% polyethylène glycol 8000 et de 5 U d'enzyme T4 DNA ligase (Amersham) à 14°C pendant 16 heures. Les bactéries, *Escherichia coli* DH5α rendues préalablement compétentes, ont été transformées (Hanahan, 1983). L'ADN plasmidique des clones obtenus, sélectionnés sur 12 *µ*g/ml tétracycline, a été extrait selon la méthode de la lyse alcaline (Birnboim et Doly, 1979) et analysé par digestion enzymatique par des enzymes de restriction. Le plasmide résultant a été appelé **pBIOC92.**

### e) Construction des plasmides binaires pBIOC93, pBIOC94, pBIOC95, pBIOC96 et pBIOC97 contenant PSLGL-LGC.

Le plasmide **pBIOC40** est décrit ci-dessus. Ce plasmide contient le fragment BgIII-XbaI portant la séquence PSLGL-LGC.

Ce fragment a été isolé par double digestion enzymatique par BgIII et XbaI, purifié par électrophorèse sur gel d'agarose 0,8%, électroélué, soumis à la précipitation alcoolique, séché, traité à la Klenow et ligué à **pBIOC28** (décrit ci-dessus) digéré par EcoRI traité à la Klenow et déphosphorylé ; à **pBIOC90** digéré par EcoRI traité à la Klenow et déphosphorylé ; à **pBIOC91** digéré par SmaI et déphosphorylé ; à **pBIOC92** digéré par HindIII traité à la Klenow et déphosphorylé pour résulter en **pBIOC93, pBIOC94, pBIOC95** et **pBIOC96** respectivement.

Le plasmide **pBIOC97** résulte du clonage du fragment KpnI-EcoRV portant la cassette d'expression "pGEA6D-PSLGL-LGC-t35S", traité par l'enzyme T4 DNA polymérase, purifié par électrophorèse sur gel d'agarose 0,8%, électroélué, soumis à la précipitation alcoolique, séché et ligué à pSCV1.2 digéré par SmaI et déphosphorylé. La cassette d'expression "pGEA6D-PSLGL-LGC-t35S" est issue de **pBIOC92.**

### III. CONSTRUCTION DE GENES CHIMERIQUES CODANT POUR LA PROTEINE RECOMBINANTE DE LIPASE GASTRIQUE HUMAINE ET PERMETTANT UNE EXPRESSION CONSTITUTIVE, PAR EXEMPLE, DANS LES FEUILLES ET GRAINES DE TABAC.

### a) Construction du plasmide binaire pBIOC82 contenant le promoteur chimérique SUPER-PROMOTEUR pSP.

L'expression dans les feuilles de tabac du gène codant pour la lipase gastrique humaine (LGH) a nécessité les séquences régulatrices suivantes :
1. le promoteur chimérique super-promoteur (pSP ; PCT/US94/12946). Il est constitué de la triple répétition d'un élément activateur transcriptionnel du promoteur du gène de l'octopine synthase d'*Agrobacterium tumefaciens,* d'un élément activateur transcriptionnel du promoteur du gène de mannopine synthase et du promoteur mannopine synthase d'*Agrobacterium tumefaciens ;*
2. la séquence terminatrice de transcription, terminateur polyA 35S, qui correspond à la région en 3' non codante de la séquence du virus à ADN bicaténaire circulaire de la mosaïque du chou-fleur produisant le transcrit 35S (Franck *et al.,* 1980).

Pour obtenir un plasmide binaire similaire à **pBIOC21** mais dont le promoteur pd35S a été remplacé par le promoteur pSP, le fragment pvuII-SalI soumis à l'action de la Klenow, contenant le promoteur pSP, a été isolé à partir du plasmide pBISN1 **(*****PCT*****/*****US94*****/*****12946*****),** purifié par électrophorèse sur gel d'agarose 1%, électroélué (Sambrook *et al.,* 1989), soumis à la précipitation alcoolique, séché et ligué à l'ADN plasmidique de pBIOC81 doublement digéré par *KpnI et EcoRI* soumis à l'action de la T4 DNA polymérase et déphosphorylé par l'enzyme phosphatase alcaline d'intestin de veau (Boehringer Mannheim) selon les recommandations du fabricant. Le plasmide pBIOC81 correspond à pBIOC21 dont le site *XbaI* a été délété. Pour ce faire, le plasmide pBIOC21 a été digéré par *XbaI,* puis soumis à l'action de l'enzyme Klenow et ligué par action avec le T4 DNA ligase.

La ligation a été réalisée avec 20 ng du vecteur déphosphorylé décrit ci-dessus et 200 ng de fragments d'ADN portant pSP décrits ci-dessus dans un milieu réactionnel de 20 *µ*l en présence de 2 *µ*l de tampon T4 DNA ligase x 10 (Amersham),de 2 *µ*l de 50% polyéthylène glycol 8000 et de 5 U d'enzyme T4 DNA ligase (Amersham) à 14°C pendant 16 heures. Les bactéries, *Escherichia coli* DH5α rendues préalablement compétentes, ont été transformées (Hanahan, 1983). L'ADN plasmidique des clones obtenus, sélectionnés sur 12 *µ*g/ml tétracycline, a été extrait selon la méthode de la lyse alcaline (Birnboim et Doly, 1979) et analysé par digestion enzymatique par des enzymes de restriction. Le plasmide résultant a été appelé pBIOC82.

La lipase gastrique humaine (LGH) est naturellement synthétisée sous forme d'un précurseur décrit dans la publication de Bodmer *et al.,* 1987. La protéine mature LGH est constituée de 379 acides aminés. Son peptide signal (PSLGH) est composé de 19 acides aminés. Le site de clivage entre PSLGH et LGH est Gly-Leu.

La séquence codant pour le précurseur de LGH a été utilisée pour la construction des plasmides binaires **pBIOC85** contenant PSLGH-LGH, **pBIOC87** contenant PSLPH-LGH et **pBIOC89** contenant PSLGL-LGH où la séquence codant pour LGH est précédée de celles codant pour son peptide signal naturel PSLGH, le peptide signal de la lipase pancréatique humaine (PSLPH ; Giller *et al*., 1992) et le peptide signal de la lipase gastrique de lapin (PSLGL ; déjà décrit précédemment ; brevet européen n° 92.403055.4) respectivement.

La séquence codant pour le précurseur de LGH a été isolée par double digestion PstI et DraI, purifiée par électrophorèse sur gel d'agarose 0,8%, électroéluée (Sambrook *et al*., 1989), précipitée en présence de 1/10 de volume de 3M acétate de sodium pH4,8 et de 2,5 volumes d'éthanol absolu à-80°C pendant 30 min., centrifugé à 12000g pendant 30 min., lavée à l'éthanol 70% et séchée. Puis, elle a été clonée aux sites PstI et SpeI (soumis à l'action de l'enzyme T4 DNA polymérase (Biolabs) selon les recommandations du fabricant) du plasmide pBSIISK+ commercialisé par Stratagene. La ligation a été réalisée avec 100 ng du vecteur et 50 ng de fragments d'ADN portant la séquence codant pour le précurseur de LGH, décrits ci-dessus, dans un milieu réactionnel de 10 *µ*l en présence de 1 *µ*l de tampon T4 DNA ligase x 10 (Amersham) et de 2,5 U d'enzyme T4 DNA ligase (Amersham) à 14°C pendant 16 heures. Les bactéries, *Escherichia coli* DH5α rendues préalablement compétentes, ont été transformées (Hanahan, 1983). L'ADN plasmidique des clones obtenus, sélectionnés sur 50 *µ*g/ml ampicilline, a été extrait selon la méthode de la lyse alcaline (Birnboim et Doly, 1979) et analysé par digestion enzymatique par des enzymes de restriction. Le plasmide résultant a été appelé **pBIOC83.**

La séquence codant pour LGH mature a été modifiée par l'introduction d'un site BamHI, inexistant préalablement, dans les neuvième et dixième codons, par mutagenèse dirigée par PCR en utilisant 2 oligodésoxynucléotides. 5' aaactgcaggctcgag TTG TTT GGA AAA TTA CAT CCT GGA tcc CCT GAA GTG ACT ATG 3' (contenant les sites PstI, XhoI et BamHI uniques) et 5' AAT GGT GGT GCC CTG GGA ATG GCC AAC ATA GTG TAG CTG C 3' (contenant le site MscI unique dans le plasmide **pBIOC83**)**.**

L'amplification PCR du fragment PstI-MscI a été réalisée dans 100 *µ*l de milieu réactionnel comprenant 10 *µ*l de tampon Taq DNA polymérase x10 (500 mM KCl, 100 mM Tris-HCl, pH9,0 et 1% Triton x100), 6 µl de 25 mM MgCl₂, 3 µl de 10 mM dNTP (dATP, dCTP, dGTP et dTTP), 100 pM de chacun des 2 oligodésoxynucléotides décrits ci-dessus, 5 ng d'ADN matrice (vecteur pBIOC83), 2,5 U de Taq DNA polymérase (Promega) et 2 gouttes d'huile de vaseline. L'ADN a été dénaturé à 94°C pendant 5 min., soumis à 30 cycles constitués chacun de 1 min. de dénaturation à 94°C, 1 min. d'hybridation à 65°C et de 1 min. d'élongation à 72°C, puis l'élongation à 72°C a été poursuivie pendant 5 min. Cette réaction PCR a été réalisée dans la machine "DNA Thermal Cycler" de PERKIN ELMER CETUS. L'huile a été éliminée par extraction au chloroforme. Puis, les fragments d'ADN du milieu réactionnel ont été précipités en présence de 1/10 de volume de 3M acétate de sodium pH4,8 et de 2,5 volumes d'éthanol absolu à -80°C pendant 30 min., centrifugés à 12000g pendant 30 min., lavés à l'éthanol 70%, séchés et digérés par les 2 enzymes de restriction PstI et MscI. Les fragments d'ADN digérés issus de l'amplification par PCR ont été purifiés par électrophorèse sur gel d'agarose 2%, électroélués (Sambrook *et al.,* 1989), précipités en présence de 1/10 de volume de 3M acétate de sodium pH4,8 et de 2,5 volumes d'éthanol absolu à -80°C pendant 30 min., centrifugés à 12000 g pendant 30 min., lavés à l'éthanol 70%, séchés, puis ligués à l'ADN plasmidique de pBIOC83 doublement digéré par PstI et MscI, purifié par électrophorèse sur gel d'agarose 0,8%, électroélué, soumis à la précipitation alcoolique, séché. La ligation a été réalisée avec 100 ng du vecteur et 50 ng de fragments d'ADN digérés issus de l'amplification PCR, décrits ci-dessus, dans un milieu réactionnel de 10 µl en présence de 1 µl de tampon T4 DNA ligase x 10 (Amersham) et de 2,5 U d'enzyme T4 DNA ligase (Amersham) à 14°C pendant 16 heures. Les bactéries, *Escherichia coli* DH5α rendues préalablement compétentes, ont été transformées (Hanahan, 1983). L'ADN plasmidique des clones obtenus, sélectionnés sur 50 µg/ml ampicilline, a été extrait selon la méthode de la lyse alcaline (Birnboim et Doly, 1979) et analysé par digestion enzymatique par des enzymes de restriction. Certains des clones retenus ont été vérifiés par séquençage à l'aide du kit de séquençage T7™ commercialisé par Pharmacia selon la méthode des didésoxynucléotides (Sanger et al., 1977). L'introduction du site de restriction BamHI ne modifie pas le code génétique de LGH. En effet, la séquence LGH naturelle GGA AGC (Gly-Ser) devient GGA TCC (Gly-Ser). Le plasmide résultant a été appelé **pBIOC84**

### b) Construction du plasmide binaire pBIOC85 contenant PSLGH-LGH.

Le fragment PstI - XbaI portant la séquence de PSLGH-LGH a été isolé par double digestion enzymatique par PstI et XbaI à partir de **pBIOC83,** purifié par électrophorèse sur gel d'agarose 0,8%, électroélué (Sambrook *et al*., 1989), soumis à la précipitation alcoolique et séché. Puis, ce fragment d'ADN a été traité par l'enzyme T4 DNA polymérase (Biolabs) selon les recommandations du fabricant et ligué à l'ADN plasmidique de **pBIOC82** digéré au site *XbaI*, traité à la Klenow (Biolabs) et déphosphorylé par l'enzyme phosphatase alcaline d'intestin de veau (Boehringer Mannheim) selon les recommandations du fabricant. La ligation a été réalisée avec 20 ng du vecteur déphosphorylé et 200 ng de fragments d'ADN contenant PSLGH-LGH, décrits ci-dessus, dans un milieu réactionnel de 20 µl en présence de 2 µl de tampon T4 DNA ligase x 10 (Amersham), de 2 µl de 50% polyethylène glycol 8000 et de 5 U d'enzyme T4 DNA ligase (Amersham) à 14°C pendant 16 heures. Les bactéries, *Escherichia coli* DH5α rendues préalablement compétentes, ont été transformées (Hanahan, 1983). L'ADN plasmidique des clones obtenus, sélectionnés sur 12 µg/ml tétracycline, a été extrait selon la méthode de la lyse alcaline (Birnboim et Doly, 1979) et analysé par digestion enzymatique par des enzymes de restriction. Le clone résultant a été appelé **pBIOC85.** La séquence nucléique du fragment codant pour la protéine recombinante PSLGH-LGH a été vérifiée par séquençage à l'aide du kit de séquençage T7™ commercialisé par Pharmacia selon la méthode des didésoxynucléotides (Sanger *et al.,* 1977). La séquence de clivage entre les séquences PSLGH et LGH mature est Gly-Leu. L'ADN plasmidique du vecteur binaire **pBIOC85** a été introduit par transformation directe dans la souche LBA4404 d'*Agrobacterium tumefaciens* selon le procédé de Holsters et al. (1978). La validité du clone retenu a été vérifiée par digestion enzymatique de l'ADN plasmidique introduit.

### c) Construction du plasmide binaire pBIOC86 contenant PSLPH-LGH.

Le plasmide **pBIOC84** a été digéré doublement par PstI et BamHI afin de supprimer la séquence codant pour le peptide signal PSLGH et les 8 premiers acides aminés de la protéine LGH mature (Leu-Phe-Gly-Lys-Leu-His-Pro-Gly). Cette séquence a été remplacée par celle codant pour le peptide signal PSLPH de 16 acides aminés (ATG CTG CCA CTT TGG ACT CTT TCA CTG CTG CTG GGA GCA GTA GCA GGA) fusionnée à celle codant pour les 8 premiers codons de la protéine LGH mature ("PSLPH - 8 premiers codons de LGH mature"). La séquence "PSLPH - 8 premiers codons de LGH mature" a été amplifiée par PCR à partir de la matrice 5' aaactgcaggctcgagaacaATG CTG CCA CTT TGG ACT CTT TCA CTG CTG CTG GGA GCA GTA GCA GGA TTG TTT GGA AAA TTA CAT CCT GGA tcc CCT G 3' à l'aide des 2 oligodésoxynucléotides, 5' aaactgcaggctcgagaacaATG C 3' et 5' C AGG gga TCC AGG ATG TAA TTT TCC 3', en suivant le protocole d'amplification PCR décrit précédemment (voir paragraphe I ci-dessus). La température d'hybridation a été adaptée. Après double digestion enzymatique par PstI et BamHI, les fragments d'ADN issus de l'amplification PCR ont été purifiés par électrophorèse sur gel d'agarose 2%, électroélués (Sambrook *et al.,* 1989), précipités en présence de 1/10 de volume de 3M acétate de sodium pH4,8 et de 2,5 volumes d'éthanol absolu à -80°C pendant 30 min., centrifugés à 12000 g pendant 30 min., lavés à l'éthanol 70%, séchés, puis ligués à l'ADN plasmidique de **pBIOC84** doublement digéré par PstI et BamHI, purifié par électrophorèse sur gel d'agarose 0,8%, électroélué (Sambrook *et al.,* 1989), soumis à la précipitation alcoolique, séché. La ligation a été réalisée avec 100 ng du vecteur et 50 ng de fragments d'ADN digérés issus de l'amplification PCR, décrits ci-dessus, dans un milieu réactionnel de 10 *µ*l en présence de 1 *µ*l de tampon T4 DNA ligase x 10 (Amersham) et de 2,5 U d'enzyme T4 DNA ligase (Amersham) à 14°C pendant 16 heures. Les bactéries, *Escherichia coli* DH5α rendues préalablement compétentes, ont été transformées (Hanahan, 1983). L'ADN plasmidique des clones obtenus, sélectionnés sur 50 *µ*g/ml ampicilline, a été extrait selon la méthode de la lyse alcaline (Birnboim et Doly, 1979) et analysé par digestion enzymatique par des enzymes de restriction. Certains des clones retenus ont été vérifiés par séquençage à l'aide du kit de séquençage T7™ commercialisé par Pharmacia selon la méthode des didésoxynucléotides (Sanger *et al*., 1977). Les séquences du PSLPH et de LGH mature ont été clonées en maintenant leurs phases de lecture ouverte (c'est-à-dire, de telle sorte qu'elles constituent une phase ouverte de lecture unique). La séquence de clivage entre les séquences PSLPH et LGH mature est Gly-Leu. Le plasmide résultant a été appelé **pBIOC86.**

A partir de **pBIOC86,** le fragment PstI - XbaI portant la séquence PSLPH-LGH a été isolé par double digestion enzymatique par PstI et XbaI, purifié par électrophorèse sur gel d'agarose 0,8%, électroélué (Sambrook *et al*., 1989), soumis à la précipitation alcoolique et séché. Puis, ce fragment d'ADN a été traité par l'enzyme T4 DNA polymérase (Biolabs) selon les recommandations du fabricant et ligué à l'ADN plasmidique de **pBIOC82** digéré au site ***XbaI**,* traité à la Klenow (Biolabs) et déphosphorylé par l'enzyme phosphatase alcaline d'intestin de veau (Boehringer Mannheim) selon les recommandations du fabricant. La ligation a été réalisée avec 20 ng du vecteur déphosphorylé décrit ci-dessus et 200 ng de fragments d'ADN contenant PSLPH-LGH décrits ci-dessus dans un milieu réactionnel de 20 *µ*l en présence de 2 *µ*l de tampon T4 DNA ligase x 10 (Amersham), de 2 *µ*l de 50% polyethylène glycol 8000 et de 5 U d'enzyme T4 DNA ligase (Amersham) à 14°C pendant 16 heures. Les bactéries, *Escherichia coli* DH5α rendues préalablement compétentes, ont été transformées (Hanahan, 1983). L'ADN plasmidique des clones obtenus, sélectionnés sur 12 *µ*g/ml tétracycline, a été extrait selon la méthode de la lyse alcaline (Birnboim et Doly, 1979) et analysé par digestion enzymatique par des enzymes de restriction. Le clone résultant a été appelé **pBIOC87.** La séquence nucléique du fragment codant pour la protéine recombinante PSLPH-LGH a été vérifiée par séquençage à l'aide du kit de séquençage T7™ commercialisé par Pharmacia selon la méthode des didésoxynucléotides (Sanger *et al.,* 1977). L'ADN plasmidique du vecteur binaire **pBIOC87** a été introduit par transformation directe dans la souche LBA4404 d'*Agrobacterium tumefaciens* selon le procédé de Holsters et al. (1978). La validité du clone retenu a été vérifiée par digestion enzymatique de l'ADN plasmidique introduit.

### d) Construction du plasmide binaire pBIOC89 contenant PSLGL-LGH.

Le plasmide **pBIOC84** a été digéré doublement par PstI et BamHI afin de supprimer la séquence codant pour le peptide signal PSLGH et les 8 premiers acides aminés de la protéine LGH mature (Leu-Phe-Gly-Lys-Leu-His-Pro-Gly). Cette séquence a été remplacée par celle codant pour le peptide signal PSLGL de 19 acides aminés (ATG TGG GTG CTT TTC ATG GTG GCA GCT TTG CTA TCT GCA CTT GGA ACT ACA CAT GGT) fusionnée à celle codant pour les 8 premiers codons de la protéine LGH mature ("PSLGL - 8 premiers codons de LGH mature"). La séquence "PSLGL - 8 premiers codons de LGH mature" a été amplifiée par PCR à partir de la matrice 5' aaactgcaggctcgagaacaATG TGG GTG CTT TTC ATG GTG GCA GCT TTG CTA TCT GCA CTT GGA ACT ACA CAT GGTTTG TTT GGA AAA TTA CAT CCT GGA tcc CCT G 3' à l'aide des 2 oligodésoxynucléotides, 5' aaactgcaggctcgagaacaATG TGG 3' et 5' C AGG gga TCC AGG ATG TAA TTT TCC 3', en suivant le protocole d'amplification PCR décrit précédemment (voir paragraphe I ci-dessus). La température d'hybridation a été adaptée. Après double digestion enzymatique par PstI et BamHI, les fragments d'ADN issus de l'amplification PCR ont été purifiés par électrophorèse sur gel d'agarose 2%, électroélués (Sambrook *et al.,* 1989), précipités en présence de 1/10 de volume de 3M acétate de sodium pH4,8 et de 2,5 volumes d'éthanol absolu à -80°C pendant 30 min., centrifugés à 12000 g pendant 30 min., lavés à l'éthanol 70%, séchés, puis ligués à l'ADN plasmidique de **pBIOC84** doublement digéré par PstI et BamHI, purifié par électrophorèse sur gel d'agarose 0,8%, électroélué (Sambrook *et al.,* 1989), soumis à la précipitation alcoolique, séché. La ligation a été réalisée avec 100 ng du vecteur et 50 ng de fragments d'ADN digérés issus de l'amplification PCR, décrits ci-dessus, dans un milieu réactionnel de 10 *µ*l en présence de 1 *µ*l de tampon T4 DNA ligase x 10 (Amersham) et de 2,5 U d'enzyme T4 DNA ligase (Amersham) à 14°C pendant 16 heures. Les bactéries, *Escherichia coli* DH5α rendues préalablement compétentes, ont été transformées (Hanahan, 1983). L'ADN plasmidique des clones obtenus, sélectionnés sur 50 µg/ml ampicilline, a été extrait selon la méthode de la lyse alcaline (Birnboim et Doly, 1979) et analysé par digestion enzymatique par des enzymes de restriction. Certains des clones retenus ont été vérifiés par séquençage à l'aide du kit de séquençage T7™ commercialisé par Pharmacia selon la méthode des didésoxynucléotides (Sanger *et al.,* 1977). Les séquences du PSLGL et de LGH mature ont été clonées en maintenant leurs phases de lecture ouverte (c'est-à-dire, de telle sorte qu'elles constituent une phase ouverte de lecture unique). La séquence de clivage entre les séquences PSLGL et LGH mature est Gly-Leu. Le plasmide résultant a été appelé **pBIOC88.**

A partir de **pBIOC88,** le fragment PstI - XbaI portant la séquence PSLGL-LGH a été isolé par double digestion enzymatique par PstI et XbaI, purifié par électrophorèse sur gel d'agarose 0,8%, électroélué (Sambrook *et al*., 1989), soumis à la précipitation alcoolique et séché. Puis, ce fragment d'ADN a été traité par l'enzyme T4 DNA polymérase (Biolabs) selon les recommandations du fabricant et ligué à l'ADN plasmidique de **pBIOC82** digéré au site ***XbaI**,* traité à la Klenow (Biolabs) et déphosphorylé par l'enzyme phosphatase alcaline d'intestin de veau (Boehringer Mannheim) selon les recommandations du fabricant. La ligation a été réalisée avec 20 ng du vecteur déphosphorylé et 200 ng de fragments d'ADN contenant PSLGL-LGH, décrits ci-dessus, dans un milieu réactionnel de 20 *µ*l en présence de 2 *µ*l de tampon T4 DNA ligase x 10 (Amersham), de 2 *µ*l de 50% polyethylène glycol 8000 et de 5 U d'enzyme T4 DNA ligase (Amersham) à 14°C pendant 16 heures. Les bactéries, *Escherichia coli* DH5α rendues préalablement compétentes, ont été transformées (Hanahan, 1983). L'ADN plasmidique des clones obtenus, sélectionnés sur 12 *µ*g/ml tétracycline, a été extrait selon la méthode de la lyse alcaline (Birnboim et Doly, 1979) et analysé par digestion enzymatique par des enzymes de restriction. Le clone résultant a été appelé **pBIOC89.** La séquence nucléique du fragment codant pour la protéine recombinante PSLGL-LGH a été vérifiée par séquençage à l'aide du kit de séquençage T7™ commercialisé par Pharmacia selon la méthode des didésoxynucléotides (Sanger *et al*., 1977). L'ADN plasmidique du vecteur binaire pBIOC89 a été introduit par transformation directe dans la souche LBA4404 d'*Agrobacterium tumefaciens* selon le procédé de Holsters *et al*. (1978). La validité du clone retenu a été vérifiée par digestion enzymatique de l'ADN plasmidique introduit.

### IV. CONSTRUCTION DE GENES CHIMERIQUES CODANT POUR LA PROTEINE RECOMBINANTE DE LIPASE GASTRIQUE DE CHIEN ET PERMETTANT UNE EXPRESSION DANS LES SEMENCES DE MAIS.

### a) Construction des plasmides pBIOC98 et pBIOC99 contenant PSLGL-LGC et permettant une expression constitutive dans les semences de maïs.

L'expression constitutive dans les semences de maïs du gène animal codant pour la lipase gastrique de chien (LGC) a nécessité les séquences régulatrices suivantes :
1. un des deux promoteurs permettant une expression constitutive :
   - promoteur actine de riz suivi de l'intron actine de riz (pAR-IAR) contenu dans le plasmide pAct1-F4 décrit par McElroy *et al.* (1991) ;
   - promoteur constitutif double 35S (pd35S) du CaMV (virus de la mosaïque du chou-fleur). II correspond à une duplication des séquences activant la transcription situées en amont de l'élément TATA du promoteur 35S naturel (Kay *et al.,* 1987);
2. un des deux terminateurs :
   - la séquence terminatrice de transcription, terminateur polyA 35S, qui correspond à la région en 3' non codante de la séquence du virus à ADN bicaténaire circulaire de la mosaïque du chou-fleur produisant le transcrit 35S (Franck *et al.,* 1980) ;
   - la séquence terminatrice de transcription, terminateur polyA NOS, qui correspond à la région en 3' non codante du gène de la nopaline synthase du plasmide Ti d'*Agrobacterium tumefaciens* souche à nopaline (Depicker *et al.,* 1982).

Le plasmide **pBIOC98** où la séquence codant pour PSLGL-LGC est placée sous contrôle de pAR-IAR a été obtenu par clonage du fragment BgIII-XbaI portant la séquence codant pour PSLGL-LGC aux sites "NcoI et SaII" de pBSII-pAR-IAR-tNOS.

Le fragment BgHI-XbaI portant la séquence codant pour PSLGL-LGC a été isolée à partir de **pBIOC40** (décrit ci-dessus) par double digestion enzymatique par BgIII et XbaI, purifié par électrophorèse sur gel d'agarose 0,8%, électroélué, soumis à la précipitation alcoolique, séché, puis traité par l'enzyme Klenow. Le plasmide pBSII-pAR-IAR-tNOS a été digéré doublement par SaII et NcoI, purifié, traité à l'enzyme Mung Bean Nucléase (Biolabs) et déphosphorylé par l'enzyme phosphatase alcaline de veau (Boehringer Mannheim) selon les recommandations des fabricants. La ligation a été réalisée avec 20 ng du vecteur déphosphorylé et 200 ng de fragments d'ADN contenant la séquence codant pour PSLGL-LGC, décrits ci-dessus, dans un milieu réactionnel de 20 *µ*l en présence de 2 *µ*l de tampon T4 DNA ligase x 10 (Amersham), de 2 *µ*l de 50% polyethylène glycol 8000 et de 5 U d'enzyme T4 DNA ligase (Amersham) à 14°C pendant 16 heures. Les bactéries, *Escherichia coli* DH5α rendues préalablement compétentes, ont été transformées (Hanahan, 1983). L'ADN plasmidique des clones obtenus, sélectionnés sur 50 *µ*g/ml ampicilline, a été extrait selon la méthode de la lyse alcaline (Birnboim et Doly, 1979) et analysé par digestion enzymatique par des enzymes de restriction. Le plasmide résultant a été appelé **pBIOC98.**

Le plasmide pBSII-pAR-IAR-tNOS résulte du clonage aux sites "Eco0109I traité à la Klenow et KpnI" de pBSII-tNOS du fragment SnaBI-KpnI portant la séquence correspondant à "pAR-IAR-début de la séquence codante pour le gène gus" isolé à partir du plasmide pAct1-F4. La ligation a été réalisée avec 100 ng de vecteur et 50 ng de fragments d' ADN, décrits ci-dessus, dans un milieu réactionnel de 10 *µ*l digérés en présence de 1 *µ*l de tampon T4 DNA ligase x 10 (Amersham) et de 2,5 U d'enzyme T4 DNA ligase (Amersham) à 14°C pendant 16 heures. Les bactéries, *Escherichia coli* DH5α rendues préalablement compétentes, ont été transformées (Hanahan, 1983). L'ADN plasmidique des clones obtenus, sélectionnés sur 50 *µ*g/ml ampicilline, a été extrait selon la méthode de la lyse alcaline (Birnboim et Doly, 1979) et analysé par digestion enzymatique par des enzymes de restriction.

Le plasmide pBSII-tNOS a été obtenu par clonage au site EcoRV déphosphorylé de pBSIISK+ commercialisé par Stratagene, du fragment SacI-EcoRI portant la séquence tNOS isolé à partir de pBI121 commercialisé par Clontech par double digestion enzymatique par SacI et EcoRV, soumis à une purification par électrophorèse sur gel d'agarose à 2%, et traité à l'enzyme T4 DNA polymérase. La ligation a été réalisée avec 20 ng du vecteur déphosphorylé et 200 ng de fragments d'ADN contenant la séquence tNOS, décrits ci-dessus, dans un milieu réactionnel de 20 *µ*l en présence de 2 *µ*l de tampon T4 DNA ligase x 10 (Amersham), de 2 *µ*l de 50% polyethylène glycol 8000 et de 5 U d'enzyme T4 DNA ligase (Amersham) à 14°C pendant 16 heures. Les bactéries, *Escherichia coli* DH5α rendues préalablement compétentes, ont été transformées (Hanahan, 1983). L'ADN plasmidique des clones obtenus, sélectionnés sur 50 *µ*g/ml ampicilline, a été extrait selon la méthode de la lyse alcaline (Birnboim et Doly, 1979) et analysé par digestion enzymatique par des enzymes de restriction.

Le plasmide **pBIOC99** où la séquence codant pour PSLGL-LGC est placée sous contrôle de pd35S a été obtenu par clonage aux sites "KpnI et BamHI" du plasmide pBSII-t35S, du fragment KpnI-BamHI portant la séquence correspondant à "pd35S-PSLGL-LGC" isolé à partir de **pBIOC41** décrit ci-dessus. La ligation a été réalisée avec 100 ng de vecteur et 50 ng de fragments d'ADN, décrits ci-dessus, dans un milieu réactionnel de 10 *µ*l digérés en présence de 1 ml de tampon T4 DNA ligase x 10 (Amersham) et de 2,5 U d'enzyme T4 DNA ligase (Amersham) à 14°C pendant 16 heures. Les bactéries, *Escherichia coli* DH5α rendues préalablement compétentes, ont été transformées (Hanahan, 1983). L'ADN plasmidique des clones obtenus, sélectionnés sur 50 µg/ml ampicilline, a été extrait selon la méthode de la lyse alcaline (Birnboim et Doly, 1979) et analysé par digestion enzymatique par des enzymes de restriction.

Le plasmide pBSII-t35S a été obtenu par clonage au site SpeI traité à la Klenow, déphosphorylé du plasmide pBSIISK+ commercialisé par Stratagène, du fragment SmaI-EcoRV portant la séquence t35S isolé à partir de pJIT163 (décrit ci-dessus) par double digestion enzymatique par SmaI et EcoRV, soumis à une purification par électrophorèse sur gel d'agarose à 2%. La ligation a été réalisée avec 20 ng du vecteur déphosphorylé et 200 ng de fragments d'ADN contenant la séquence t35S, décrits ci-dessus, dans un milieu réactionnel de 20 µl en présence de 2 µl de tampon T4 DNA ligase x 10 (Amersham), de 2 µl de 50% polyethylène glycol 8000 et de 5 U d'enzyme T4 DNA ligase (Amersham) à 14°C pendant 16 heures. Les bactéries, *Escherichia coli* DH5α rendues préalablement compétentes, ont été transformées (Hanahan, 1983). L'ADN plasmidique des clones obtenus, sélectionnés sur 50 µg/ml ampicilline, a été extrait selon la méthode de la lyse alcaline (Birnboim et Doly, 1979) et analysé par digestion enzymatique par des enzymes de restriction.

### b) Construction des plasmides pBIOC100 et pBIOC101 contenant PSLGL-LGC et PSLGL-LGC-KDEL respectivement et permettant une expression dans l'albumen des semences de maïs.

L'expression dans l'albumen des semences de maïs du gène animal codant pour la lipase gastrique de chien (LGC) a nécessité les séquences régulatrices suivantes :
1. le promoteur du gène de γzéine de maïs (pγzéine) contenu dans le plasmide pγ63 décrit dans Reina et al., 1990. Le plasmide pγ63 résulte du clonage de pγzéine aux sites HindIII et XbaI d'un plasmide **pUC18** renfermant, entre ses sites HindIII et EcoRI, la cassette d'expression "p35S-gus-tNOS" de pBI221 commercialisé par Clontech. Il permet une expression dans l'albumen des semences de maïs.
2. la séquence terminatrice de transcription, terminateur polyA NOS, qui correspond à la région en 3' non codante du gène de la nopaline synthase du plasmide Ti d'Agrobacterium tumefaciens souche à nopaline (Depicker *et al*., 1982).

Le plasmide **pBIOC100** où la séquence codant pour PSLGL-LGC est placée sous contrôle du pγzéine, a été obtenu par clonage aux sites "SacI traité par l'enzyme T4 DNA polymérase et BamHI" du plasmide pγ63, du fragment "XbaI traité à la Klenow - BglII" isolé à partir de **pBIOC40** (décrit ci-dessus). La ligation a été réalisée avec 100 ng du vecteur et 50 ng de fragments d'ADN, décrits ci-dessus, dans un milieu réactionnel de 10 µl en présence de 1 µl de tampon T4 DNA ligase x 10 (Amersham) et de 2,5 U d'enzyme T4 DNA ligase (Amersham) à 14°C pendant 16 heures. Les bactéries, *Escherichia coli* DH5α rendues préalablement compétentes, ont été transformées (Hanahan, 1983). L'ADN plasmidique des clones obtenus, sélectionnés sur 50 µg/ml ampicilline, a été extrait selon la méthode de la lyse alcaline (Birnboim et Doly, 1979) et analysé par digestion enzymatique par des enzymes de restriction. Le clone résultant a été appelé **pBIOC100.**

Le plasmide **pBIOC101** résulte de la substitution du fragment NcoI-AflII de pBIOC100 par le fragment NcoI-AflII portant la séquence codant pour le tétrapeptide KDEL (permettant un adressage dans le réticulum endoplasmique) placée avant le codon stop obtenu par amplification PCR selon les procédés décrits ci-dessus. Les 2 oligodésoxynucléotides utilisés lors de cette réaction ont été : 5' AAT CAC TTG GAC TTT ATC TGG Gcc atg gAT GCC 3' (site NcoI unique) et 5' AAT ctt aag AAA CTT TAT TGC CAA ATG TTT GAA CGA TCG GGG AAA TTC GAC GCG TCT AGA ACT ATA GCT CAT CCT TAT TAT CTG TTC CCA TCA TGG 3' (séquence codant pour KDEL et site AflII unique). La température d'hybridation a été de 70°C. Le clonage a été effectué comme décrit précédemment.

### V. EXEMPLE D'OBTENTION DE PLANTS DE COLZA TRANSGENIQUES.

Les graines de colza de printemps (*Brassica napus* cv WESTAR ou lignées Limagrain) sont désinfectées pendant 40 minutes dans une solution de Domestos à 15%. Après 4 rinçages à l'eau stérile, les graines sont mises à germer, à raison de 20 graines par pot de 7 cm de diamètre et 10 cm de haut, sur du milieu minéral de Murashige et Skoog (Sigma M 5519) avec 30g/l de saccharose et solidifié avec de l'agargel à 5g/l. Ces pots sont placés dans une chambre de culture à 26°C avec une photopériode de 16h/8h et sous une intensité lumineuse de l'ordre de 80 µE m⁻² S⁻¹.

Après 5 jours de germination, les cotylédons sont prélevés stérilement en coupant chaque pétiole environ 1 mm au-dessus du noeud cotylédonaire.

Parallèlement une préculture d'*Agrobacterium tumefaciens* souche LBA4404, contenant le plasmide pBIOC29 (ou pBIOC25), à savoir, le plasmide pGAZE dans lequel a été inséré la séquence codant pour la lipase gastrique de chien fusionnée à celle codant pour un signal d'adressage PPS (ou PS) sous contrôle du promoteur pCRU (ou pd35S), est réalisée en erlen meyer de 50 ml, pendant 36 h à 28°C dans 10 ml de milieu bactérien 2YT (Sambrook *et al.,* 1989) complémenté avec les antibiotiques utiles à la sélection de la souche utilisée.

Cette préculture sert à ensemencer à 1% une nouvelle culture bactérienne effectuée dans les mêmes conditions. Au bout de 14 h la culture est centrifugée 15 min à 3000 g et les bactéries sont reprises dans un volume équivalent de milieu de germination liquide. Cette suspension est distribuée dans des boîtes de pétri de 5 cm de diamètre à raison de 5 ml/boîte.

L'extrémité sectionnée du pétiole est immergée quelques secondes dans la solution d'agrobactéries ainsi préparées, puis le pétiole est enfoncé de quelques millimètres dans le milieu de régénération. Ce milieu a la même composition de base que le milieu de germination avec en plus 4 mg/l de benzyl-amino-purine (BAP), phytohormone favorisant la néoformation de bourgeons. Dix explants (cotylédon avec pétiole) sont mis en culture par boîte de pétri de 9 cm de diamètre (Greiner référence 664102).

Après 2 jours de coculture dans les mêmes conditions environnementales que les germinations, les explants sont repiqués dans des boîtes phytatray (Sigma, référence P1552) contenant le milieu précédent complémenté avec un agent sélectif: 45 mg/l de sulfate de kanamycine (Sigma, référence K4000) et un bactériostatique: mélange de 1/6 (en poids) de sel de potassium d'acide clavulanique et de 5/6 sel de sodium d'amoxicilline (Augmentin injectable) à raison de 600 mg/l.

Deux fois de suite, à 3 semaines d'intervalle, les explants sont repiqués stérilement sur du milieu neuf dans les mêmes conditions.

Les bourgeons verts apparus à la fin du deuxième ou du troisième repiquage sont séparés de l'explant et mis en culture individuellement dans des pots transparents de 5 cm de diamètre et de 10 cm de haut contenant un milieu identique au précédent mais dépourvu de BAP. Après 3 semaines de culture la tige du bourgeon transformé est sectionnée et le bourgeon est repiqué dans un pot de milieu frais. Au bout de trois à quatre semaines les racines sont assez développées pour permettre l'acclimatation de la plantule dans un phytotron. Les bourgeons qui ne sont pas verts ou enracinés sont éliminés. Ces plantules sont alors transplantées dans des godets de 7 cm de côté remplis de terreau (norme NF U44551: 40% tourbe brune, 30% bruyère tamisée et 30% sable) saturé en eau. Après deux semaines d'acclimatation en phytotron (température 21 °C, photopériode 16h/8h et 84% d'humidité relative), les plantules sont rempotées dans des pots de 12 cm de diamètre remplis du même terreau enrichi en engrais retard (Osmocote, à raison de 4 g/l de terreau) puis transportées en serre (classe S2) régulée à 18°C, avec deux arrosages quotidien de 2 minutes à l'eau.

Dès l'apparition de fleurs celles-ci sont ensachées (Crispac, référence SM 570y 300 mm*700 mm) de façon à empêcher la fécondation croisée.

Lorsque les siliques sont arrivées à maturité, elles sont récoltées, séchées, puis battues. Les graines obtenues servent au dosage de l'activité biochimique. La sélection de la descendance transgénique se fait par germination sur un milieu contenant du sulfate de kanamycine à raison de 100 à 150 mg/l (selon les génotypes). Les conditions opératoires sont identiques à celles décrites ci-dessus à ceci près que les germinations sont effectuées en tubes de verre avec une seule graine par tube. Seules les plantules développant des racines secondaires les trois premières semaines sont acclimatées en phytotron avant d'être passées en serre.

### VI. EXEMPLE D'OBTENTION DE PLANTS DE SOLANACEES TRANSGENIQUES.

### a) Obtention de plantes de tabac transgéniques

Les plants de tabac utilisées pour les expériences de transformation (*Nicotiana tabacum* var. *Xanthi* NC et PBD6) sont cultivées *in vitro* sur le milieu de base de Murashige et Skoog (1962) additionné des vitamines de Gamborg *et al.* (1968, Sigma référence M0404) de saccharose à 20 g/l et d'agar (Merck) à 8 g/l. Le pH du milieu est ajusté à 5,8 avec une solution de potasse avant autoclavage à 120°C pendant 20 min. Les plantules de tabac sont repiquées par bouture des entre-noeuds tous les 30 jours sur ce milieu de multiplication MS20.

Toutes les cultures *in vitro* sont réalisées en enceinte climatisée, dans les conditions définies ci-dessous:
- intensité lumineuse de 30 µE. m⁻². S⁻¹; photopériode de 16h;
- thermopériode de 26°C le jour, 24°C la nuit.

La technique de transformation utilisée est dérivée de celle de Horsch *et al.* (1985).

Une préculture d'*Agrobacterium tumefaciens* souche LBA4404 contenant les plasmides pBIOC29 ou pBIOC26 ou pBIOC25 est réalisée durant 48h à 28 °C sous agitation, dans du milieu LB (Sambrook *et al.,* 1989) additionné des antibiotiques adéquats (rifampicine et tétracycline). La préculture est ensuite diluée au 50ème dans le même milieu et cultivée dans les mêmes conditions. Après une nuit, la culture est centrifugée (10 min., 3000 g), les bactéries sont reprises dans un volume équivalent de milieu MS30 (30 g/l saccharose) liquide et cette suspension est diluée au 10ème.

Des explants d'environ 1 cm² sont découpés à partir des feuilles des plantules décrites ci-dessus. Ils sont ensuite mis au contact de la suspension bactérienne pendant 1h, puis séchés rapidement sur papier filtre et placés sur un milieu de coculture (MS30 solide).

Après 2 jours, les explants sont transférés en boîtes de Pétri sur le milieu de régénération MS30, contenant un agent sélectif, la kanamycine (200 mg/l), un bactériostatique, l'augmentin (400 mg/l) et les hormones nécessaires à l'induction de bourgeons (BAP, 1 mg/l et ANA, 0,1 mg/l). Un repiquage des explants est effectué sur le même milieu après 2 semaines de culture. Après 2 semaines supplémentaires, les bourgeons sont repiqués en boîtes de Pétri sur le milieu de développement composé du milieu MS20 additionné de kanamycine et d'augrnentin. Après 15 jours, les bourgeons sont repiqués de moitié. L'enracinement prend environ 20 jours, au terme desquels les plantules peuvent être clonées par bouture d'entre-noeuds ou sorties en serre.

### b) Obtention de plantes de tomate transgéniques.

Les graines de tomate cv. UC82B sont stérilisées au Domestos 10% 15 min. et rincées 3 fois dans de l'eau stérile. Le dernier rinçage est effectué pendant 10 min. sous agitation.

Les graines ainsi stérilisées sont mises à germer sur milieu MSSV/2 (milieu de base de Murashige et Skoog (1962, Sigma référence M6899)/2 additionné des vitamines de Nitsch (Thomas et Pratt, 1981), de saccharose à 30 g/l, d'agar (Merck) à 8 g/l, pH5,9, pendant 7 ou 8 jours en chambre climatisée (intensité lumineuse de 30 µE.m⁻², s⁻¹, photopériode de 16 h/8 h, 26°C).

La technique de transformation utilisée est dérivée de celle de Fillatti *et al*. (1987).

Une préculture *d'Agrobacterium tumefaciens* souche LBA4404 contenant les plasmides **pBIOC25** ou **pBIOC26** est réalisée pendant 24 h à 28°C sous agitation dans du milieu LB additionné des antibiotiques adéquats (rifampicine et tétracycline). La préculture est ensuite diluée au 50ème dans le même milieu et cultivée dans les mêmes conditions pendant une nuit. La DO à 600 nm est mesurée, les agrobactéries sont centrifugées (10 min, 3000 g) et repris dans du milieu KCMS liquide (décrit dans la publication de Fillatti *et al.,* 1987) de manière à obtenir une DO à 600 nm de 0,8.

Des améliorations techniques ont été apportées à certaines étapes du protocole de Fillatti *et al.* (1987).

La préculture des explants et la coculture sont réalisées comme décrit par Fillatti *et al.* (1987) excepté que le milieu KCMS est supplémenté en acétosyringone (200 mM).

Le milieu de lavage 2Z diffère par l'addition de céfotaxime à 500 mg/l au lieu de carbénicilline. Le milieu de développement utilisé est composé du milieu de base de Murashige et Skoog (Sigma MS6899) additionné des vitamines de Nitsch, de saccharose à 20 g/l, de kanamycine à 50 mg/l, d'augmentin à 200 mg/l, d'ANA à 1 mg/l et de zéatine à 0,5 mg/l.

### VII. OBTENTION DE PLANTES DE MAÏS TRANSGENIQUES.

### a) Obtention et utilisation de cal de maïs comme cible pour la transformation génétique.

La transformation génétique du maïs, quelle que soit la méthode employée (électroporation; *Agrobacterium,* microfibres, canon à particules), requiert généralement l'utilisation de cellules indifférenciées en divisions rapides ayant conservé une aptitude à la régénération de plantes entières. Ce type de cellules compose le cal friable embryogène (dit de type II) de maïs.

Ces cals sont obtenus à partir d'embryons immatures de génotype HI II ou (A188 x B73) selon la méthode et sur les milieux décrits par Armstrong (Malze Handbook; (1994) M. Freeling, V. Walbot Eds.; pp. 665-671). Les cals ainsi obtenus sont multipliés et maintenus par repiquages successifs tous les quinze jours sur le milieu d'initiation.

Des plantules sont ensuite régénérées à partir de ces cals en modifiant l'équilibre hormonal et osmotique des cellules selon la méthode décrite par Vain et al. (Plant Cell Tissue and Organ Culture (1989, 18:143-151). Ces plantes sont ensuite acclimatées en serre où elles peuvent être croisées ou autofécondées.

### b) Utilisation du canon à particules pour la transformation génétique du maïs.

Le paragraphe précédent décrit l'obtention et la régénération des lignées cellulaires nécessaires à la transformation ; on décrit ici une méthode de transformation génétique conduisant à l'intégration stable des gènes modifiés dans le génome de la plante. Cette méthode repose sur l'utilisation d'un canon à particules identique à celui décrit par J. Finer (Plant Cell Report (1992) 11 : 323-328) ; les cellules cibles sont des fragments de cals décrits dans le paragraphe 1. Ces fragments d'une surface de 10 à 20 mm² ont été disposés, 4 h avant bombardement, à raison de 16 fragments par boite au centre d'un boite de petri contenant un milieu de culture identique au milieu d'initiation, additionné de 0,2 M de mannitol + 0,2 M de sorbitol. Les plasmides portant les gènes à introduire sont purifiés sur colonne Qiagen®, en suivant les instructions du fabricant. Ils sont ensuite précipités sur des particules de tungsten (M10) en suivant le protocole décrit par Klein (Nature (1987) 327:70-73). Les particules ainsi enrobées sont projetées vers les cellules cibles à l'aide du canon et selon le protocole décrit par J. Finer (Plant Cell Report (1992) 11:323-328).

Les boites de cals ainsi bombardées sont ensuite scellées à l'aide de Scellofrais® puis cultivées à l'obscurité à 27°C. Le premier repiquage a lieu 24 h après, puis tous les quinze jours pendant 3 mois sur milieu identique au milieu d'initiation additionné d'un agent sélectif dont la nature et la concentration peuvent varier selon le gène utilisé (voir paragraphe 3). Les agents sélectifs utilisables consistent généralement en composés actifs de certains herbicides (Basta®, Round up®) ou certains antibiotiques (Hygromycine, Kanamycine...).

On obtient après 3 mois ou parfois plus tôt, des cals dont la croissance n'est pas inhibée par l'agent sélection, habituellement et majoritairement composés de cellules résultant de la division d'une cellule ayant intégré dans son patrimoine génétique une ou plusieurs copies du gène de sélection. La fréquence d'obtention de tels cals est d'environ 0,8 cal par boite bombardée.

Ces cals sont identifiés, individualisés, amplifiés puis cultivés de façon à régénérer des plantules (cf. paragraphe a). Afin d'éviter toute interférence avec des cellules non transformées toutes ces opérations sont menées sur des milieux de culture contenant l'agent sélectif.

Les plantes ainsi régénérées sont acclimatées puis cultivées en serre où elles peuvent être croisées ou autofécondées.

### VIII. ANALYSE DE L'EXPRESSION DE LA LIPASE GASTRIQUE DE CHIEN DANS LES PLANTES DE TABAC TRANSGENIQUES.

### a) Protocole.

Le protocole d'extraction de la lipase à partir de feuilles de tabac prélevées sur des plantes en serre est le suivant: 1 g de feuilles (poids frais) est broyé dans l'azote liquide puis à 4°C dans 5 ml de tampon Tris-HCl 25 mM pH 7,5, additionné d'EDTA 1 mM et de mercaptoéthanol 10 mM (tampon A) ou de tampon glycine-HCl 25 mM pH 3, additionné d'EDTA 1 mM, de β-mercaptoéthanol 10 mM, de Triton X-100 0,2% et de NaCl 250 mM (tampon B). Le broyat total est immédiatement centrifugé à 4°C pendant 15 min à 10000 g.

Pour les graines de tabac, l'extraction est réalisée dans le tampon B à raison de 0,1 g de graines pour 4 ml de tampon.

L'activité lipasique est déterminée à l'aide d'un pH-STAT par la méthode titrimétrique de Gargouri *et al*. (1986) dans laquelle le substrat utilisé est la tributyrine. L'émulsion de tributyrine (4 ml pour 30 ml d'émulsion) est réalisée au vortex en présence de sels biliaires (1,04 g/l) d'albumine bovine (0,1 g/l) et de NaCl (9 g/l). Le dosage consiste à neutraliser l'acide butyrique libéré sous l'action de la lipase par une solution de soude à un pH de consigne de 5,5 et à 37°C. Une unité lipase correspond à la quantité d'enzyme qui provoque la libération d'une micromole d'acides gras en 1 min. à 37°C et dans les conditions optimales de pH (5,5). La lipase gastrique de chien naturelle (purifiée) a une activité spécifique de 570 unités/mg de protéine. L'activité lipase peut être mesurée sur le broyat total, sur le culot ou sur le surnageant de centrifugation.

Sur le surnageant de centrifugation (tampon A), un dosage des protéines totales solubles est réalisé par la méthode de Bradford (1976).

Un test ELISA en sandwich (Carrière *et al*., 1993) est également réalisé sur le surnageant de centrifugation avec 2 populations d'anticorps polyclonaux anti-LGC naturelle. La première population inclut les anticorps réagissant avec la lipase gastrique humaine et ont été purifiés par affinité à partir de l'antisérum total sur lipase gastrique humaine greffée sur colonne Affigel 10 (Aoubala *et al.,* 1993). Ces anticorps sont utilisés pour revêtir les puits de plaques ELISA à une concentration de 1 µg/ml. La 2ème population comprend les anticorps qui ne connaissent pas la lipase humaine. Ils sont purifiés ultérieurement sur colonne de Sépharose couplés à la protéine A puis couplés à la biotine. La fixation de l'anticorps est mise en évidence par le biais d'un conjugué streptavidine/péroxydase dont l'activité enzymatique est révélée au moyen du substrat o-phénylènediamine. Les résultats obtenus ont une valeur qualitative et sont notés à l'aide des symboles + et -.

Le rendement d'extraction peut être amélioré en ajoutant dans le tampon d'extraction un détergent de type CHAPS (3-(3-cholamidopropyl)diméthyl-ammonio-1-propane sulfonate)(SIGMA). En effet, dans ce cas, les rendements d'extraction dans le surnageant augmentent d'environ 100% (cf. tableau 1).

**Tableau 1**

| Activité lipase (U/gPF) dans les extraits obtenus à partir de 4 plantes de tabac issues de la transformation génétique avec pBIOC25, en présence de CHAPS 1%. | | |
|---|---|---|
| Plantes | NaCl 0.2M/EDTA 1 mM pH 3.0 | NaCl 0.2M/EDTA 1 mM pH 3.0 + CHAPS 1% |
| 1 | 80 | 112 |
| 2 | 40 | 104 |
| 3 | 43 | 109 |
| 4 | 54 | 92 |

### b) Expression avec les peptides signaux végétaux ; dosages sur les feuilles et les graines de tabac transformées avec pBIOC25 et pBIOC26; test ELISA.

Les résultats obtenus sur 98 plantes T0 du génotype Xanthi (stade 15 à 20 feuilles) sont donnés dans le tableau 2. Les dosages ont tous été réalisés sur les broyats de feuilles ou de graines avant centrifugation. Pour chaque construit, les activités mesurées présentent une grande variabilité suivant les transformants. Environ 20% des plantes n'ont aucune activité ou une activité trop faible pour être décelée. Les activités moyennes et les activités maximales sont données dans le tableau 2.

Les quantités de protéines totales sont similaires pour les 3 construits avec des moyennes de 7 et 8 mg/g PF dans les feuilles et de 34, 31 et 38 mg/g PF dans les graines.

L'activité lipase moyenne dans les feuilles transformées avec le construit pBIOC26 est de 34 U/g PF soit 0,8% d'expression par rapport aux protéines totales. Dans les graines, l'activité moyenne est de 36 U/g PF soit 0.2%. L'activité maximale obtenue sur un des transformants est de 146 U/g PF (feuilles; 2,5% d'expression) et de 148 U/g PF (graines; 0,7% d'expression).

Les activités enzymatiques analysées sur les plantes transformées avec le construit pBIOC25 sont similaires. L'activité moyenne dans les feuilles est de 34 U/g PF (0,8% d'expression) et l'activité maximale de 134 U/g PF (3% des protéines totales). Dans les graines, l'activité moyenne est de 42 U/g PF (0,3 %) et l'activité maximale de 159 U/g PF (1%).

Pour les plantes transformées avec le construit pBIOC29, aucune activité n'est décelée dans les feuilles (promoteur semence-spécifique). Dans les graines, l'activité moyenne est de 12 U/g PF (0,1% d'expression) et l'activité maximale de 137 U/g PF (0,7%).

L'expression dans les feuilles et les graines d'un même événement de transformation est généralement bien corrélée.

Les résultats des tests ELISA concordent également avec ceux des dosages d'activité enzymatique, c'est-à-dire qu'une plante présentant une activité lipase donne un résultat positif en test ELISA.

Des résultats obtenus ultérieurement sur ces mêmes plantes montrent que l'activité lipase est susceptible de varier au cours du développement de la plante. En effet, une plante dont l'expression par rapport aux protéines totales était de 3% au stade 12-15 feuilles, a donné une expression de 6% lors d'un dosage ultérieur (plante plus âgée ayant fleuri).

**TABLEAU 2:**

| EXPRESSION DE LA LIPASE DANS LES FEUILLES ET LES GRAINES DE TABAC XANTHI. | | | | |
|---|---|---|---|---|
| CONSTRUIT | ORGANE | PROTEINES TOTALES mg/g PF | ACTIVITE LIPASE | |
| | | | U/g PF | Expression (% protéines totales) |
| | | min-max (moyenne) | min-max (moyenne) | min-max (moyenne) |
| pBIOC26 | Feuilles | 3 - 15 | 0 - 145,6 | 0 - 2,5 |
| | (n = 34) | (7) | (34,4) | (0,8) |
| | Graines | 24 - 43 | 0 - 147,6 | 0 - 0,7 |
| | (n = 34) | (34) | (36,3) | (0,2) |
| pBIOC25 | Feuilles | 3 - 18 | 0 - 133,5 | 0 - 3 |
| | (n = 35) | (8) | (34) | (0,8) |
| | Graines | 17 - 35 | 0 - 158,5 | 0 - 1 |
| | (n = 35) | (31) | (41,9) | (0,3) |
| pBIOC29 | Graines | 29 - 55 | 0 - 137,4 | 0 - 0,7 |
| | (n = 29) | (38) | (11,8) | (0,1) |
| PF, poids frais; min., valeur obtenue pour l'événement de transformation exprimant le moins; max., valeur obtenue pour l'événement de transformation exprimant le plus; n, nombre d'événements de transformation analysés. | | | | |

### c) Expression avec le peptide signal de la LGL ; dosages d'activité lipase sur les feuilles de tabac.

Les résultats obtenus sur 44 plantes T0 des génotypes Xanthi et pBD6 (stade 15 à 20 feuilles) sont les suivants :

Les dosages ont tous été réalisés sur les broyats de feuilles avant centrifugation. Les activités analysées présentent une grande variabilité suivant les transformants. Environ 20% des plantes n'ont aucune activité ou une activité trop faible pour être décelée. L'activité lipase moyenne dans les feuilles transformées avec le construit pBIOC41 est de 38 U/g PF pour le génotype Xanthi et de 48 U/g PF pour le génotype PBD6. Les activités maximales sont respectivement de 152 et 226 U/gPF.

### IX. ANALYSE DE L'EXPRESSION DE LA LIPASE GASTRIQUE DE CHIEN DANS LES PLANTES DE TOMATE TRANSGENIQUES.

### a) Protocole.

Le protocole d'extraction de la lipase à partir de feuilles et des fruits de tomate est similaire à celui décrit pour les feuilles de tabac, excepté que 1 g de matériel frais est repris dans 4 ml de tampon B. L'activité lipasique est déterminée comme décrit pour les feuilles de tabac.

### b) Dosage dans les fruits de tomate.

Les fruits d'une trentaine de transformants primaires ont été analysés.

L'activité lipase dosée dans les fruits est variable suivant les fruits sur un même transformant. Elle est en moyenne de 5 U/g PF pour les fruits mûrs et de 35 U/g PF pour les fruits verts, indépendamment du construit testé (pBIOC25 ou pBIOC26).

Il est à noter qu'au cours du mûrissement du fruit, l'activité diminue. Par exemple, pour un transformant primaire donné, l'activité lipase est de 132 U/g PF pour un fruit vert de 10 mm de diamètre, de 44 U/g PF pour un fruit rouge-vert de 33 mm de diamètre et de 36 U/g PF pour un fruit rouge de 45 mm de diamètre.

### X. IMMUNODETECTION DE TYPE "WESTERN" DE LA LGC RECOMBINANTE.

### a) LGC exprimée dans les feuilles et graines de tabac et de colza transgéniques.

### a.1) Expression avec les peptides signaux végétaux ; immunodétection dans les feuilles et graines de tabac et de colza transformées avec pBIOC25, pBIOC26 et pBIOC29.

Des expériences d'immunodétection de type "western" ("western-blots") (Renart et Sandoval, 1984) de la lipase gastrique de chien ont été réalisées sur les protéines de feuilles de tabac et de graines de tabac et de colza extraites avec les tampons A et B (voir protocole d'extraction ci-dessus). Pour la réalisation de ces expériences, les protéines extraites (30 µg protéines totales par échantillon) sont tout d'abord séparées sur gel de polyacrylamide dénaturant à 12,5% selon la technique de Laemmli U.K. (1970) puis transférés sur une membrane de nitrocellulose. Un anticorps polyclonal anti-lipase de chien obtenu chez le cobaye est utilisé comme sonde et la révélation est effectuée au moyen d'un anticorps anti-IgG de cobaye marqué à la phosphatase alcaline.

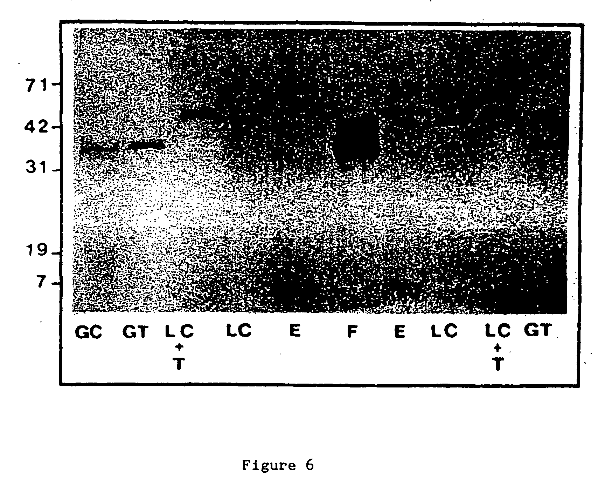

La protéine témoin est la lipase gastrique de chien naturelle (LC. Fig. 6) qui migre sous la forme d'une seule bande d'un poids moléculaire apparent d'environ 50 kDa. La migration de la lipase gastrique de chien est légèrement retardée en présence d'extrait de feuilles de tabac non transformées (Fig. 6, LC + T).

Aucune bande n'est détectée dans les extraits protéiques de feuilles et de graines de tabac et de colza non transformées. La lipase produite dans les feuilles de tabac se présente sous la forme de 2 bandes. La bande la plus importante quantitativement a un poids moléculaire apparent d'environ 37 kDa et correspond au polypeptide (Δ54) susmentionné. La bande mineure a un poids moléculaire apparent d'environ 49 kDa et correspond au polypeptide (Δ4) susmentionné (Fig. 6, F). Dans les extraits protéiques de graines, seule la bande de poids moléculaire inférieur est visible (Fig. 6, GT et GC).

### a.2) Expression avec le peptide signal de la LGL ; immunodétection dans les feuilles et les graines de tabac transformées avec pBIOC41

Des western blots (Renart et Sandoval, 1984) ont été réalisés sur les protéines de feuilles et de graines de tabac extraites avec le tampon B (voir protocole d'extraction ci-dessus). Les protéines extraites sont tout d'abord séparées sur gel de polyacrylamide dénaturant (SDS-PAGE) selon la technique de Laemmli (1970) puis transférées sur une membrane de nitrocellulose. Un anticorps polyclonal anti-lipase de chien obtenu chez le cobaye est utilisé comme sonde et la révélation est effectuée au moyen d'un anticorps anticobaye marqué à la phosphatase alcaline.

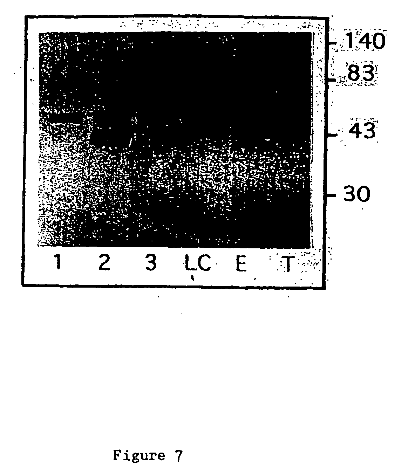

Un exemple de western blot de feuilles de tabac est montré sur la figure 7. La protéine témoin est la lipase gastrique de chien qui migre sous la forme d'une seule bande de poids moléculaire apparent d'environ 50 kDa. Aucune bande n'est détectée dans les extraits protéiques de feuilles de tabac non transformées (T). La lipase produite dans les extraits de feuilles se présente sous la forme d'une bande majeure de poids moléculaire apparent d'environ 49 kDa, et correspond au polypeptide (D4) susmentionné.

Dans les graines de tabac transformées avec le construit pBIOC41, la lipase recombinante se présente sous la même forme que dans les feuilles.

### b) LGC dans un extrait de protéines de feuilles de tabac transformées: expérience de déglycosylation.

Le protocole d'extraction des protéines pour les expériences de déglycosylation est le suivant: 0,5 g de feuilles (poids frais) sont broyés dans l'azote liquide puis à 4°C dans 1 ml de tampon de dénaturation (tampon phosphate 100 mM pH 7,5, additionné de β-mercaptoéthanol 1%, d'EDTA 25 mM et de SDS 1%). Le broyat est centrifugé à 4°C pendant 15 min à 10000g. Le surnageant est incubé durant 5 min à 100°C afin de réaliser la dénaturation des protéines puis centrifugé 2 min à 10000 g. Le surnageant est ensuite dilué au 10ème dans le tampon de déglycosylation (tampon phosphate 100 mM pH 7,5, additionné de β-mercaptoéthanot 1%, d'EDTA 25 mM, de SDS 0,1% et d'octylglucoside 1%). L'enzyme (N-glycosidase F, PNGase Boehringer) est ajoutée à raison de 1 U pour 100 µl de surnageant. Un témoin sans enzyme est réalisé pour chaque échantillon. La déglycosylation de la protéine témoin (lipase gastrique de chien ou de lapin) a lieu dans les mêmes conditions. Les différents échantillons protéiques sont incubés à température ambiante durant 8 heures. Les protéines sont ensuite séparées par électrophorèse sur gel de polyacrylamide et transférées sur membrane de nitrocellulose comme décrit dans le paragraphe précédent.

D'après les résultats des "western-blots", la lipase gastrique utilisée comme témoin a un poids moléculaire apparent d'environ 50 kDa. Après déglycosylation, son poids moléculaire apparent n'est plus que de 43 kDa environ.

La lipase produite dans les feuilles de tabac, après incubation sans PNGase dans les conditions décrites ci-dessus se présente sous la forme de 3 bandes de poids moléculaires apparents de 49 (polypeptide (Δ4). 37 (polypeptide Δ54) et 28 kDa. La bande de poids moléculaire 28 kDa est sans doute le résultat d'une protéolyse ayant eu lieu durant l'incubation de 8 heures à température ambiante. Après déglycosylation, les poids moléculaires des 3 bandes sont diminués d'environ 1 à 2 kDa, ce qui montre que les protéines produites dans les feuilles de tabac sont glycosylées.

### c) LGC exprimée dans les feuilles et les fruits de tomate transgéniques.

Des expériences d'immunodétection de type "Western" de la lipase gastrique de chien ont été réalisées sur des protéines de feuilles et de fruits de tomate extraites avec le tampon B (voir protocole d'extraction ci-dessus). Pour la réalisation des expériences, les protéines extraites (15 µg et 6 µg de protéines totales solubles pour les feuilles et les fruits respectivement) sont séparées sur gel de polyacrylamide dénaturant comme décrit dans le paragraphe X.a).

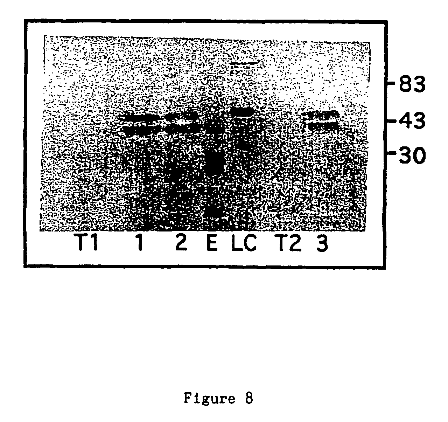

La protéine témoin est la lipase gastrique naturelle (piste 4, Fig. 8) qui migre sous la forme d'une seule bande d'un poids apparent d'environ 50 kDa.

Aucune bande n'est détectée dans les extraits protéiques de feuilles et de fruits de tomate non transformés.

La lipase produite dans les feuilles et les fruits de tomate se présente sous forme de 2 bandes, quel que soit le construit utilisé (pBIOC25 ou pBIOC26). La bande la plus importante quantitativement a un poids apparent d'environ 37 kDa et correspond au polypeptide (Δ54) susmentionné. La bande mineure a un poids moléculaire apparent d'environ 49 kDa et correspond au polypeptide (Δ4) susmentionné (Fig. 8).

### XI. PURIFICATION DE LA LIPASE GASTRIQUE DE CHIEN A PARTIR DE PLANTES.

### a) Purification de la LGC à partir de feuilles de tabac

L'activité de la lipase gastrique de chien produite dans les feuilles de tabac est déterminée selon une méthode titrimétrique, le pH de consigne est fixé à 5,5 et la température à 37°C à l'aide d'un pH-stat (Mettler-Toledo-DL25) en utilisant la tributyrine comme substrat: 1 ml de tributyrine est émulsifié dans 29 ml d'une solution aqueuse de NaCl 0,15 M au vortex. Le dosage consiste à neutraliser l'acide butyrique libéré sous l'action de la lipase, par addition de soude 0,02 N, alors que l'émulsion est maintenue par une agitation mécanique vigoureuse. Une unité lipase correspond à 1 micromole d'acide gras libérée par minute dans ces conditions de pH et de température.

Après la première étape chromatographique, l'activité lipase, avec une prise d'essai de 0,5 ml, est mise en évidence sur une émulsion de 1 ml de tributyrine dans 29 ml d'une solution 0,15 M en NaCl, 2 mM en Taurodeoxycholate de sodium et 1,5 µM en Sérum Albumine Bovine conformément au dosage décrit par Gargouri *et al*. (1986).

Un gramme de feuilles lyophilisées est broyé à 4°C dans 30 ml de tampon glycine 20 mM pH 2,5 et laissé sous agitation douce pendant 15 minutes. Pendant la macération le pH est maintenu à 2,5 par addition d'HCl 1N. Le produit de la macération est centrifugé à 15000 g pendant 5 minutes. Le pH du surnageant est ajusté à 4 par addition de NaOH 1 N. Après filtration sur MIRACLOTH (Calbiochem), la totalité du surnageant est déposée sur une colonne de résine échangeuse de cations (résine S-Sépharose Fast Flow - Pharmacia) de 10 ml (diamètre 1,6 cm) équilibrée dans un tampon acétate de sodium 20 mM pH 4,0, NaCl 20 mM à un débit de 1 ml par minute. Des fractions de 2 ml sont collectées. Après passage du surnageant, la colonne est lavée avec 40 ml du tampon d'équilibration. Les protéines retenues sur la colonne sont éluées suivant le protocole ci-dessous:
- gradient linéaire en tampon acétate de sodium 20 mM pH 4,0 de 20 mM à 210 mM en NaCl en 30 minutes pour l'élution d'un premier ensemble de pics de protéines ne contenant pas d'activité lipasique, le test est effectué sur des prises d'essai de 1 ml,
- plateau à 210 mM en NaCl pendant 20 minutes,
- gradient linéaire en tampon acétate de sodium 20 mM pH 4,0 de 210 mM à 500 mM en NaCl en 30 minutes pour l'élution d'un second ensemble de pics. L'activité lipase, mesurée sur des prises d'essai de 0,5 ml, est éluée lors de ce deuxième gradient, à une force ionique située entre 300 et 400 mM.

Les fractions actives sont rassemblées, concentrées grâce à une cellule de concentration OMEGACELL de limite de poids moléculaire de 30 kDa (Filtron Technology Corporation).

Le concentrat est dialysé 12 heures contre un tampon Tris-HCl 10 mM pH 8 puis appliqué sur une colonne de résine échangeuse d'anions (MonoQ HR 5/5 de diamètre 0,5 cm et de hauteur 5 cm - Pharmacia) équilibrée en tampon Tris-HCl 10 mM pH 8. L'activité lipase est mesurée sur une prise d'essai de 0,5 ml. Le débit est maintenu à 1 ml par minute, soit une pression de 2,0 MPa. Après élution de la fraction non retenue et lavage de la colonne avec 10 ml de tampon Tris-HCl 10 mM pH 8, il est appliqué un gradient linéaire de force ionique de 0 à 400 mM en NaCl en 60 minutes. L'activité lipase est éluée pour une force ionique située entre 100 mM et 200 mM.

Les fractions actives sont rassemblées et concentrées grâce à une cellule de concentration OMEGACELL de limite de poids moléculaire de 30 kDa. Le concentrat constitue la forme purifiée de la lipase gastrique de chien extraite des feuilles de tabac.

La détermination de la concentration en protéines est effectuée selon la méthode de Bradford M. (1976) sur les surnageants de concentration et selon la méthode de Lowry O.H. (1951) pour les solutions après la première chromatographie d'échange d'ions.

Les différentes étapes de la purification sont analysées par électrophorèse sur gel de polyacrylamide dénaturant (SDS-PAGE 12,5%) selon la technique de Laemmli U.K. (1970). Les protéines ainsi séparées sur gel de polyacrylamide sont, d'une part, mises en évidence par coloration au bleu de coomassie, et d'autre part transférées sur membrane de nitrocellulose par la technique d'électrotransfert semi-sec (Transblot SD, BIORAD) en tampon Tris Base 20 mM, Glycine 150 mM et éthanol 20%, à 2,3 mA par cm² de membrane. La lipase gastrique de chien recombinante transférée sur membrane de nitrocellulose est mise en évidence par immunodétection selon le protocole suivant:
- marquage de la protéine cible par un anticorps polyclonal anti-lipase gastrique de chien obtenu chez le cobaye et dilué au 1/5000ème dans du tampon PBS additionné de lait en poudre délipidé à 5 % et de Tween 20 à 0.1% pendant 1 heure à température ambiante,
- rinçage de la membrane dans trois bains successifs de tampon PBS additionné de lait en poudre délipidé à 1% et de Tween 20 à 0,1 % pendant 10 minutes pour chaque bain,
- marquage avec un anticorps anti-cobaye couplé à la péroxydase (Sigma) dilué au 1/2000ème dans du tampon PBS additionné de lait en poudre délipidé à 1% et de Tween 20 à 0,1% pendant 1 heure à température ambiante,
- rinçage de la membrane dans trois bains successifs de tampon PBS additionné de lait en poudre délipidé à 1% et de Tween 20 à 0,1% pendant 10 min pour chaque bain,
- révélation par action de la péroxydase sur le 4-chloro 1-naphtol (Sigma) en présence d'H₂O₂ pour produire une coloration bleue stable dans le temps.

La lipase produite dans les feuilles se présente sous la forme de deux bandes d'environ 49 (polypeptide (Δ4)) kDa et 37 (polypeptide (Δ54)) kDa.

### b) Variante de la purification de la LGC à partir de feuilles de tabac

L'activité de la lipase gastrique de chien produite dans les feuilles de tabac est déterminée selon les méthodes titrimétriques décrites par Gargouri *et al.* (1986) à l'aide d'un pH-stat (METTLER-TOLEDO DL25).

Le dosage sur triglycérides à chaînes courtes est effectué en utilisant la tributyrine comme substrat : 1 ml de tributyrine est émulsifié dans 29 ml d'une solution acqueuse de NaCl 0,15 M, Sérum Albumine Bovine (BSA) 1,5 *µ*M et taurodeoxycholate de sodium (NaTDC) 2 mM. Le pH de consigne est fixé à 5.0, la température à 37°C.

Le dosage consiste à neutraliser l'acide butyrique libéré sous l'action de la lipase par addition de soude 0,02 N alors que l'émulsion est maintenue par une agitation mécanique vigoureuse.

Le dosage sur triglycérides à chaînes longues est effectué en utilisant comme substrat une émulsion aqueuse à 30% d'huile de soja purifiée, 1,2% de phospholipides d'oeuf purifiés, 1,67% de glycerol anhydre (Intralipid™ 30% - PHARMACIA AB Stockholm, Sweden). Dix ml de cette suspension sont émulsifiés dans 20 ml d'une solution aqueuse de NaCl 0,15 M, BSA 30 µM et CaCl₂ 3,5 mM. Le pH de consigne est fixé à 4.0, la température à 37°C.

Le dosage consiste à neutraliser les acides gras libérés sous l'action de la lipase par addition de soude 0,2 N après un saut de pH de 4 à 9 alors que l'émulsion est maintenue par une agitation mécanique vigoureuse.

Une unité lipase correspond à une micromole d'acide gras libérée par minute dans les conditions définies de pH et de température pour chacun des substrats.

Deux grammes de feuilles lyophilisées sont broyés à 4°C dans 60 ml de NaCl 0,2 M, pH 3 et laissés sous agitation douce 15 minutes à 4°C. Pendant cette macération, le pH est maintenu à 3 par addition d'HCl 1N. Le produit de la macération (homogénat) est centrifugé à 10.000 g 10 minutes. Après filtration sur MIRACLOTH (Calbiochem) et filtre MILLIPORE 0,45 *µ*, la totalité du surnageant est injecté sur une colonne de résine échangeuse de cations (RESOURCE S 6 ml -Pharmacia - 16 mm d.i. x 30 mm) équilibrée dans un tampon acétate de sodium 20 mM, NaCl 0,2 M pH 3 à un débit de 8 ml/min (240 cm h⁻¹).

Après passage de la fraction non retenue, la colonne est lavée avec 30 fois son volume de tampon d'équilibration. Les protéines retenues sur la colonne sont éluées avec un gradient linéaire en tampon acétate de sodium 20 mM pH 3 de 0,2 M NaCl à 0,5 M NaCl en 7 volumes colonnes. Des fractions de 4 ml sont collectées.

L'activité lipase mesurée sur des prises d'essai de 0,5 ml, est éluée à une force ionique de 0,35 M en NaCl.

Les fractions actives sont rassemblées et concentrées grâce à une cellule de concentration OMEGACELL (Filtron Technology Corporation) de limite de poids moléculaire de 30 kDa.

La détermination de la concentration en protéines est effectuée selon la méthode de Lowry O.H. (1951).

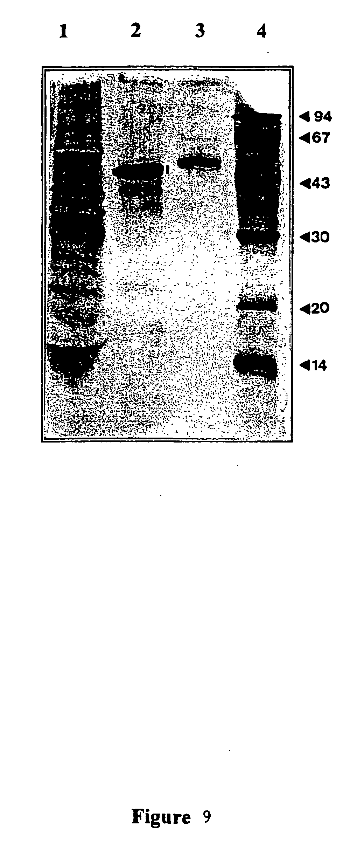

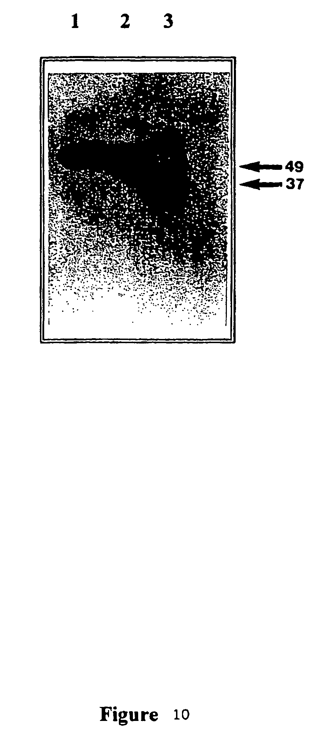

Un exemple de gel de polyacrylamide et le résultat du transfert de ce gel sur membrane de nitrocellulose et révélation à partir d'anticorps anti-lipase gastrique de chien sont montrés (fig. 9 et 10). La révélation a été effectuée par action de la peroxydase sur le Luminol en présence d'activateur (ECL western Blotting - AMERSHAM LIFE SCIENCE) et impression d'un film photographique.
- La présence de résidus glycaniques sur la protéine a été déterminée selon le protocole suivant :
   . immobilisation de la protéine sur membrane de nitrocellulose
   . traitement au périodate
   . réaction spécifique avec la streptavidine couplée à la phosphatase alcaline.
   . réaction colorée de la phosphatase alcaline (GLYCOTRACK-OXFORD GLYLOSYSTEM).

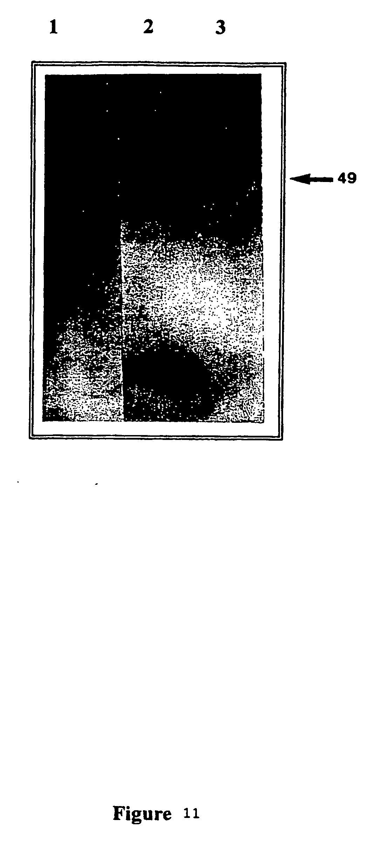

Un exemple de membrane de détection des résidus glycaniques est montré (fig. 11)

La pureté des fractions est assurée par chromatographie liquide haute performance
Chromatographe WATERS 625 LC
Détecteur à barette de diodes WATERS 991
Colonne VYDAC C4
Conditions d'élutions :

| Temps (min) | Débit (ml/min) | % A | % B |
|---|---|---|---|
| 0 | 1 | 100 | 0 |
| 35 | 1 | 40 | 60 |
| 40 | 1 | 40 | 60 |
| 45 | 1 | 20 | 80 |
| 50 | 1 | 100 | 0 |

Tp A : 89.9 % H20 10 % Acétonitrile, 0,1 % acide trifluoroacétique
Tp B : 100 % Acétonitrile.

| Tableau de purification : LGC recombinante | | | | | |
|---|---|---|---|---|---|
| | Unités * totales | mg de protéines | Activité spécifique | Facteur de purification | Rendement |
| Homogénat | 3200 | / | / | / | / |
| Surnageant | 2800 | 230 | 13 | 1 | 88 % |
| sortie Resource S | 1600 | 6 | 250 | 20 | 50 % |

| | | | | | |
|---|---|---|---|---|---|
| * Unités tributyrine | | | | | |

Activités spécifiques comparées de la lipase gastrique de chien naturelle (n-LGC) et de la lipase gastrique de chien recombinante (r-LGC)

| | n-LGC* | r-LGC extraite des feuilles de tabac |
|---|---|---|
| Tributyrine | 570 U/mg | 250 U/mg |
| Intralipide 30 % | 1000 U/mg | 950 U/mg |

| | | |
|---|---|---|
| * réf. : Carrière et al (1991) Eur. J. Biochem, 202, 75-83. | | |

### c) Purification de la LGC à partir de graines de Colza

L'activité de la LGC recombinante produite dans les graines de colza est déterminée de la même façon que dans le cas d'une extraction à partir de feuilles.

Dix grammes de graines de colza sont broyés dans l'azote liquide. La farine obtenue est délipidée à l'hexane par une première macération à 4°C sous agitation douce pendant 12 heures. Le tout est décanté, l'hexanne est éliminé. La farine est rincée deux fois avec 100 ml d'hexanne sous agitation douce 1/2 heure à 4°C pour chaque rinçage. L'hexanne est éliminé par décantation. La farine est séchée à l'évaporateur rotatif (HEIDOLPH 94200). La farine délipidée se conserve à -20°C.

L'extraction de la LGC est effectuée à partir de la farine délipidée par macération à 4°C dans une solution aqueuse à 0,2 M de NaCl pH3 (HCl 1N) pendant 30 minutes à raison de 2 ml de solution aqueuse pour 0,1 g de farine. Le résultat de la macération est centrifugé à 10 000 g pendant 10 minutes à 4°C.

Le culot est éliminé. Le surnageant constitue l'extrait de graines.

L'extrait de graines est dialysé contre un tampon acétate de sodium 10 mM pH4, NaCl 140 mM, KCl 3mM, puis est appliqué sur une colonne d'immunoaffinité constituée par des anticorps polyclonaux anti-lipase gastrique de chien obtenus chez le cobaye couplés à une résine (hydrazide Avidgel - BIOPROBE INTERNATIONAL, Inc.).

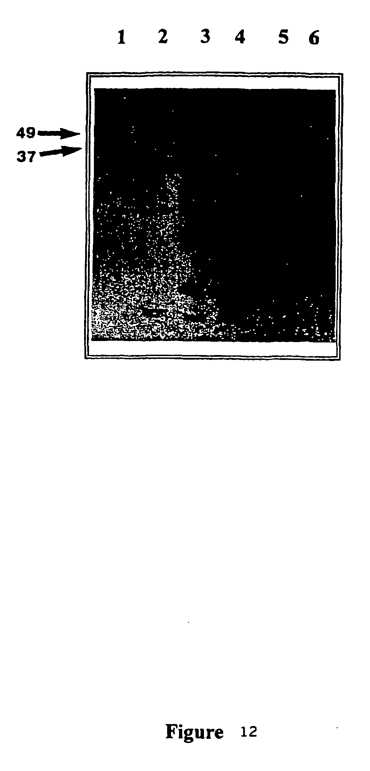

Le contact résine/extrait de graine est de 30 minutes à 4°C sous agitation douce. La résine est ensuite rincée avec 10 volumes colonne de tampon acétate 10 mM, pH4, NaCl 150 mM, KCl 3 mM. La LGC est éluée avec 5 volumes colonne de tampon glycine 0,2 M, pH 2,8, NaCl 150 mM. Les fractions de collecte ont un volume égal à 1 volume colonne et contiennent 1/20ème V/V de tampon Tris 1M pH9. L'analyse par électrophorèse sur gel de polyacrylamide en milieu dénaturant (cf. fig. 12) montre une protéine de poids moléculaire d'environ 37 kDa.

### XII. SYNTHESE D'ESTERS D'ACIDES GRAS.

Les essais ont été réalisés avec des graines de colza non transformées, la lipase étant apportée:
- soit sous forme de préparation enzymatique immobilisée (lipozyme (NOVO)) ou libre (lipase gastrique de lapin (JO 4002)),
- soit sous forme de graines de tabac transformées avec le gène de la lipase gastrique de chien (tabac T14-44 0,85% d'expression).

Les réactions d'estérification sont conduites à 37°C pendant 16 heures dans des flacons de verre bouchés hermétiquement disposés sur une table d'agitation (250 rpm). Le solvant organique utilisé est l'hexane dans lequel les acides gras sont solubles. Le méthanol est ajouté en proportion stoechiométrique à la quantité théorique de triacylglycérol contenu dans les graines de colza.

Le composant majeur des acides gras de colza est l'acide oléique, aussi le témoin de référence choisi est-il un méthylester d'acide oléique. Le suivi de synthèse se fait par chromatographie couche mince (CCM). Le solvant de migration est un mélange d'hexane, diéthyléther, eau (70:10:1). La révélation des plaques se fait à chaud après pulvérisation d'acide sulfurique (5%) dans de l'éthanol.

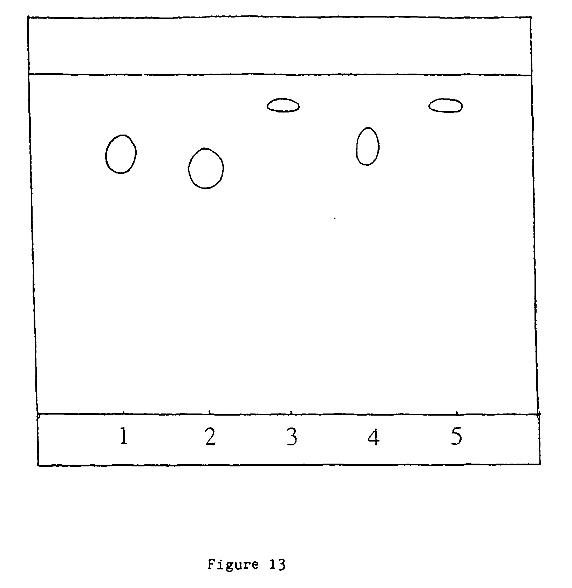

Dans un premier essai, à 0,2 g d'huile de colza (0,22 mmole) sont ajoutés 27 µl de méthanol (0,66 mmole) et 0,02 g de lipozyme: aucune tache n'apparaît au niveau du méthylester oléique de référence (Figure 13, colonne 2).

Dans le deuxième essai, l'huile de colza est remplacée par 0,5 g de graines de colza broyées à sec et 1 ml d'hexane est ajouté: il y a synthèse d'un ester méthylique (Figure 13, colonne 5).

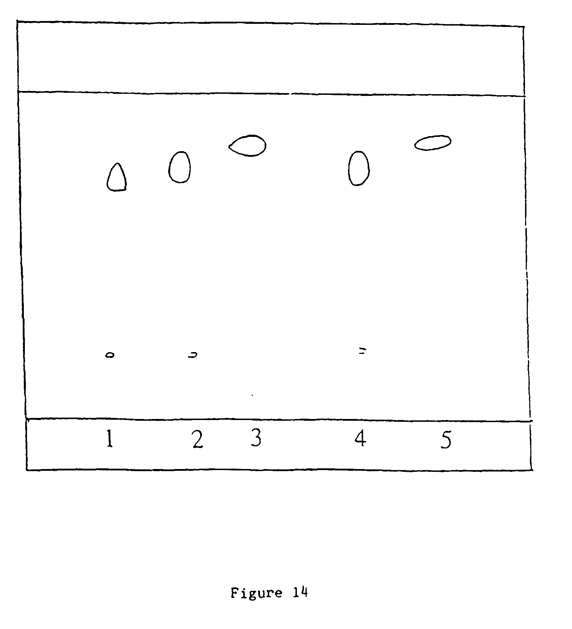

Dans les essais suivants, les conditions ci-dessus sont reprises avec les modifications suivantes: la quantité de lipozyme est réduite à 0,006 g et le lipozyme est remplacé par la lipase gastrique de lapin (0,007 g de JO 4002). Il y a synthèse de méthylester oléique en présence de lipozyme mais pas en présence de JO 4002 (Figure 14, colonne 2).

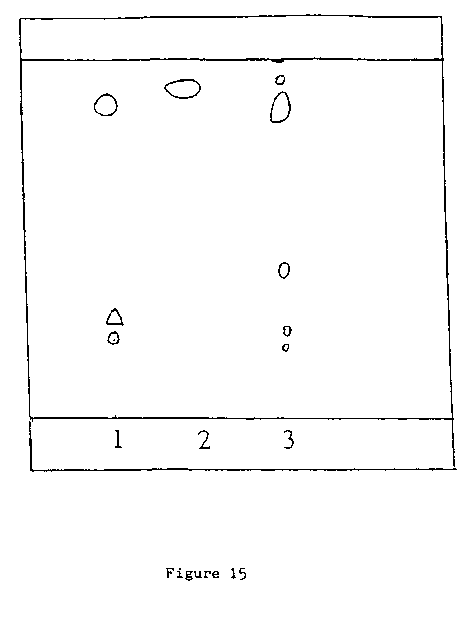

Enfin, dans les derniers essais, la lipase est apportée par des graines de tabac transformées (1 g de graines de tabac). Une tache caractéristique d'un ester méthylique apparaît (Figure 15, colonne 3).

En conclusion, les résultats décrits ci-dessus montrent que lorsque l'on met en présence des extraits de graines de colza, de l'alcool et de la lipase gastrique de chien recombinante produite par des tabacs transgéniques, on obtient une réaction d'estérification qui conduit à la synthèse d'un ester méthylique pouvant être utilisé comme biocarburant.

### Légende des figures:

- figure 1: séquence nucléotidique de l'ADNc codant pour la LGC,
- figure 2: séquence en acides aminés de la LGC,
- figure 3: séquence nucléotidique dérivée de l'ADNc codant pour la LGC, et codant pour la LGC représentée sur la figure 2,
- figure 4 : séquence nucléotidique de l'ADNc codant pour la LGH, et séquence en acides aminés de la LGH,
- figure 5 : séquence en acides aminés de la LGH,
- figure 6 : immunodétection des polypeptides recombinants produits par les feuilles et graines de tabac Xanthi, et de graines de colza transformées avec les construits pBIOC26, pBIOC25 et pBIOC29; E, échelle de poids moléculaire; LG, lipase gastrique de chien; F, feuilles de tabac et GT, graines de tabac transformées avec le construit pBIOC25; GC, graines de colza transformées avec le construit pBIOC29; T, feuilles de tabac non transformées,
- figure 7 : immunodétection des polypeptides recombinants produits par les feuilles de tabac transformées avec les construits pBIOC25 et pBIOC41; E, échelle de poids moléculaire; LC, lipase gastrique de chien; 1 et 3, feuilles de tabac transformées avec le construit pBIOC41; 2, feuilles de tabac transformées avec le construit pBIOC25; T, feuilles de tabac non transformées,
- figure 8 : immunodétection des polypeptides recombinants produits par les feuilles et les fruits de tomate transformés avec les construits pBIOC25 et pBIOC26 :
   T1 : fruit de tomate non transformé
   1 et 3 : fruits de tomate transformés
   2 : feuille de tomate transformée
   E : échelle de poids moléculaire
   LC : lipase gastrique de chien
   T2 : feuille de tomate non transformée.
- figure 9 : analyse par électrophorèse sur gel de polyacrylamide en milieu dénaturant (SDS PAGE).
   1: extrait de graines de colza.
   2 : lipase gastrique de chien recombinante produite à partir des feuilles de tabac
   3 : lipase gastrique de chien naturelle
   4 : échelle de poids moléculaire.
- figure 10 : révélation immunologique de l'analyse précédante après transfert sur membrane de nitrocellulose.
   1 : lipase gastrique de chien naturelle
   2 : lipase gastrique de chien recombinante produite à partir des feuilles de tabac
   3 : extrait de graines de colza
- figure 11 : révélation de la présence de résidus glycaniques à partir d'un gel de plolyacrylamide après transfert sur membrane de nitrocellulose.
   1 : extrait de graines de colza
   2 : lipase gastrique de chien recombinante produite à partir des feuilles de tabac
   3 : lipase gastrique de chien naturelle
- figure 12 : analyse SDS-PAGE de l' immunopurification de la r-DGL produite dans les graines de colza.
   1 : lipase gastrique de chien naturelle
   2 : lipase gastrique de chien recombinante
   3 à 6 : fractions non retenues éluées de la colonne d'immunopurification.
- figure 13: révélation d'une plaque CCM; 1: huile de colza, pas d'enzyme; 2: huile de colza + lipozyme; 3: méthylester d'acide oléique; 4: graines de colza, pas d'enzyme; 5: graines de colza + lipozyme,
- figure 14 : révélation d'une plaque CCM; 1 et 4: graines de colza sans enzyme; 2: graines de colza + JO4002; 3: méthylester d'acide oléique; 5: graines de colza + lipozyme,
- figure 15 : révélation d'une plaque CCM; 1: monooléine, dioléine, trioléine; 2: méthylester d'acide oléique; 3: graines de colza + graines de tabac transformées.

### REFERENCES BIBLIOGRAPHIQUES

An *et al.* Plant Physiol., **81**, 301-305 (1986).
An G., Plant Physiol., **81,** 86-91 (1986).
Aoubala M., Daniel C., De Caro A., Ivanova M.G., Hirn M., Sarda L. et Verger R., Eur. J. Biochem. **211,** 99-104 (1993).
Barta *et al.,* Plant Mol. Biol., **6,** 347-357 (1986).
Bednarek S. Y. et Ralkhel N. V., The Plant Cell, **3,** 1195-1206 (1991).
Berg D.E., Berg C.M., Biotechnology, **1**, 417-435 (1983).
Bernard C., C.R. Acad. Sci., **28,** 249-253 (1849).
Bevan *et al.,* Nature, **304,** 184-187 (1983).
Bevan M., Nucleic Acids. Res., **12,** 8711-8721 (1984).
Birnboim H.C., Doly J., Nucl. Acids. Res., **7,** 1513-1523 (1979).
Bodmer M.W. *et al.,* Biochem Biophys.Acta, **909,** 237-244 (1987).
Bradford M., Anal Biochem., **72,** 248 (1976).
Brodelius *et al.,* FEBS Letters, **103**, 93-97 (1979).
Brodelius, In: Moo-Young M. (Ed), Bioreactor Immobilized Enzymes and Cells: Fundamentals and Applications, Elsevier, Londres (1988).
Carrière *et al.,* Eur. J. Biochem., 202, 75-83 (1991).
Carrière F., Laugier R., Barrowman J.A., Douchet I., Piymenko N. et Verger R., Scand. J. Gastroenterology, **28,** 443-454 (1993).
Close J.J., Rodriguez R.L., Gene, **20,** 305-316 (1982). De La Penna *et al.,* Nature, 325, 274-276 (1987).
Deno *et al.,* J. Plant. Physiol., **131**, 315-322 (1987).
Depicker *et al.,* J. Mol. Appl. Genet., **1,** 561-573 (1982).
Depigny-This et al., Plant. Mol. Biol., **20,** 467-479 (1992).
De Zoeten *et al.,* Virology, **172,** 213-222 (1989).
Docherty A.J.P. *et al.,* Nucl. Ac. Res., **13,** 1891-1903 (1985).
Edelbaum *et al.,* J. of Interferon Research, **12,** 449-453 (1992).
Franck *et al.* Cell, **21,** 285-294 (1980).
Fillatti J.J., Kiser J., Rose R. et Comai L., Biotechnologie, **5,** 726-730 (1987).
Gamborg O.L., Miller R.A. et Ojima K., Exp. Cell. Res., **50,** 151-158 (1968).
Gargouri Y., Pieroni G., Rivière C., Saunière J-F., Lowe P.A., Sarda L. et Verger R., Gastroenterology, **91,** 919-925 (1986).
Gargouri Y. *et al.,* Biochem. Biophys. Act., **1006,** 255-271 (1989).
Gaubier et al., Mol. Gen. Genet., **238,** 409-418 (1993).
Giller et al., J. Biol. Chem., **267,** 16509-16516 (1992).
Guerineau F., Mullineaux P., Plant Molecular Biology LABFAX, Groy R.R.D. (Ed), Nios. Scientific Publishers, Blackwell Scientific Publications, 121-147 (1993).
Hanahan D., J. Mol. Biol., **166,** 557-580 (1983).
Hein R., International Meeting of Production of Recombinant Proteins in Plants, Leicester, p. 22 (1994).
Herrera-Estrella *et al.,* Nature, **303,** 209-213 (1983a).
Herrera-Estrella *et al.,* EMBO J., **2,** 987-995 (1983b).
Hiatt et Ma, FEBS, **307,** 71-75 (1992).
Hiatt *et al.,* Nature, **342,** 76-79 (1989).
Higo *et al.,* Biosci. Biochem., **57,** 1477-1481 (1993).
Holsters *et al.,* Mol. Gen. Genet., **163,** 181-187 (1978).
Horsch R.B., Fry J.E., Hoffmann N.L., Eichholtz D. et Rogers S.G., Science, **227,** 1229-1231 (1985).
Jouanin *et al.,* Plant Sci., **53,** 53-63 (1987).
Kay *et al.,* Science, **236,** 1299-1302 (1987).
Laemmli U.K., Nature, **227,** 680-685 (1970).
Liu *et al.,* Mol. Plant Microb. Interactions, **6,** 144-156 (1993).
Lowry O.H., J. Biol. Chem., **173,** 265-275 (1951).
Ma *et al.,* International Meeting of Production of Recombinant Proteins in Plants, Leicester, p. 39-40 (1994).
McElroy et al., Mol. Gen. Genet., **231,** 150-160 (1991).
Marx, Science, **216,** 1305-1307 (1982).
Mason *et al.,* Proc. Natl. Acad. Sci. USA, **89,** 11745-11749 (1992).
Matsuoka K., Nakamura K., Proc. Natl. Acad. Sci. USA, **88,** 834-838 (1991).
Moloney *et al.,* International Meeting of Production of Recombinant Proteins in Plant, Leicester, p. 36-38 (1994).
Moreau H. *et al.,* Biochem. Biophys. Act., **960,** 268-293 (1988).
Murakami *et al.,* Plant Mol. Biol., **7,** 343-355 (1986).
Murashige T. et Skoog F., Physiol. Plantarum, **15,** 473-497 (1962).
Ni *et al.,* Plant J., **7,** 661-676 (1995).
Reina *et al.,* Nucleic Acid Research, **18,** 6426 (1990).
Renart J. et Sandoval I.V., Meth. Enzymol., **104,** 455-460 (1984).
Russel D., International Meeting of Production of Recombinant Proteins in Plants, Leicester, p. 43 (1994).
Sambrook *et al.,* Molecular Cloning Laboratory Manual; 2^{nd} Edition, Cold Spring Harbor Laboratory Press (1989).
Sanford J.C., Trends in Biotechnology, **6,** 299-302 (1988).
Sanger *et al.,* Proc. Natl. Acad. Sci., 74, 5463-5467 (1977).
Schroeder M. R., Borkhsenious O. N., Matsuoka K., Nakamura K. et Ralkhel N. V., Plant Physiol., **101,** 451-458 (1993).
Shonheyder F., et Volquartz K., Acta Physiol. Scand., **9,** 57-67 (1945).
Sijmons *et al.,* Biotechnology, **8,** 217-221 (1990).
Thomas B.R. et Pratt D., Theor. Appl. Genet., **59,** 215-219 (1981).
Truve *et al.,* Biotechnology, **11,** 1048-1052 (1993).
Vandekerckhove *et al.,* Biotechnology, 7, 929-932 (1989).
Volhard F., Z. Klin. Med., **42,** 414-429 (1901).

## Revendications

1. Utilisation d'une séquence nucléotidique recombinante représentée sur la figure 3 et codant pour la lipase gastrique de chien (LGC) représentée sur la figure 2 d'une part, et d'autre part, les éléments permettant à une cellule végétale de produire la lipase gastrique de chien codée par ledit ADNc, notamment un promoteur et un terminateur de transcription reconnus par la machinerie transcriptionnelle des cellules végétales, pour la transformation de cellules de plantes en vue de l'obtention, à partir de ces cellules, ou de plantes obtenues à partir de ces dernières, de LGC recombinante sous forme d'enzyme active.

2. Séquence nucléotidique recombinante, **caractérisée en ce qu'**elle contient d'une part, l'ADNc représenté sur la figure 3 et codant pour la lipase gastrique de chien (LGC) représentée sur la figure 2, et d'autre part, les éléments permettant à une cellule végétale de produire la lipase gastrique de chien codée par ledit ADNc, notamment un promoteur et un terminateur de transcription reconnus par la machinerie transcriptionnelle des cellules végétales.

3. Séquence nucléotidique recombinante selon la revendication 2, **caractérisée en ce qu'**elle contient:
- en aval dudit ADNc, le terminateur polyA 35S du virus de la mosaïque du chou-fleur (CaMV), ou le terminateur polyA NOS d'Agrobacterium tumefaciens,
- en amont dudit ADNc, le promoteur 35S, ou le promoteur constitutif double 35S (pd35S) du CaMV, ou le promoteur pCRU du gène de la cruciférine de radis, ou le promoteur pGEA1 ou pGEA6 d' Arabidopsis thaliana, ou le promoteur chimérique pSP d' Agrobacterium tumefaciens, ou le promoteur pAR-IAR du riz, ou le promoteur p-γ-zéine du maïs.

4. Séquence nucléotidique recombinante selon l'une des revendications 2 ou 3, **caractérisée en ce qu'**elle contient une séquence codant pour un peptide responsable de l'adressage de la LGC recombinante dans un compartiment déterminé de la cellule végétale.

5. Séquences nucléotidiques recombinantes selon l'une des revendications 2 à 4, **caractérisées en ce qu'**elles sont choisies parmi les suivantes:
- celle (désignée pd35S-PS-LGC) contenant dans le sens 5' > 3' le promoteur pd35S du CaMV, la séquence codant pour le peptide signal de la sporamine A, cette dernière étant immédiatement suivie par la séquence nucléotidique représentée sur la figure 3, puis le terminateur poly A 35S du CaMV,
- celle (désignée pd35S-PPS-LGC) contenant dans le sens 5' > 3' le promoteur pd35S du CaMV, la séquence codant pour le prépropeptide de la sporamine A, cette dernière étant immédiatement suivie par la séquence nucléotidique représentée sur la figure 3, puis le terminateur poly A 35S du CaMV,
- celle (désignée pd35S-PSLGL-LGC) contenant dans le sens 5' > 3' le promoteur pd35S du CaMV, la séquence codant pour une partie du peptide signal la LGL (à savoir pour une séquence constituée des 19 premiers acides aminés, indiquée dans les exemples qui suivent ci-après, les 9 nucléotides codant pour les 3 derniers acides aminés C-terminaux étant supprimés), cette dernière étant immédiatement suivie par l'ADNc représenté sur la figure 3, puis le terminateur poly A 35S du CaMV,
- celle (désignée pCRU-PS-LGC) contenant dans le sens 5' > 3' le promoteur pCRU de la cruciférine, la séquence codant pour le peptide signal de la sporamine A, cette dernière étant immédiatement suivie par la séquence nucléotidique représentée sur la figure 3, puis le terminateur poly A 35S du CaMV,
- celle (désignée pCRU-PPS-LGC) contenant dans le sens 5' > 3' le promoteur pCRU de la cruciférine, la séquence codant pour le prépropeptide de la sporamine A, cette dernière étant immédiatement suivie par la séquence nucléotidique représentée sur la figure 3, puis le terminateur poly A 35S du CaMV,
- celle (désignée pCRU-PSLGL-LGC) contenant dans le sens 5' > 3' le promoteur pCRU de la cruciférine, la séquence codant pour une partie du peptide signal de la LGL (telle que décrite ci-dessus), cette dernière étant immédiatement suivie par l' ADNc représenté sur la figure 3, puis le terminateur polyA 35S du CaMV,
- celle (désignée pGEA1-PSLGL-LGC) contenant dans le sens 5' > 3' le promoteur pGEA1 d'Arabidopsis thaliana, la séquence codant pour une partie du peptide signal de la LGL (telle que décrite ci-dessus), cette dernière étant immédiatement suivie par l'ADNc représenté sur la figure 3, puis le terminateur polyA 35S du CaMV,
- celle (désignée pGEA6-PSLGL-LGC) contenant dans le sens 5' > 3' le promoteur pGEA6 d'Arabidopsis thaliana, la séquence codant pour une partie du peptide signal de la LGL (telle que décrite ci-dessus), cette dernière étant immédiatement suivie par l'ADNc représenté sur la figure 3, puis le terminateur poly A 35S du CaMV,
- celle (désignée pAR-IAR-PSLGL-LGC) contenant dans le sens 5' > 3' le promoteur pAR-IAR du riz, la séquence codant pour une partie du peptide signal de la LGL (telle que décrite ci-dessus), cette dernière étant immédiatement suivie par l'ADNc représenté sur la figure 3, puis le terminateur polyA 35S du CaMV, ou le terminateur polyA NOS d'Agrobacterium tumefaciens,
- celle (désignée p-γ-zéine-P5LGL-LGC) contenant dans le sens 5' > 3' le promoteur p-γ-zéine du maïs, la séquence codant pour une partie du peptide signal de la LGL (telle que décrite ci-dessus), cette dernière étant immédiatement suivie par l'ADNc représenté sur la figure 3, puis le terminateur poly A 35S du CaMV,
- celle (désignée p-γ-zéine-PSLGL-LGC-KDEL) contenant dans le sens 5' > 3' le promoteur p-γ-zéine du maïs, la séquence codant pour une partie du peptide signal de la LGL (telle que décrite ci-dessus), cette dernière étant immédiatement suivie par l'ADNc représenté sur la figure 3, puis la séquence codant pour le tétrapeptide KDEL, puis le terminateur poly A 35S du CaMV.

6. Vecteur, notamment plasmide, contenant une séquence nucléotidique selon l'une des revendications 2 à 5, en un site non essentiel pour sa réplication.

7. Hôte cellulaire, notamment bactérie telle que Agrobacterium tumefaciens, transformé par un vecteur selon la revendication 6.

8. Procédé d'obtention de LGC recombinante sous forme d'enzyme active, **caractérisé en ce qu'**il comprend:
- la transformation de cellules végétales, notamment à l'aide d'un hôte cellulaire selon la revendication 7, lui-même transformé par un vecteur selon la revendication 6, de manière à intégrer dans le génome de ces cellules une séquence recombinante selon l'une des revendications 2 à 5,
- le cas échéant, l'obtention de plantes transformées à partir des cellules transformées susmentionnées,
- la récupération de la LGC recombinante susmentionnée produite dans lesdites cellules ou plantes transformées susmentionnées, notamment par extraction, suivie, le cas échéant, par une purification.

9. Plantes, ou parties de plantes, notamment feuilles et/ou fruits et/ou semences et/ou cellules de plantes, transformées génétiquement, **caractérisées en ce qu'**elles contiennent une (ou plusieurs) séquence(s) nucléotidique(s) recombinante(s) selon l'une des revendications 2 à 5, intégrée(s) de façon stable dans leur génome, et le cas échéant, de la LGC recombinante à activité enzymatique, et plus particulièrement à activité lipasique, choisies notamment parmi le colza, le tabac, le maïs, le pois, la tomate, la carotte, le blé, l'orge, la pomme de terre, le soja, le tournesol, la laitue, le riz, la luzerne, et la betterave.

10. LGC recombinante enzymatiquement active, dont la séquence en acides aminés est celle représentée sur la figure 2, ou polypeptide choisi parmi le polypeptide délimité par les acides aminés situés aux positions 55 et 379, de la figure 2, ou le polypeptide délimité par les acides aminés situés aux positions 35 et 379 de la figure 2, ces polypeptides présentant une activité lipasique, obtenus sous forme essentiellement pure par mise en oeuvre d'un procédé selon la revendication 8, ce procédé comprenant une étape de purification de la LGC recombinante et/ou du (ou des) polypeptide(s) susmentionné(s), notamment par chromatographie réalisée à partir des extraits enzymatiques obtenus.

11. Extrait végétal à activité enzymatique susceptible d'être obtenu par mise en oeuvre d'un procédé selon la revendication 8, **caractérisé en ce qu'**il contient de la LGC recombinante enzymatiquement active, dont la séquence en acides aminés est celle représentée sur la figure 2, et/ ou des polypeptides choisis parmi le polypeptide délimité par les acides aminés situés aux positions 55 et 379, de la figure 2, ou le polypeptide délimité par les acides aminés situés aux positions 35 et 379 de la figure 2, ces polypeptides présentant une activité lipasique.

12. Extrait végétal à activité enzymatique selon la revendication 11, **caractérisée en ce que** le pourcentage en poids de polypeptides recombinants enzymatiquement actifs, représente environ 0,1% à 20%, notamment environ 1 % à environ 15 %, par rapport au poids total de protéines présentes dans ces extraits, ce qui correspond à des mesures d'activité enzymatique d'environ 0,5 U par g de poids frais (PF) de feuilles à environ 1000 U/g PF de feuilles, notamment d'environ 10 U/g PF à environ 300 U / g PF de feuilles, ou encore d'environ 1 U / g PF de graines à environ 5000 U/g PF, notamment à environ 10 U/g PF de graines à environ 1000 U/g PF de graines.

13. Utilisation de plantes, ou parties de plantes, de préférence feuilles et/ou fruits et/ou semences et/ou cellules de plantes, transformés génétiquement, selon la revendication 9, pour la préparation d'un médicament destiné à faciliter l'absorption des graisses animales ou végétales ingérées par un individu sain ou atteint d'une ou plusieurs pathologies affectant ou non le taux de production de lipase gastrique et/ou pancréatique, de préférence chez les individus subissant un traitement médical altérant le mécanisme d'absorption des graisses, ou encore chez les personnes âgées, et de préférence pour la préparation d'un médicament destiné au traitement des pathologies liées à l'insuffisance de lipases (de préférence de lipase(s) gastrique et/ou pancréatique) dans l'organisme, et plus préférentiellement de pathologies telles que la mucoviscidose, et l'insuffisance pancréatique exocrine.

14. Utilisation de LGC recombinante selon la revendication 10 pour la préparation d'un médicament destiné à faciliter l'absorption des graisses animales ou végétales ingérées par un individu sain ou atteint d'une ou plusieurs pathologies affectant ou non le taux de production de lipase gastrique et/ou pancréatique, de préférence chez les individus subissant un traitement médical altérant le mécanisme d'absorption des graisses, ou encore chez les personnes âgées, et de préférence pour la préparation d'un médicament destiné au traitement des pathologies liées à l'insuffisance de lipases (de préférence de lipase(s) gastrique et/ou pancréatique) dans l'organisme, et plus préférentiellement de pathologies telles que la mucoviscidose, et l'insuffisance pancréatique exocrine.

15. Utilisation d'extraits enzymatiques selon l'une des revendications 11 et 12 pour la préparation d'un médicament destiné à faciliter l'absorption des graisses animales ou végétales ingérées par un individu sain ou atteint d'une ou plusieurs pathologies affectant ou non le taux de production de lipase gastrique et/ou pancréatique, de préférence chez les individus subissant un traitement médical altérant le mécanisme d'absorption des graisses, ou encore chez les personnes âgées, et de préférence pour la préparation d'un médicament destiné au traitement des pathologies liées à l'insuffisance de lipases (de préférence de lipase(s) gastrique et/ou pancréatique) dans l'organisme, et plus préférentiellement de pathologies telles que la mucoviscidose, et l'insuffisance pancréatique exocrine.

16. Composition pharmaceutique, **caractérisée en ce qu'**elle comprend de la LGC recombinante et/ou plusieurs polypeptides selon la revendication 10, et/ou un ou plusieurs extraits enzymatiques selon l'une des revendications 11 et 12, le cas échéant en association avec un véhicule pharmaceutiquement acceptable.

17. Utilisation de plantes, ou parties de plantes, de préférence feuilles et/ou fruits et/ou semences et/ou cellules de plantes, transformés génétiquement, selon la revendication 9, pour la préparation d'aliments destinés à l'alimentation humaine ou animale, de préférence d'aliments fonctionnels plus particulièrement destinés à faciliter l'absorption des graisses animales ou végétales ingérées par un individu sain ou atteint d'une ou plusieurs pathologies affectant ou non le taux de production de lipase gastrique et/ou pancréatique.

18. Utilisation de LGC recombinante selon la revendication 10, pour la préparation d'aliments destinés à l'alimentation humaine ou animale, de préférence d'aliments fonctionnels plus particulièrement destinés à faciliter l'absorption des graisses animales ou végétales ingérées par un individu sain ou atteint d'une ou plusieurs pathologies affectant ou non le taux de production de lipase gastrique et/ou pancréatique.

19. Aliments fonctionnels **caractérisés en ce qu'**ils comprennent une ou plusieurs plantes et/ou parties de ces plantes, selon la revendication 9, et/ou de la LGC recombinante et/ou un ou plusieurs polypeptides selon la revendication 10 et/ou un ou plusieurs extraits enzymatiques selon l'une des revendications 11 et 12.

## Patentansprüche

1. Verwendung einer in Abbildung 3 dargestellten, rekombinanten Nukleotidsequenz, die einerseits die in Abbildung 2 dargestellte Hundemagenlipase (HML) kodiert und andererseits die Elemente, die einer Pflanzenzelle die Herstellung der von der besagten cDNA kodierten Hundemagenlipase ermöglichen, insbesondere ein Transkriptionspromotor und -terminator, die von der Transkriptionsmaschinerie der Pflanzenzellen erkannt werden, für die Transformation von Pflanzenzellen im Hinblick auf die Gewinnung von rekombinanter HML aus diesen Zellen oder aus Pflanzen, die aus Letzteren gewonnen wurden, in Form von aktivem Enzym.

2. Rekombinante Nukleotidsequenz, **dadurch gekennzeichnet, dass** sie einerseits die in Abbildung 3 dargestellte cDNA enthält, welche die in Abbildung 2 dargestellte Hundemagenlipase (HML) kodiert, und andererseits die Elemente, die einer Pflanzenzelle die Herstellung der von der cDNA kodierten Hundemagenlipase ermöglichen, insbesondere ein Transkriptionspromotor und -terminator, die von der Transkriptionsmaschinerie der Pflanzenzellen erkannt werden.

3. Rekombinante Nukleotidsequenz nach Anspruch 2, **dadurch gekennzeichnet, dass** sie enthält:
- den polyA 35S-Terminator des Blumenkohlmosaikvirus (CaMV) oder den polyA NOS-Terminator von Agrobacterium tumefaciens stromabwärts der cDNA,
- den 35S-Promotor oder den konstitutiven Doppel-35S-Promotor (pd35S) des CaMV oder den pCRU-Promotor des Rettich-Cruciferin-Gens oder den pGEA1- oder pGEA6-Promotor von Arabidopsis thaliana oder den chimären pSP-Promotor von Agrobacterium tumefaciens oder den Reis-pAR-IAR-Promotor oder den Mais-p-γ-Zein-Promotor stromaufwärts der cDNA.

4. Rekombinante Nukleotidsequenz nach einem der Ansprüche 2 oder 3, **dadurch gekennzeichnet, dass** sie eine für ein Peptid, das für das Targeting der rekombinanten HML in ein bestimmtes Kompartiment der Pflanzenzelle verantwortlich ist, kodierende Sequenz enthält.

5. Rekombinante Nukleotidsequenzen nach einem der Ansprüche 2 bis 4, **dadurch gekennzeichnet, dass** sie unter den Folgenden ausgewählt sind:
- diejenige (als pd35S-SP-HML bezeichnet), die in 5'>3'-Richtung den CaMV-pd35S-Promotor, die für das Sporamin A-Signalpeptid kodierende Sequenz, wobei letztere unmittelbar von der in Abbildung 3 dargestellten Nukleotidsequenz gefolgt ist, und dann den CaMV-poly A 35S-Terminator enthält,
- diejenige (als pd35S-SPP-HML bezeichnet), die in 5'>3'-Richtung den CaMV-pd35S-Promotor, die für das Sporamin A-Präpropeptid kodierende Sequenz, wobei letztere unmittelbar von der in Abbildung 3 dargestellten Nukleotidsequenz gefolgt ist, und dann den CaMV-poly A 35S-Terminator enthält,
- diejenige (als pd35S-SPKML-HML bezeichnet), die in 5'>3'-Richtung den CaMV-pd35S-Promotor, die für einen Teil des Kaninchenmagenlipase (KML)-Signalpeptids kodierende Sequenz (und zwar für eine Sequenz bestehend aus den 19 ersten Aminosäuren, wobei die 9 für die 3 letzten C-terminalen Aminosäuren kodierenden Nukleotide ausgeschlossen sind), wobei letztere unmittelbar von der in Abbildung 3 dargestellten cDNA gefolgt ist, und dann den CaMV-poly A 35S-Terminator enthält,
- diejenige (als pCRU-SP-HML bezeichnet), die in 5'>3'-Richtung den Cruciferin-pCRU-Promotor, die für das Sporamin A-Signalpeptid kodierende Sequenz, wobei letztere unmittelbar von der in Abbildung 3 dargestellten Nukleotidsequenz gefolgt ist, und dann den CaMV-poly A 35S-Terminator enthält,
- diejenige (als pCRU-SPP-HML bezeichnet), die in 5'>3'-Richtung den Cruciferin-pCRU-Promotor, die für das Sporamin A-Präpropeptid kodierende Sequenz, wobei letztere unmittelbar von der in Abbildung 3 dargestellten Nukleotidsequenz gefolgt ist, und dann den CaMV-poly A 35S-Terminator enthält,
- diejenige (als pCRU-SPKML-HML bezeichnet), die in 5'>3'-Richtung den Cruciferin-pCRU-Promotor, die für einen Teil des KML-Signalpeptids kodierende Sequenz (wie oben beschrieben), wobei letztere unmittelbar von der in Abbildung 3 dargestellten cDNA gefolgt ist, und dann den CaMV-poly A 35S-Terminator enthält,
- diejenige (als pGEA1-SPKML-HML bezeichnet), die in 5'>3'-Richtung den pGEA1-Promotor aus Arabidopsis thaliana, die für einen Teil des KML-Signalpeptids kodierende Sequenz (wie oben beschrieben), wobei letztere unmittelbar von der in Abbildung 3 dargestellten cDNA gefolgt ist, und dann den CaMV-poly A 35S-Terminator enthält,
- diejenige (als pGEA6-SPKML-HML bezeichnet), die in 5'>3'-Richtung den pGEA6-Promotor aus Arabidopsis thaliana, die für einen Teil des KML-Signalpeptids kodierende Sequenz (wie oben beschrieben), wobei letztere unmittelbar von der in Abbildung 3 dargestellten cDNA gefolgt ist, und dann den CaMV-poly A 35S-Terminator enthält,
- diejenige (als pAR-IAR-SPKML-HML bezeichnet), die in 5'>3'-Richtung den Reis-pAR-IAR-Promotor, die für einen Teil des KML-Signalpeptids kodierende Sequenz (wie oben beschrieben), wobei letztere unmittelbar von der in Abbildung 3 dargestellten cDNA gefolgt ist, und dann den CaMV-poly A 35S-Terminator oder den polyA NOS-Terminator aus Agrobacterium tumefaciens enthält,
- diejenige (als p-γ-Zein-SPKML-HML bezeichnet), die in 5'>3'-Richtung den Mais-p-γ-Zein-Promotor, die für einen Teil des KML-Signalpeptids kodierende Sequenz (wie oben beschrieben), wobei letztere unmittelbar von der in Abbildung 3 dargestellten cDNA gefolgt ist, und dann den CaMV-poly A 35S-Terminator enthält,
- diejenige (als p-γ-Zein-SPKML-HML-KDEL bezeichnet), die in 5'>3'-Richtung den Mais-p-γ-Zein-Promotor, die für einen Teil des KML-Signalpeptids kodierende Sequenz (wie oben beschrieben), wobei letztere unmittelbar von der in Abbildung 3 dargestellten cDNA gefolgt ist, dann die für das KDEL-Tetrapeptid kodierende Sequenz und dann den CaMV-poly A 35S-Terminator enthält.

6. Vektor, insbesondere Plasmid, enthaltend eine Nukleotidsequenz nach einem der Ansprüche 2 bis 5 an einer für seine Replikation nicht essentiellen Stelle.

7. Wirtszelle, insbesondere Bakterium wie Agrobacterium tumefaciens, die mit einem Vektor nach Anspruch 6 transformiert wurde.

8. Verfahren zur Gewinnung rekombinanter HML in Form von aktivem Enzym, **dadurch gekennzeichnet, dass** es umfasst:
- die Transformation von Pflanzenzellen, insbesondere mit Hilfe einer Wirtszelle nach Anspruch 7, die ihrerseits mit einem Vektor nach Anspruch 6 transformiert ist, so dass eine rekombinante Sequenz nach einem der Ansprüche 2 bis 5 in das Genom dieser Zellen integriert wird,
- gegebenenfalls die Gewinnung der transformierten Pflanzen aus den oben genannten transformierten Zellen,
- die Gewinnung der oben genannten rekombinanten HML, die in den oben genannten transformierten Zellen oder Pflanzen hergestellt wird, insbesondere durch Extraktion, gegebenenfalls gefolgt von einer Aufreinigung.

9. Genetisch transformierte Pflanzen oder Pflanzenteile, insbesondere Blätter und/oder Früchte und/oder Samen und/oder Pflanzenzellen, **dadurch** charakterisiert, dass sie eine (oder mehrere) rekombinante, stabil in ihr Genom integrierte Nukleotidsequenz(en) nach einem der Ansprüche 2 bis 5 und gegebenenfalls rekombinante HML mit enzymatischer Aktivität und insbesondere mit Lipaseaktivität enthalten, insbesondere ausgewählt aus Raps, Tabak, Mais, Erbse, Tomate, Karotte, Weizen, Gerste, Kartoffel, Soja, Sonnenblume, Kopfsalat, Reis, Luzerne und Rübe.

10. Rekombinante, enzymatisch aktive HML, deren Aminosäuresequenz die in Abbildung 2 dargestellte ist, oder Polypeptid, ausgewählt aus dem Polypeptid, das durch die an den Positionen 55 und 379 liegenden Aminosäuren der Abbildung 2 begrenzt ist, oder dem Polypeptid, das durch die an den Positionen 35 und 379 liegenden Aminosäuren der Abbildung 2 begrenzt ist, wobei diese Polypeptide eine Lipaseaktivität aufweisen, in weitgehend reiner Form durch Durchführung eines Verfahrens nach Anspruch 8 gewonnen, wobei dieses Verfahren einen Schritt der Aufreinigung der rekombinanten HML und/oder des (der) oben genannten Polypeptids (Polypeptide) umfasst, insbesondere durch Chromatographie, die ausgehend von den gewonnenen enzymatischen Extrakten durchgeführt wird.

11. Pflanzenextrakt mit enzymatischer Aktivität, das geeignet ist, durch Durchführung eines Verfahrens nach Anspruch 8 gewonnen zu werden, **dadurch gekennzeichnet, dass** es rekombinante, enzymatisch aktive HML, deren Aminosäuresequenz die in Abbildung 2 dargestellte ist, und/oder Polypeptide enthält, die ausgewählt sind aus dem Polypeptid, das durch die an den Positionen 55 und 379 liegenden Aminosäuren der Abbildung 2 begrenzt ist, oder dem Polypeptid, das durch die an den Positionen 35 und 379 liegenden Aminosäuren der Abbildung 2 begrenzt ist, wobei diese Polypeptide eine Lipaseaktivität aufweisen.

12. Pflanzenextrakt mit enzymatischer Aktivität nach Anspruch 11, **dadurch gekennzeichnet, dass** der Gewichtsprozentanteil rekombinanter, enzymatisch aktiver Polypeptide ungefähr 0,1 % bis 20 % beträgt, insbesondere ungefähr 1 % bis ungefähr 15 %, im Vergleich zum Gesamtgewicht der in diesen Extrakten vorliegenden Proteine, was Messungen der enzymatischen Aktivität von ungefähr 0,5 U pro g Frischgewicht (FG) an Blättern bis ungefähr 1000 U/g FG an Blättern entspricht, insbesondere von ungefähr 10 U/g FG bis ungefähr 300 U/g FG an Blättern, oder auch von ungefähr 1 U/g FG an Samen bis ungefähr 5000 U/g FG, insbesondere von ungefähr 10 U/g FG an Samen bis ungefähr 1000 U/g FG an Samen.

13. Verwendung von genetisch transformierten Pflanzen oder Pflanzenteilen, vorzugsweise Blättern und/oder Früchten und/oder Samen und/oder Pflanzenzellen, nach Anspruch 9, für die Herstellung eines Arzneimittels, das dazu bestimmt ist, die Absorption von tierischen oder pflanzlichen Fetten zu erleichtern, die von einem gesunden oder von einer oder mehreren Krankheiten befallenen Individuum eingenommen wurden, wobei die Krankheiten die Herstellungsrate von Magen- und/oder Pankreaslipase beeinflussen oder nicht, vorzugsweise bei den Individuen, die sich einer medizinischen Behandlung unterziehen, welche den Fettabsorptionsmechanismus verändert, oder auch bei älteren Personen, und vorzugsweise für die Herstellung eines Arzneimittels, das zur Behandlung von Krankheiten bestimmt ist, die mit der Lipaseninsuffizienz (vorzugsweise von Magen- und/oder Pankreaslipase(n)) im Organismus zusammenhängen, und noch mehr bevorzugt von Krankheiten wie der Mukoviszidose und der exokrinen Pankreasinsuffizienz.

14. Verwendung von rekombinanter HML nach Anspruch 10 für die Herstellung eines Arzneimittels, das dazu bestimmt ist, die Absorption von tierischen oder pflanzlichen Fetten zu erleichtern, die von einem gesunden oder von einer oder mehreren Krankheiten befallenen Individuum eingenommen wurden, wobei die Krankheiten die Herstellungsrate von Magen- und/oder Pankreaslipase beeinflussen oder nicht, vorzugsweise bei den Individuen, die sich einer medizinischen Behandlung unterziehen, welche den Fettabsorptionsmechanismus verändert, oder auch bei älteren Personen, und vorzugsweise für die Herstellung eines Arzneimittels, das zur Behandlung von Krankheiten bestimmt ist, die mit der Lipaseninsuffizienz (vorzugsweise von Magen- und/oder Pankreaslipase(n)) im Organismus zusammenhängen, und noch mehr bevorzugt von Krankheiten wie der Mukoviszidose und der exokrinen Pankreasinsuffizienz.

15. Verwendung von enzymatischen Extrakten nach einem der Ansprüche 11 und 12 für die Herstellung eines Arzneimittels, das dazu bestimmt ist, die Absorption von tierischen oder pflanzlichen Fetten zu erleichtern, die von einem gesunden oder von einer oder mehreren Krankheiten befallenen Individuum eingenommen wurden, wobei die Krankheiten die Herstellungsrate von Magen- und/oder Pankreaslipase beeinflussen oder nicht, vorzugsweise bei den Individuen, die sich einer medizinischen Behandlung unterziehen, welche den Fettabsorptionsmechanismus verändert, oder auch bei älteren Personen, und vorzugsweise für die Herstellung eines Arzneimittels, das zur Behandlung von Krankheiten bestimmt ist, die mit der Lipaseninsuffizienz (vorzugsweise von Magen- und/oder Pankreaslipase(n)) im Organismus zusammenhängen, und noch mehr bevorzugt von Krankheiten wie der Mukoviszidose und der exokrinen Pankreasinsuffizienz.

16. Pharmazeutische Zusammensetzung, **dadurch gekennzeichnet, dass** sie rekombinante HML und/oder mehrere Polypeptide nach Anspruch 10 und/oder einen oder mehrere enzymatische Extrakte nach einem der Ansprüche 11 und 12 enthält, gegebenenfalls in Verbindung mit pharmazeutisch annehmbaren Träger.

17. Verwendung von genetisch transformierten Pflanzen oder Pflanzenteilen, vorzugsweise Blättern und/oder Früchten und/oder Samen und/oder Pflanzenzellen, nach Anspruch 9, für die Herstellung von Nahrungsmitteln, die zur menschlichen oder tierischen Ernährung bestimmt sind, vorzugsweise von funktionellen Nahrungsmitteln, die insbesondere dazu dienen, die Absorption der tierischen oder pflanzlichen Fette zu erleichtern, die von einem gesunden oder von einer oder mehreren Krankheiten befallenen Individuum eingenommen wurden, wobei die Krankheiten die Herstellungsrate von Magen- und/oder Pankreaslipase beeinflussen oder nicht.

18. Verwendung von rekombinanter HML nach Anspruch 10 für die Herstellung von Nahrungsmitteln, die zur menschlichen oder tierischen Ernährung bestimmt sind, vorzugsweise von funktionellen Nahrungsmitteln, die insbesondere dazu dienen, die Absorption der tierischen oder pflanzlichen Fette zu erleichtern, die von einem gesunden oder von einer oder mehreren Krankheiten befallenen Individuum eingenommen wurden, wobei die Krankheiten die Herstellungsrate von Magen- und/oder Pankreaslipase beeinflussen oder nicht.

19. Funktionelle Nahrungsmittel, **dadurch gekennzeichnet, dass** sie eine oder mehrere Pflanzen und/oder Teile dieser Pflanzen nach Anspruch 9 und/oder rekombinante HML und/oder ein oder mehrere Polypeptide nach Anspruch 10 und/oder einen oder mehrere enzymatische Extrakte nach einem der Ansprüche 11 und 12 umfassen.

## Claims

1. Use of a recombinant nucleotide sequence as shown in Figure 3, coding on the one hand for the dog gastric lipase (DGL) shown in Figure 2, and on the other hand, the elements enabling a plant cell to produce the dog gastric lipase coded for by said cDNA, notably a transcription promoter and terminator recognized by the transcriptional machinery of the plant cells, for transforming plant cells with a view to obtaining, from said cells, or plants obtained from the latter, recombinant DGL in an active enzyme form.

2. Recombinant nucleotide sequence, **characterised in that** it contains, on the one hand, the cDNA as shown in Figure 3 and coding for the dog gastric lipase as shown in Figure 2, and on the other hand, the elements enabling a plant cell to produce the dog gastric lipase coded for by said cDNA, notably a transcription promoter and terminator recognized by the transcriptional machinery of the plant cells.

3. Recombinant nucleotide sequence according to claim 2, **characterised in that** it contains :
- downstream of said cDNA, the polyA 35S terminator from cauliflower mosaic virus (CaMV), or the polyA NOS terminator from Agrobacterium tumefaciens,
- upstream of said cDNA, the 35S promoter, or the constitutive double 35S promoter (pd35S) from CaMV, or the pCRU promoter from the radish cruciferin gene, or the pGEA1 or pGEA6 promoters from Arabidopsis thaliana, or the chimeric pSP promoter from Agrobacterium tumefaciens, or the pAR-IAR rice promoter, or the p-γ-zein maize promoter.

4. Recombinant nucleotide sequence according to any one of claim 2 or claim 3, **characterised in that** it contains a sequence coding for a peptide responsible for addressing the recombinant DGL to a predetermined plant cell compartment.

5. Recombinant nucleotide sequences according to any one of claim 2 to claim 4, **characterised in that** they are selected from the following :
- a sequence, designated pd35S-PS-LGC, and containing, in the 5' to 3' direction, the CaMV pd35S promoter, the sequence coding for the sporamine A signal peptide, said latter sequence being immediately followed by the nucleotide sequence shown in Figure 3, and then the CaMV polyA 35S terminator;
- a sequence, designated pd35S-PPS-LGC, and containing, in the 5' to 3' direction, the CaMV pd35S promoter, the sequence coding for the sporamine A prepropeptide, said latter sequence being immediately followed by the nucleotide sequence shown in Figure 3, and then the CaMV polyA 35S terminator;
- a sequence, designated pd35S-PSLGL-LGC, and containing, in the 5' to 3' direction, the CaMV pd35S promoter, the sequence coding for a part of the RGL (rabbit gastric lipase) signal peptide, that is for a sequence consisting of the first 19 amino acids, given in the examples hereafter, the 9 nucleotides coding for the last three C-terminal amino acids having been deleted, said latter sequence being immediately followed by the nucleotide sequence shown in Figure 3, and then the CaMV polyA 35S terminator;
- a sequence, designated pCRU-PS-LGC, and containing, in the 5' to 3' direction, the pCRU cruciferin promoter, the sequence coding for the sporamine A signal peptide, said latter sequence being immediately followed by the nucleotide sequence shown in Figure 3, and then the CaMV polyA 35S terminator;
- a sequence, designated pCRU-PPS-LGC, containing , in the 5' to 3' direction, the pCRU cruciferin promoter, the sequence coding for the sporamine A prepropeptide, said latter sequence being immediately followed by the nucleotide sequence shown in Figure 3, and then the CaMV polyA 35S terminator;
- a sequence, designated pd35S-PSLGL-LGC, and containing, in the 5' to 3' direction, the pCRU cruciferin promoter, the sequence coding for a part of the signal peptide of RGL, as described above, said latter sequence being immediately followed by the nucleotide sequence shown in Figure 3, and then the CaMV polyA 35S terminator;
- a sequence, designated pGEA1-PSLGL-LGC, and containing, in the 5' to 3' direction, the pGEA1 Arabidopsis thaliana promoter, the sequence coding for a part of the signal peptide of RGL, as described above, said latter sequence being immediately followed by the nucleotide sequence shown in Figure 3, and then the CaMV polyA 35S terminator;
- a sequence, designated pGEA6-PSLGL-LGC, and containing, in the 5' to 3' direction, the pGEA6 Arabidopsis thaliana promoter, the sequence coding for a part of the signal peptide of RGL, as described above, said latter sequence being immediately followed by the nucleotide sequence shown in Figure 3, and then the CaMV polyA 35S terminator;
- a sequence, designated pAR-IAR-PSLGL-LGC, and containing, in the 5' to 3' direction, the pAR-IAR rice promoter, the sequence coding for a part of the signal peptide of RGL, as described above, said latter sequence being immediately followed by the nucleotide sequence shown in Figure 3, and then the CaMV polyA 35S terminator, or the polyA NOS terminator from Agrobacterium tumefaciens;
- a sequence, designated p-γ-zein-PSLGL-LGC, and containing, in the 5' to 3' direction, the γ-zein maize promoter, the sequence coding for a part of the signal peptide of RGL, as described above, said latter sequence being immediately followed by the nucleotide sequence shown in Figure 3, and then the CaMV polyA 35S terminator;
- a sequence, designated p-γ-zein-PSLGL-LGC-KDEL, and containing, in the 5' to 3' direction, the γ-zein maize promoter, the sequence coding for a part of the signal peptide of RGL, as described above, said latter sequence being immediately followed by the nucleotide sequence shown in Figure 3, and then the sequence coding for the KDEL tetrapeptide, followed by the CaMV polyA 35S terminator.

6. Vector, notably a plasmid, containing a nucleotide sequence according to any one of claims 2 to 5, at a site which is non-essential for replication.

7. Cell host, notably a bacteria such as Agrobacterium tumefaciens, transformed by a vector according to claim 6.

8. Process for obtaining recombinant DGL in an active enzyme form, **characterised in that** it comprises :
- transforming plant cells, notably with the aid of a host cell according to claim 7, the latter having been obtained by transformation with a vector according to claim 6, such that a recombinant sequence according to any one of claims 2 to 5 is integrated into the genome of said plant cells;
- optionally obtaining transformed plants from the transformed plant cells mentioned above;
- recovering the abovementioned recombinant DGL produced in said abovementioned transformed cells or transformed plants, notably via extraction, and optionally followed by purification.

9. Genetically transformed plants, or plant parts, notably leaves, and/or fruit, and/or seeds, and/or plant cells, **characterised in that** they contain one or more recombinant nucleotide sequences according to any one of claims 2 to 5, stably integrated into their genome, and optionally, recombinant DGL having enzymatic activity, and more particularly lipase activity, selected notably from rape, tobacco, maize, pea, tomato, carrot, wheat, barley, potato, soya, sunflower, lettuce, rice, lucerne and beetroot.

10. Enzymatically active recombinant DGL, the amino acid sequence of which is shown in Figure 2, or the polypeptide chosen from the polypeptide delimited by the amino acids located at positions 55 and 379 of Figure 2, or the polypeptide delimited by the amino acids located at positions 35 and 379 of Figure 2, these polypeptides having lipase activity, obtained in substantially pure form by application of the process according to claim 8, the process comprising a purification step for the recombinant DGL, and/or the abovementioned polypeptides, notably through chromatography carried out on the obtained enzymatic extracts.

11. Enzymatically active plant extract obtainable by application of a process according to claim 8, **characterised in that** it contains enzymatically active recombinant DGL, the amino acid sequence of which is shown in Figure 2, and/or polypeptides chosen from the polypeptide delimited by the amino acids located at positions 55 and 379 of Figure 2, or the polypeptide delimited by the amino acids located at positions 35 and 379 of Figure 2, these polypeptides having lipase activity.

12. Enzymatically active plant extract according to claim 11, **characterised in that** the percentage by weight of enzymatically active recombinant polypeptides represents about 0.1% to 20%, notably about 1% to 15%, with regard to the total protein weight present in these extracts, which corresponds to a measured enzymatic activity of about 0.5U per gram fresh weight (FW) of leaves to about 1000U per gram fresh weight (FW) of leaves, notably about 10U per gram fresh weight (FW) to about 300U per gram fresh weight (FW) of leaves, or about 1U/g to 5000U/g FW of seeds, notably about 10U/g FW to 1000U/g FW of seeds.

13. Use of genetically transformed plants, and/or plant parts, preferably leaves, and/or fruit, and/or seeds, and/or plant cells, according to claim 9, for the manufacture of a medicament for facilitating absorption of animal or vegetable fats when ingested by a healthy individual or an individual struck by one or more pathologies affecting, or not, the rate of production of gastric and/or pancreatic lipase, preferably with individuals undergoing medical treatment that modifies the fat absorption mechanism, or with the elderly, and preferably for the manufacture of a medicament for treating pathologies linked to lipase insufficiency in the organism, preferably gastric and/or pancreatic lipase, and more preferably pathologies such as cystic fibrosis and exocrine pancreatic insufficiency.

14. Use of a recombinant DGL according to claim 10 for the manufacture of a medicament for facilitating absorption of animal or vegetable fats when ingested by a healthy individual or an individual struck by one or more pathologies affecting, or not, the rate of production of gastric and/or pancreatic lipase, preferably with individuals undergoing medical treatment that modifies the fat absorption mechanism, or with the elderly, and preferably for the manufacture of a medicament for treating pathologies linked to lipase insufficiency in the organism, preferably gastric and/or pancreatic lipase, and more preferably pathologies such as cystic fibrosis and exocrine pancreatic insufficiency.

15. Use of enzymatic extracts according to any one of claims 11 and 12 for the manufacture of a medicament for facilitating absorption of animal or vegetable fats when ingested by a healthy individual or an individual struck by one or more pathologies affecting, or not, the rate of production of gastric and/or pancreatic lipase, preferably with individuals undergoing medical treatment that modifies the fat absorption mechanism, or with the elderly, and preferably for the manufacture of a medicament for treating pathologies linked to lipase insufficiency in the organism, preferably gastric and/or pancreatic lipase, and more preferably pathologies such as cystic fibrosis and exocrine pancreatic insufficiency.

16. Pharmaceutical composition, **characterised in that** it comprises recombinant DGL and/or several polypeptides according to claim 10, and/or one or more enzymatic extracts according to any one of claims 11 and 12, optionally in association with a pharmaceutically acceptable vehicle.

17. Use of genetically transformed plants, and/or plant parts, preferably leaves, and/or fruit, and/or seeds, and/or plant cells, according to claim 9, for the manufacture of foodstuffs intended for human or animal nutrition, preferably functional foods for facilitating absorption of animal or vegetable fats when ingested by a healthy individual or an individual struck by one or more pathologies affecting, or not, the rate of production of gastric and/or pancreatic lipase.

18. Use of recombinant DGL according to claim 10, for the manufacture of foodstuffs intended for human or animal nutrition, preferably functional foods for facilitating absorption of animal or vegetable fats when ingested by a healthy individual or an individual struck by one or more pathologies affecting, or not, the rate of production of gastric and/or pancreatic lipase.

19. Functional foods, **characterised in that** they comprise one or more plants, and/or plant parts, according to claim 9, and/or recombinant DGL and/or one or more polypeptides according to claim 10, and/or one or more enzymatic extracts according to any one of claims 11 and 12.
